(19) Europäisches Patentamt
European Patent Office
Office européen des brevets

(11) Veröffentlichungsnummer : **0 202 529 B1**

(12) # EUROPÄISCHE PATENTSCHRIFT

(45) Veröffentlichungstag der Patentschrift : **13.12.89**

(21) Anmeldenummer : **86106126.5**

(22) Anmeldetag : **05.05.86**

(51) Int. Cl.⁴ : **C 07 C 39/18**, C 07 C 69/88, C 07 C 43/205, C 07 D309/12, C 07 F 7/18

(54) **Neue Phenolderivate, diese enthaltende Arzneimittel und Verfahren zur Herstellung dieser Verbindungen und Arzneimittel.**

(30) Priorität : **24.05.85 DE 3518655**

(43) Veröffentlichungstag der Anmeldung : **26.11.86 Patentblatt 86/48**

(45) Bekanntmachung des Hinweises auf die Patenterteilung : **13.12.89 Patentblatt 89/50**

(84) Benannte Vertragsstaaten : **AT BE CH DE FR GB IT LI LU NL SE**

(56) Entgegenhaltungen : **US-A- 3 033 668**

(73) Patentinhaber : **Grünenthal GmbH**
**Zieglerstrasse 6**
**D-5100 Aachen (DE)**

(72) Erfinder : **Zimmer, Oswald, Dr.**
**Weckspfad 4**
**D-5160 Düren (DE)**
Erfinder : **Vollenberg, Werner, Dr.**
**Eupenerstrasse 26a**
**D-5190 Stolberg (DE)**
Erfinder : **Loschen, Gerriet, Dr.**
**Erzweg 4**
**D-5190 Stolberg (DE)**
Erfinder : **Winter, Werner, Prof. Dr.**
**Birkenstrasse 5**
**D-5100 Aachen (DE)**
Erfinder : **Kiesewetter, Erwin, Dr.**
**Obere Donnerbergstrasse 57**
**D-5190 Stolberg (DE)**
Erfinder : **Seipp, Ulrich, Dr.**
**Pfeilstrasse 5**
**D-5100 Aachen (DE)**

**EP 0 202 529 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Polyungesättigte höhere Fettsäuren wie z. B. die Arachidonsäure dienen im Stoffwechsel des Menschen und von Säugetieren als Substrate für die enzymatisch katalysierte Bildung physiologisch bedeutsamer Eicosanoide wie z. B. Prostaglandine und Leukotriene, einer auch unter dem Namen « Slow reacting Substance of Anaphylaxis » (SRS-A) bekannten Substanzklasse. Dabei wird die Prostaglandinbildung durch die Cyclo-oxygenase (auch als « Prostaglandinsynthetase » bezeichnet), die Leukotrienbildung durch 5-Lipoxygenase katalysiert.

Während die Prostaglandine eine Anzahl erwünschter Wirkungen im Organismus entfalten, ist von den Leukotrienen bzw. der SRS-A, bekannt, daß sie für die Entstehung von allergischen Reaktionen, Bronchokonstriktionen, Entzündungen, Asthma und eine Vielzahl weiterer unerwünschter Effekte verantwortlich sind. Es wäre daher wünschenswert, über chemisch und metabolisch stabile Verbindungen verfügen zu können, die im Organismus die Prostaglandinbildung unbeeinflußt lassen, gleichzeitig aber möglichst selektiv bzw. spezifisch die 5-Lipoxygenase hemmen und so die Bildung der unerwünschten Leukotriene verhindern. Es wurde nunmehr gefunden, daß bestimmte, in 4-Stellung einen ungesättigten 11-Hydroxyalkylrest tragende Phenolderivate chemisch und metabolisch für eine therapeutische Anwendung hinreichend stabil sind und eine spezifische Hemmwirkung gegenüber der 5-Lipoxygenase besitzen.

Diese neuen Phenolderivate entsprechen der allgemeinen Formel

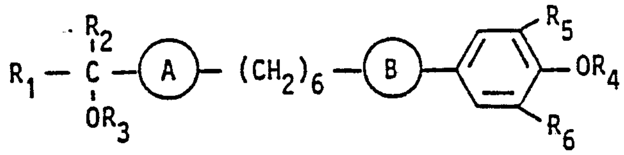

(I)

in der

A und B gleich oder verschieden sind und jeweils für eine der Gruppen $-C{\equiv}C-$, cis-CH=CH— oder trans-CH=CH stehen,

$R_1$ Wasserstoff oder einen geradkettigen Alkylrest mit 1 bis 6 Kohlenstoffatomen, oder eine 5- bis 7-gliedrige Cycloalkylgruppe, oder eine Gruppe der Formel $-(CH_2)_m-O-R_7$, worin m für eine der Zahlen 1, 2 oder 3 und $R_7$ für Methyl oder Ethyl steht, oder eine Gruppe der Formel

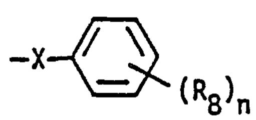

worin X für eine einfache Bindung, eine $-CH_2$-Gruppe oder für eine $-CH_2O$-Gruppe steht und $R_8$ ein Wasserstoff-, Chlor- oder Fluoratom oder eine Methyl-, Methoxy- oder Trifluormethylgruppe bedeutet und n für eine der Zahlen 1 oder 2 steht,
bedeutet,

$R_2$ für Wasserstoff, Methyl oder Ethyl steht,

$R_3$ Wasserstoff oder einen Acetyl- oder Propionylrest bedeutet,

$R_4$ Wasserstoff, einen Acetyl- oder Propionylrest oder einen geradkettigen oder verzweigten Alkylrest $R_9$ mit 1 bis 4 Kohlenstoffatomen darstellt,

$R_5$ für eine Gruppe der Formel $-COOR_{10}$, worin $R_{10}$ ein Wasserstoffatom, ein pharmazeutisch verträgliches, insbesondere ein einwertiges Kation, einen geradkettigen oder verzweigten Alkylrest mit 1 bis 6 Kohlenstoffatomen oder die Gruppe $-(CH_2)_2-N-[(CH_2)_p-CH_3]_2$ ist, in der p für Null oder eine der Zahlen 1 bis 3 steht, sowie von pharmazeutisch verträglichen Salzen dieser basischen Ester mit Säuren, oder für eine Gruppe der Formel

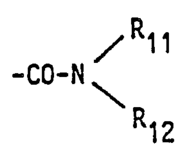

worin $R_{11}$ und $R_{12}$ gleich oder verschieden sind und für Wasserstoff, den Alkylrest $R_9$ oder für 2-Hydroxyethyl stehen oder einer dieser Reste eine Hydroxylgruppe, der andere Wasserstoff bedeutet, oder $R_{11}$ und $R_{12}$ zusammengenommen die Gruppe $-(CH_2)_q$- darstellen, in der q für eine der Zahlen 4, 5 oder 6 steht, oder für eine Gruppe der Formel

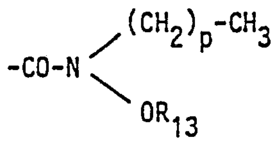

worin $R_{13}$ für Wasserstoff, $—(CH_2)_p—CH_3$, Carboxymethyl, Acetyl oder Propionyl steht und p jeweils die gleiche Bedeutung wie oben hat, oder für eine Gruppe der Formel $—CO—NH—(CH_2)_r—N\ (CH_3)_2$, worin r für eine der Zahlen 2 oder 3 steht, und deren pharmazeutisch verträgliche Salze mit Säuren, oder für eine Gruppe der Formel

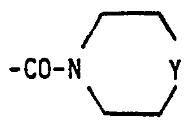

in der Y ein Sauerstoffatom oder die Gruppe $>N—CH_3$ bedeutet und gegebenenfalls deren pharmazeutisch verträgliche Salze mit Säuren, oder für einen Nitrilrest oder für eine Hydroxy-, Acetyloxy- oder Propionyloxygruppe, eine Alkoxygruppe $OR_9$ oder — zusammengenommen mit $OR_4$ — für die Methylendioxygruppe steht und

$R_6$ Wasserstoff, eine Hydroxy-, Acetyloxy- oder Propionyloxygruppe, den Alkylrest $R_9$ oder eine Alkoxygruppe $OR_9$, worin $R_9$ jeweils die gleiche Bedeutung wie oben hat, darstellt.

Wenn $R_1$ und $R_2$ nicht die gleiche Bedeutung haben, tritt an dem diese Reste tragenden Kohlenstoffatom ein Asymmetriezentrum auf. Zum Gegenstand der Erfindung gehören in diesen Fällen sowohl die Razemate als auch die optisch aktiven Formen der Verbindungen der Formel I.

In bevorzugten Verbindungen der Formel I haben die Gruppen A und B jeweils die gleiche Bedeutung und stehen insbesondere für —C≡C— oder für cis-CH=CH—. Wenn A und B verschiedene Gruppen darstellen, bedeutet B vorzugsweise —C≡C—.

$R_4$ stellt vorzugsweise ein Wasserstoffatom dar.

Diese bevorzugten Gruppen von Verbindungen der Formel I lassen sich durch folgende Formelbilder wiedergeben :

1) Wenn A und B für die Gruppe —C≡C— stehen

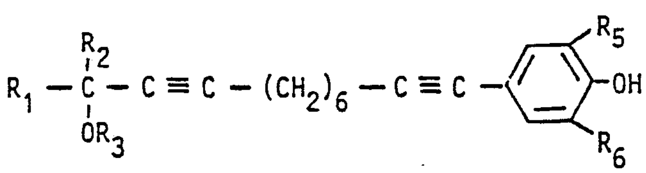

(I')

2) Wenn A und B für die Gruppe -cis-CH=CH— stehen

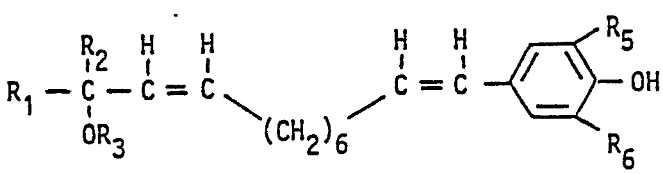

(I'')

3) Wenn A und B verschieden sind und B für die Gruppe —C≡C— steht

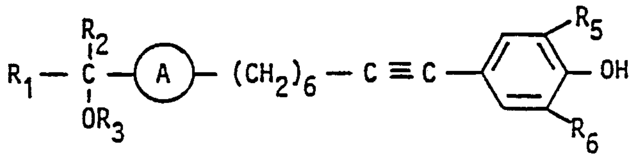

(I''')

worin jeweils $R_1$ bis $R_3$, $R_5$, $R_6$ und A die gleiche Bedeutung wie oben haben.

In besonders bevorzugten Verbindungen der Formel I bzw. Verbindungen der Formeln I', I'' und I''' stehen $R_2$ und $R_3$ für Wasserstoff, während $R_1$ darin vorzugsweise Wasserstoff, einen geradkettigen Alkylrest mit 1 bis 6 Kohlenstoffatomen oder einen Phenyl- oder Cyclohexylrest bedeutet.

Bevorzugte Bedeutungen von $R_5$ sind die Gruppen

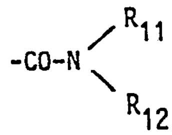 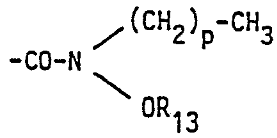 , 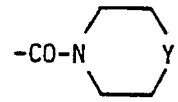

$OR_9$ (worin $R_9$, $R_{11}$, $R_{12}$, $R_{13}$ und Y die gleichen Reste wie oben darstellen, wobei $R_9$ insbesondere für Methyl und $R_{13}$ für Wasserstoff stehen) und die Hydroxygruppe.

Wie bereits einleitend ausgeführt, besitzen die Verbindungen der Formel I eine spezifische Hemmwirkung gegenüber der 5-Lipoxygenase, was durch in-vitro-Experimente ermittelt wurde.

Zur Ermittlung der 5-Lipoxygenasehemmung wurden basophile leukämische Leukozyten der Ratte in-vitro gezüchtet und nach Erreichen einer Zelldichte von ca. $10^6$ Zellen pro ml bei 400 g vom Nährmedium abzentrifugiert. Der Rückstand wurde in 50 mM Kaliumphosphatpuffer vom pH 7,4 so suspendiert, daß die Zellzahl $1{,}5 \cdot 10^7$ Zellen pro ml betrug.

Jeweils 1 ml dieser Suspension wurde mit Indomethacin (10 µM) und Calcium chlorid (2 mM) versetzt und in An- oder Abwesenheit einer der Testsubstanzen mit radioaktiv markierter Arachidonsäure und dem Calcium-Ionophor A 23 187 bei Raumtemperatur für 5 Minuten inkubiert. Nach Ansäuern auf pH 5 wurden die unter dem Einfluß der 5-Lipoxygenase gebildeten Arachidonsäuremetaboliten mit Ethylacetat extrahiert und mit einem für die Leukotriene geeigneten Fließmittelgemisch dünnschichtchromatographisch getrennt [vgl. Jakschik et al. Biochem. Biophys. Res. Commun. 102, 624 (1981)]. Die Radioaktivitätsverteilung auf die verschiedenen Metaboliten wurde mit Hilfe eines Dünnschichtscanners gemessen. Setzt man die prozentualen Bildungsanteile der 5-Lipoxygenaseprodukte (5-HETE und $LTB_4$) zu der Menge bzw. Konzentration der eingesetzten Gesamtradioaktivität bzw. der Testsubstanz der Formel I in Relation, so läßt sich der « $IC_{50}$-Wert I » (d. h. die Konzentration, die eine 50 %-ige Hemmung der 5-Lipoxygenase bewirkt) ermitteln.

Der Einfluß der Verbindungen der Formel I auf die Cyclooxygenaseaktivität wurde mit Hilfe von Schafssamenblasenmikrosomen, suspendiert in Kaliumphosphatpuffer (50 mM, pH 7,5) durch Inkubation für 10 Minuten bei Raumtemperatur mit der Testsubstanz und $^{14}C$-markierter Arachidonsäure untersucht. Nach Zugabe von Eisessig wurde mit Ethylacetat extrahiert und der eingeengte Extrakt mit Ether/Hexan/Eisessig (50 : 50 : 1) auf Kieselgeldünnschichtplatten aufgetrennt. Die Radioaktivitätsverteilung der gebildeten Prostaglandine bzw. der unveränderten Arachidonsäure wurde bestimmt und daraus der « $IC_{50}$-Wert II » (für eine 50 %-ige Hemmung der Cyclooxygenase) für die jeweilige Substanz ermittelt.

So wurden beispielsweise für die Produkte der in der folgenden Tabelle genannten Beispiele die jeweils genannten $IC_{50}$-Werte gefunden, aus denen sich der Quotient $IC_{50}II/IC_{50}I$ errechnen läßt.

| Beispiel | $IC_{50}$ [µM] für die Hemmung der 5-Lipoxygenase (I) | Cyclooxygenase (II) | Quotient |
|---|---|---|---|
| 7 | 0,15 | 38 | 253,3 |
| 9 | 0,31 | 28 | 90,3 |
| 13 | 0,35 | 31 | 88,6 |
| 14 | 0,21 | 25 | 119,1 |
| 15 | 0,32 | >100 | >320,0 |
| 16 | 0,28 | 37 | 132,1 |
| 18 | 0,1 | 38 | 380,0 |
| 22c | 2,9 | 130 | 44,8 |
| 22d | 2,8 | 180 | 64,3 |

Aus dieser Tabelle ergibt sich, daß die $IC_{50}$-Werte für die Cyclooxygenasehemmung zumeist um mehr als das 50-fache höher als die Werte für die 5-Lipoxygenasehemmung liegen, d. h. daß die Testsubstanzen sehr spezifisch nur die Hemmung der 5-Lipoxygenaseaktivität bewirken.

Aufgrund dieses günstigen Einflusses auf die Metabolisierung polyungesättigter Fettsäuren, insbesondere der Hemmwirkung auf die Bildung von Arachidonsäuremetaboliten der 5-Lipoxygenase wie 5-Hydroxyperoxyeicosatetraensäure (5-HPETE), 5-Hydroxyeicosatetraensäure (5-HETE) bzw. SRS-A bewirken die erfindungsgemäßen Verbindungen der Formel I im Organismus des Menschen bzw. von Säugetieren zahlreiche physiologisch wertvolle Effekte wie z. B. antiallergische, antianaphylaktische,

antiphlogistische, antiasthmatische, blutdrucksenkende und durchblutungsfördernde (coronarer und cerebraler Kreislauf) Wirkungen, Verminderung der Leukozytenaggregation bzw. der Bildung von Leukozytenthromben usw.

Diese therapeutisch wertvollen Eigenschaften können beispielsweise durch folgende tierexperimentell erhaltene Ergebnisse belegt werden :

Durch subplantare Injektion von 0,1 ml einer Kaolinsuspension (100 mg/ml) wird bei Sprague-Dawley-Ratten ein Ödem ausgelöst und das Pfotenvolumen plethysmometrisch stündlich gemessen.

Drei Stunden nach Ödemauslösung wird die jeweilige Prüfsubstanz, suspendiert in 5 ml 1 %-iger Natriumcarboxymethylcelluloselösung je kg Körpergewicht der Versuchstiere, intraperitoneal verabreicht. Die Änderung des Pfotenvolumens durch Ödemhemmung unter der Substanzwirkung im Vergleich zu dem unmittelbar vor Substanzgabe gemessenen Volumen ergibt sich aus der folgenden Tabelle (Werte über 100 % zeigen eine Abnahme des Ödemvolumens unter den Ausgangswert an) :

| Produkt des Beispiels | Dosis (mg/kg) | % Hemmung (Maximalwert) | Zeit nach Substanzgabe (Stunden) |
|---|---|---|---|
| 7 | 46,4 | 65 | 1 |
| | 100,0 | 164 | 1 |
| 14 | 46,4 | 68 | 1 |
| | 100,0 | 127 | 2 |
| 15 | 100,0 | 73 | 2 |
| 16 | 100,0 | 54 | 1 |

Erfolgt die Verabreichung der Testsubstanz gleichzeitig mit der Kaolinapplikation durch Injektion von 1 mg Substanz pro Pfote, gelöst in 0,1 ml 1 %-iger Natriumcarboxymethylcelluloselösung, so werden folgende Maximalwerte der Ödemhemmung bzw. der Ausbildung des Ödems gemessen :

| Produkt des Beispiels | % Hemmung (Maximalwert) | Zeit nach Substanzgabe (Stunden) |
|---|---|---|
| 7 | 36 | 2 |
| 5 | 61 | 3 |
| 14 | 46 | 2 |
| 15 | 64 | 5 |
| 16 | 45 | 7 |

Aus dieser Tabelle ergibt sich außerdem (vgl. insbesondere die nach 5 bzw. 7 Stunden für die Produkte der Beispiele 15 und 16 beobachteten Maximalwerte der Wirkung), daß die erfindungsgemäßen Verbindungen der Formel I gegen einen metabolischen Abbau recht stabil sind und daher lange wirksam bleiben.

Aufgrund dieser wertvollen Eigenschaften hinsichtlich Stabilität und Wirkung sind die Verbindungen der Formel I z. B. als Antiallergica, Antianaphylaktica, Antiphlogistica, Antiasthmatica, Antihypertensiva, antithrombotische Mittel, Mittel zur Prophylaxe oder Therapie von ischämischem Herzinfarkt, Störungen der coronaren und/oder cerebralen Arterien usw. geeignet.

Die erfindungsgemäßen Verbindungen besitzen nur eine geringe Toxizität, die sich erst bei weit höheren als den therapeutisch oder prophylaktisch anzuwendenden Dosierungen bemerkbar macht. Sie können daher als solche in geeigneten pharmazeutischen Zubereitungen an Mensch oder Tier verabreicht werden.

Die Erfindung betrifft dementsprechend auch Arzneimittel, die als Wirkstoff eine oder mehrere der erfindungsgemäßen Verbindungen der Formel I enthalten. Die an den Patienten zu verabreichende Wirkstoffmenge variiert in Abhängigkeit z. B. vom Gewicht des Patienten, vom Applikationsweg, der Indikation und dem Schweregrad der Erkrankung. Unter Berücksichtigung dieser Faktoren beträgt der Wirkstoffgehalt pro Einzeldosis im allgemeinen etwa 0,01-50 mg, und zwar bei Zubereitungsformen für die parenterale Applikation 0,01 bis 10 mg, bei Zubereitungen für orale oder rektale Applikation etwa 0,1 bis 50 mg.

Arzneimittel zur parenteralen Applikation können sowohl Lösungen als auch Suspensionen darstellen, es kommen aber auch leicht rekonstituierbare Trockenzubereitungen in Betracht.

Gut geeignete Applikationsformen sind auch Sprays zur intranasalen oder oralen Applikation bzw. zur Verabreichung der Substanzen über die Bronchien.

Für viele prophylaktische oder therapeutische Anwendungen kommen zweckmäßig auch oral anwendbare Zubereitungsformen der Verbindungen der Formel I, wie Tabletten, Dragees, Kapseln, Granulate, Tropfen und Säfte bzw. Sirupe, aber auch Suppositorien sowie perkutane Applikationszubereitungen (wie z. B. die Wirkstoffe in einem Depot in gelöster Form, gegebenenfalls unter Zusatz von die Hautpenetration fördernden Mitteln enthaltende Pflaster oder dergleichen) in Betracht. Vorteilhaft werden diese oral, rektal oder perkutan anwendbaren Zubereitungsformen so hergestellt, daß daraus der Wirkstoff verzögert freigesetzt wird, um so über einen längeren Zeitraum (beispielsweise 24 Stunden) eine gleichförmige Versorgung des Patienten mit dem Wirkstoff zu gewährleisten.

Alle vorstehend erwähnten pharmazeutischen Zubereitungsformen sind an sich bekannt, und da die erfindungsgemäßen Verbindungen der Formel I chemisch recht stabil sind, wirft ihre Einarbeitung in diese Zubereitungsformen für den Fachmann keinerlei Probleme auf. Bei dieser erfindungsgemäßen Herstellung dieser Arzneimittel muß selbstverständlich mit der üblichen Sorgfalt bei Auswahl der Hilfsstoffe wie Trägermaterialien, Farbstoffe, Geschmackskorrigentien, Bindemitteln, Tablettensprengmitteln usw. vorgegangen werden und insbesondere bei der Herstellung von parenteral anzuwendenden Zubereitungsformen ist auf Sterilität und — wenn sie in flüssiger Form vorliegen — Isotonie zu achten.

Die erfindungsgemäße Herstellung der Verbindungen der Formel I kann in der Weise erfolgen, daß man

A) zunächst eine Verbindung der allgemeinen Formel II

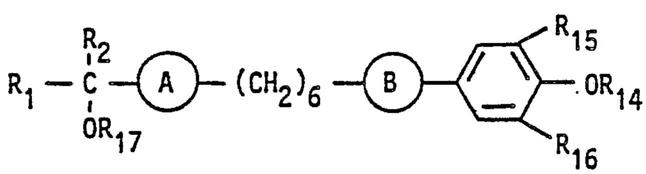

(II)

in der A, B, $R_1$ und $R_2$ die gleiche Bedeutung wie in Formel I haben, $R_{14}$ für den Alkylrest $R_9$ oder für eine unter milden Bedingungen abspaltbare Schutzgruppe, wie einen Tetrahydropyranyl-2-rest oder insbesondere eine tert-Butyldimethyl- oder -diphenylsilylgruppe, steht, $R_{15}$ für die Gruppe $OR_{14}$ steht oder die gleiche Bedeutung wie $R_5$ mit der Maßgabe hat, daß darin $R_{10}$ nicht für ein Kation stehen kann und die in $R_5$ gegebenenfalls vorhandenen basischen Gruppen nicht in Salzform vorliegen können oder $R_{15}$ zusammengenommen mit der Gruppe $OR_{14}$ für eine Methylendioxygruppe steht, $R_{16}$ ein Wasserstoffatom oder eine der Gruppen $R_9$ oder $OR_{14}$ bedeutet und $R_{17}$ eine unter milden Bedingungen abspaltbare Schutzgruppe (wie z. B. eine der in der Definition für $R_{14}$ genannten) ist,

1) durch Reaktion einer Verbindung der Formel

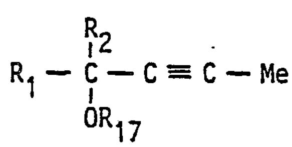

(III)

worin $R_1$, $R_2$ und $R_{17}$ die gleiche Bedeutung wie oben haben und Me für ein Lithium-, Natrium- oder Kaliumatom oder einen der Reste —MgBr oder —MgI steht, mit einer Verbindung der Formel

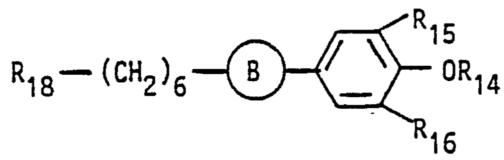

(IV)

in der B und $R_{14}$ bis $R_{16}$ die gleiche Bedeutung wie oben haben und $R_{18}$ für ein Brom- oder ein Jodatom steht, gegebenenfalls in Gegenwart von katalytischen Mengen von Kupfer-(I)-halogeniden, Kupfer-(I)-cyanid oder sonstigen Kupfersalzen herstellt. Als Lösungsmittel werden inerte Lösungsmittel wie aliphatische Kohlenwasserstoffe oder vorzugsweise wasserfreie Ether wie Diethyl- oder Diisopropylether, Tetrahydrofuran, Dioxan usw. verwendet. Insbesondere wenn Me für ein Lithiumatom steht, wird zweckmäßig zu einer Lösung der Verbindung der Formel III in einem der genannten Lösungsmittel langsam eine Lösung der Verbindung der Formel IV in Hexamethylphosphorsäuretriamid, 1,3-Dimethyl-tetrahydro-2-(1H)-pyrimidinon, 1,3-Dimethyl-2-imidazolidinon oder z. B. in N,N,N',N'-Tetraethylsulfamid bzw. entsprechend dipolaren aprotischen Kosolventien zugegeben.

Die Reaktion wird bei Temperaturen von etwa —80° bis +75 °C, vorzugsweise, wenn Me ein Lithium-, Natrium- oder Kaliumatom ist, bei —80° bis 0 °C und, wenn Me einen Rest —MgBr oder —MgI bedeutet, bei —5° bis +25 °C durchgeführt.

Insbesondere wenn der Rest $R_{15}$ für die Gruppe $OR_{14}$ steht, kann man auf den Zusatz polarer Kosolventien verzichten und bei höherer Temperatur, wie z. B. bei der Siedetemperatur des verwendeten Lösungsmittels arbeiten.

2) Steht in der Verbindung der Formel IV der Rest $R_{15}$ für die Gruppe $OR_{14}$, so läßt sich die Herstellung der Verbindung der Formel II auch durch deren Umsetzung mit einer Verbindung der Formel

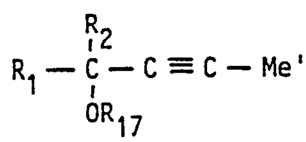

(IIIa)

worin $R_1$, $R_2$ und $R_{17}$ die gleiche Bedeutung wie oben haben und Me' für ein Lithium-, Natrium- oder Kaliumatom steht, in flüssigem Ammoniak bewirken.

3) Die Verbindung der Formel II kann auch erhalten werden, indem man eine Verbindung der Formel

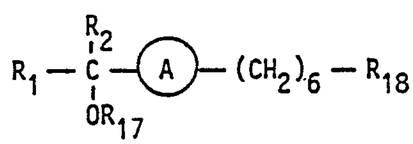

(V)

in der A, $R_1$, $R_2$, $R_{17}$ und $R_{18}$ die gleiche Bedeutung wie oben haben, mit einer Verbindung der Formel

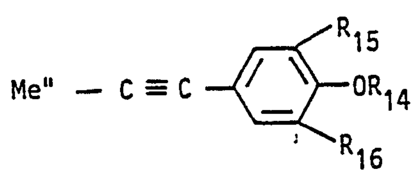

(VI)

worin $R_{14}$ bis $R_{16}$ die gleiche Bedeutung wie oben haben und Me" für eine der oben definierten Gruppen Me oder Me' steht unter den vorstehend bei 1 bzw. 2 genannten Bedingungen umsetzt.

4) Verbindungen der Formel II, in denen A für —CH=CH— steht, kann man auch dadurch erhalten, daß man auf eine Verbindung der Formel

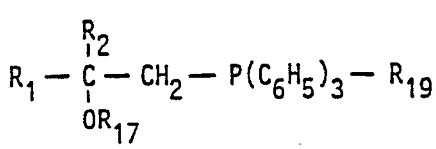

(VII)

in der $R_1$, $R_2$ und $R_{17}$ die gleiche Bedeutung wie oben haben und $R_{19}$ für ein Chlor-, Brom- oder Jodatom steht, in Gegenwart von Lösungsmitteln wie Tetrahydrofuran, n-Hexan, Benzol, Dimethylformamid, gegebenenfalls unter Zusatz von Hexamethylphosphorsäuretriamid oder z. B. Dimethylsulfoxid eine starke, wasserfreie Base, wie n-Butyllithium, Kalium-tert-butylat oder z. B. Natrium-bis-(trimethylsilyl)amid einwirken läßt, wobei unter Abspaltung von $HR_{19}$ das entsprechende Phosphoran entsteht, welches dann durch Reaktion mit einem Aldehyd der Formel

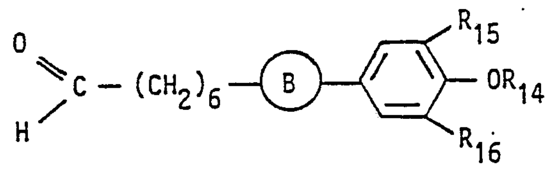

(VIII)

worin B und $R_{14}$ bis $R_{16}$ die gleiche Bedeutung wie oben haben, bei Temperaturen von etwa —80° bis +30 °C die betreffende Verbindung der Formel II liefert.

Das gleiche Produkt ist auch durch Reaktion des durch analoge Baseneinwirkung auf die Verbindung der Formel

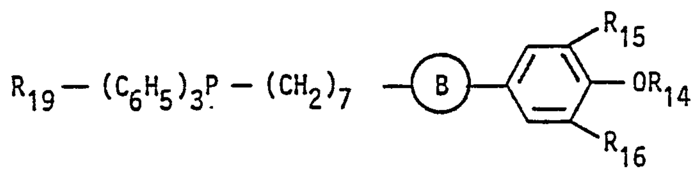

(IX)

worin B, $R_{14}$ bis $R_{16}$ und $R_{19}$ die gleiche Bedeutung wie oben haben, gebildeten Phosphorans mit einem Aldehyd der Formel

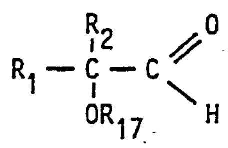

(X)

worin $R_1$, $R_2$ und $R_{17}$ die gleiche Bedeutung wie oben haben, zugänglich.

5) In Analogie zum vorstehend bei A,4) beschriebenen Vorgehen erhält man Verbindungen der Formel II, in denen B für —CH=CH— steht, durch Reaktion eines aus der Verbindung der Formel

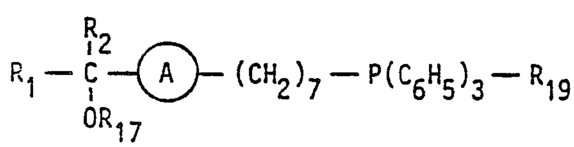

(XI)

worin A, $R_1$, $R_2$, $R_{17}$ und $R_{19}$ die gleiche Bedeutung wie oben haben durch Baseneinwirkung gebildeten Phosphorans mit einem Aldehyd der Formel

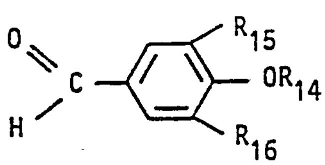

(XII)

oder durch Umsetzung des durch Baseneinwirkung auf die Verbindung der Formel

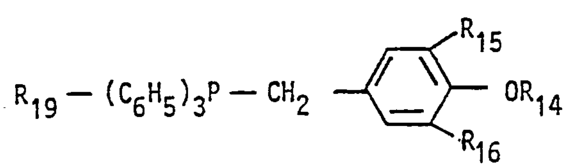

(XIII)

worin $R_{14}$ bis $R_{16}$ und $R_{19}$ die gleiche Bedeutung wie oben haben erhaltenen Phosphorans mit einem Aldehyd der Formel

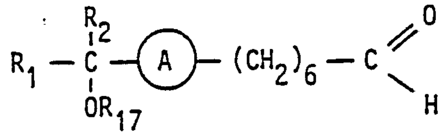

(XIV)

in der A, $R_1$, $R_2$ und $R_{17}$ die gleiche Bedeutung wie oben haben unter den bei A4 genannten Bedingungen.

Aus der so erhaltenen Verbindung der Formel II spaltet man dann in an sich bekannter Weise die in $R_{14}$ bis $R_{17}$ enthaltenen Schutzgruppen ab, wobei dies aufgrund unterschiedlicher Abspaltungsmöglichkeiten auch selektiv erfolgen kann. So kann die Abspaltung einer Tetrahydropyranyl-2-gruppe vorzugsweise in methanolischer oder ethanolischer Lösung unter Zusatz katalytischer Mengen von Pyridinium-toluol-4-sulfonat bei etwa 50°-60 °C erfolgen. Liegen als Schutzgruppen z. B. die tert-Butyldimethyl- oder -diphenylsilylgruppe vor, so kann man diese Gruppe vorzugsweise durch Einwirkung von Tetra-n-butylammoniumfluorid in einem inerten Lösungsmittel wie Tetrahydrofuran, Dioxan, Diethylether Dichlormethan etc. oder von Chlorwasserstoff, gelöst in Methanol, auf die Verbindung der Formel II bei Raumtemperatur abspalten.

Anschließend wird dann, wenn wenigstens einer der Reste $R_3$ oder $R_4$ für Acetyl oder Propionyl bzw. wenigstens einer der Reste $R_5$ und $R_6$ für Acetyloxy- oder Propionyloxy stehen soll, der betreffende Säurerest in üblicher Weise, beispielsweise durch Behandlung mit einer Lösung von Essigsäure- oder Propionsäureanhydrid in Pyridin oder durch Umsetzung mit Acetyl- oder Propionylchlorid in Gegenwart eines säurebindenden Mittels, eingeführt.

B) Bei einem bevorzugten Verfahren zur Herstellung von Verbindungen der Formel I bringt man eine Verbindung der Formel

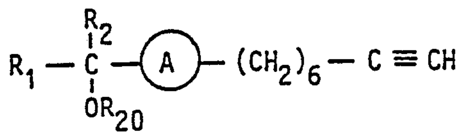

(XV)

worin A, $R_1$ und $R_2$ die gleiche Bedeutung wie oben haben und $R_{20}$ für ein Wasserstoffatom steht oder die gleiche Bedeutung wie $R_{17}$ hat in Gegenwart eines bei etwa —10° bis + 80 °C flüssigen sekundären oder tertiären Amins, insbesondere eines Dialkyl- oder Trialkylamins mit 2 oder 3 Kohlenstoffatomen in jedem der Alkylreste, Pyrrolidin oder Piperidin unter Zugabe katalytischer Mengen (darunter sollen Mengen von etwa 0,01 bis 1 Prozent pro Mol eingesetzter Verbindung der Formel XV verstanden werden) eines komplexen Palladiumkatalysators, insbesondere Bis-(triphenylphosphin)-palladium-(II)-chlorid oder -acetat oder Tetrakis-(triphenylphosphin)-palladium, und gegebenenfalls unter Zusatz katalytischer Mengen Kupfer-(I)-jodid bei etwa 0° bis 75 °C mit einer Verbindung der Formel

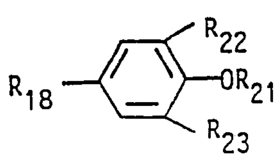

(XVI)

worin $R_{18}$ die gleiche Bedeutung wie oben hat, $R_{21}$ ein Wasserstoffatom, einen Alkylrest $R_9$ oder eine unter milden Bedingungen abspaltbare Schutzgruppe (wie z. B. eine der für $R_{14}$ genannten) bedeutet, $R_{22}$ für die Gruppe $OR_{21}$ steht oder die gleiche Bedeutung wie $R_5$ mit der Maßgabe hat, daß darin $R_{10}$ nicht für ein Kation stehen kann und die in $R_5$ gegebenenfalls vorhandenen basischen Gruppen nicht in Salzform vorliegen können, oder $R_{22}$ zusammengenommen mit der Gruppe $OR_{21}$ für eine Methylendioxygruppe steht und $R_{23}$ die gleiche Bedeutung wie $R_{16}$ hat oder für eine Hydroxylgruppe steht, zur Reaktion. Man erhält so eine Verbindung der Formel

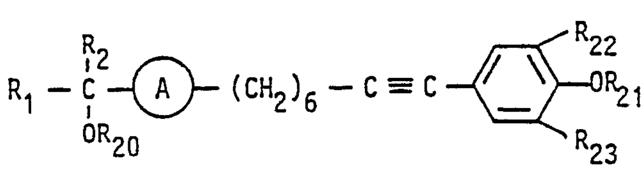

(XVII)

aus der in der für die Überführung der Verbindung der Formel II in die Verbindung der Formel I beschriebenen Weise die gegebenenfalls in $R_{20}$ bis $R_{23}$ enthaltenen Schutzgruppen abgespalten werden, wobei dies auch hier gegebenenfalls selektiv erfolgen kann. Anschließend wird, wenn wenigstens einer der Reste $R_3$ oder $R_4$ für den Acetyl- oder Propionylrest bzw. wenigstens einer der Reste $R_5$ und $R_6$ für den Acetyloxy- oder den Propionyloxyrest stehen soll, dieser Rest in üblicher Weise, beispielsweise wie oben bei A) beschrieben, eingeführt.

C) Verbindungen der Formel I kann man weiterhin dadurch erhalten, daß man eine Verbindung der Formel

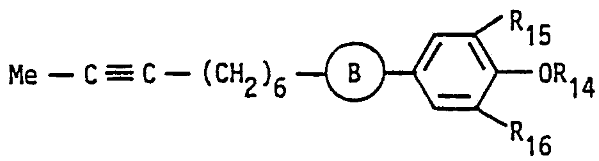

(XVIII)

worin Me, B und $R_{14}$ bis $R_{16}$ die gleiche Bedeutung wie oben haben, in Gegenwart eines indifferenten Lösungsmittels, wie Tetrahydrofuran, Diethylether oder z. B. n-Hexan, bei etwa —80° bis +30 °C mit einer Verbindung der Formel

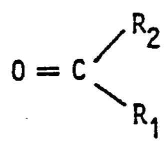

(XIX)

in der $R_1$ und $R_2$ die gleiche Bedeutung wie oben haben zu einer Verbindung der Formel

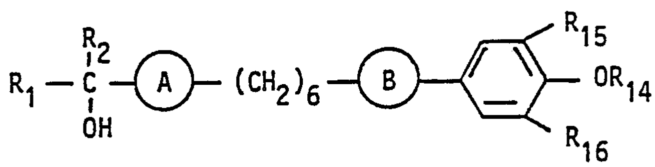

(XX)

worin A, B, $R_1$, $R_2$ und $R_{14}$ bis $R_{16}$ die gleiche Bedeutung wie oben haben umsetzt und in der oben beschriebenen Weise die gegebenenfalls in $R_{14}$ bis $R_{16}$ enthaltenen Schutzgruppen abspaltet. Wenn in der Verbindung der Formel I wenigstens einer der Reste $R_3$ und $R_4$ bzw. $R_5$ und $R_6$ einen Acetyl- oder Propionylrest darstellen bzw. enthalten soll, wird dieser anschließend in üblicher Weise eingeführt.

D) Zur Herstellung von Verbindungen der Formel I, in denen $R_1$ von Wasserstoff verschieden ist, kann man so vorgehen, daß man zunächst eine Verbindung der Formel

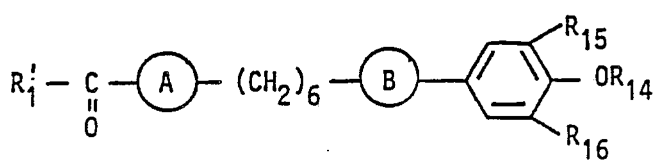

(XXI)

in der A, B und $R_{14}$ bis $R_{16}$ die gleiche Bedeutung wie oben haben und $R'_1$ mit Ausnahme von Wasserstoff die gleiche Bedeutung wie $R_1$ hat, dadurch herstellt, daß man

1) eine Verbindung der Formel

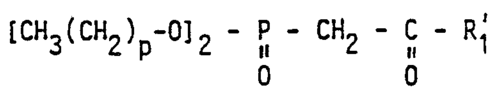

(XXII)

worin p und $R'_1$ die gleiche Bedeutung wie oben haben, wobei p vorzugsweise für Null steht, mit einer wasserfreien Base, wie Natriumhydrid oder n-Butyllithium, in einem inerten Lösungsmittel, wie Tetrahydrofuran, Dimethoxyethan, n-Hexan oder Toluol, behandelt und dann bei Temperaturen von etwa —10 °C bis + 35 °C, vorzugsweise 0 °C bis 10 °C, mit einer Verbindung der Formel VIII umsetzt oder

2) eine Verbindung der Formel

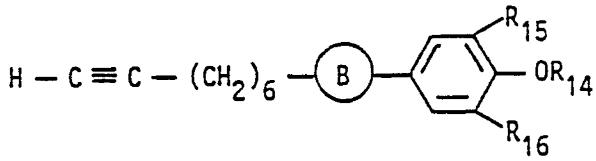

(XXIII)

in der B und $R_{14}$ bis $R_{16}$ die gleiche Bedeutung wie oben haben, in Gegenwart eines oberhalb etwa + 80°C siedenden tertiären Amins, insbesondere eines Trialkylamins mit 2 oder 3 Kohlenstoffatomen in jedem der Alkylreste unter den bei B für die Reaktion zwischen den Verbindungen der Formel XV und XVI beschriebenen Bedingungen mit einer Verbindung der Formel

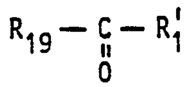

(XXIV)

worin $R'_1$ und $R_{19}$ die gleiche Bedeutung wie oben haben, umsetzt. Aus der auf einem dieser Wege hergestellten Verbindung der Formel XXI erhält man dann durch Reduktion oder durch Reaktion mit einer Verbindung der Formel $R'_2$-Me, worin Me die gleiche Bedeutung wie oben hat und $R'_2$ mit Ausnahme von Wasserstoff die gleiche Bedeutung wie $R_2$ hat, anschließende Abspaltung von in $R_{14}$ bis $R_{16}$ vorliegenden Schutzgruppen und gegebenenfalls — wenn wenigstens einer der Reste $R_3$ und $R_4$ bzw. $R_5$ und $R_6$ einen Acetyl- oder Propionylrest darstellen bzw. enthalten soll — Acylierung, die vor oder nach Abspaltung der Schutzgruppen aus $R_{14}$-$R_{16}$ erfolgen kann, die entsprechende Verbindung der Formel I (in der $R_1$ und gegebenenfalls $R_2$ nicht für Wasserstoff stehen können).

Die Reduktion der Verbindung der Formel XXI erfolgt mit Metallborhydriden, wie Zinkborhydrid oder insbesondere Natriumborhydrid, vorzugsweise unter Zusatz von Cer (III)-chlorid in Methanol, Ethanol oder auch Dimethoxyethan unter Zusatz von Wasser bei Temperaturen von etwa — 20° bis + 30 °C, bevorzugt bei etwa + 20 °C.

Gewünschtenfalls kann man in den nach A, B, C oder D erhaltenen Verbindungen der Formel I vorhandene Dreifachbindungen (in A und/oder B) zu Doppelbindungen hydrieren. Diese Hydrierung erfolgt im allgemeinen mit katalytisch angeregtem Wasserstoff unter Normaldruck bei Raumtemperatur unter Verwendung insbesondere von Palladiumkatalysatoren, vorzugsweise « Lindlar-Katalysatoren » wie z. B. mit Blei vergiftetem Palladium auf Calciumcarbonat. Dabei werden als Lösungsmittel in an sich bekannter Weise z. B. n-Hexan, Methanol, Ethanol, Essigsäureethylester, Benzol, Toluol, Diisopropylether, zweckmäßig unter Zusatz von 0,1-2 % Chinolin bzw. Pyridin oder reines Pyridin verwendet.

Steht in der gewonnenen Verbindung der Formel I der Rest $R_5$ für die Gruppe $COOR_{10}$, wobei $R_{10}$ der Alkylrest $R_9$ oder die Gruppe $—(CH_2)_2—N[(CH_2)_p—CH_3]_2$ ist, so kann diese Estergruppierung anschliessend noch in an sich bekannter Weise z. B. zur freien Säure (gegebenenfalls gefolgt von einer Salzbildung durch Neutralisation mit einer in Salzform pharmazeutisch verträglichen Base, wie z. B. verdünnter Natronoder Kalilauge) verseift werden, wobei dieser Ester mit einer wässrigen Lösung von z. B. Natrium-, Kalium- oder Lithiumhydroxid, zweckmäßig bei Raumtemperatur in Gegenwart eines mit Wasser mischbaren Lösungsmittels wie Methanol, Ethanol oder Tetrahydrofuran behandelt werden.

Andererseits lassen sich aus den zunächst erhaltenen Estern durch Umsetzung mit Aminen der Formeln

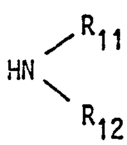 , $H_2N-(CH_2)_r-N(CH_3)_2$ oder 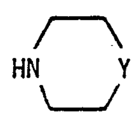

worin $R_{11}$, $R_{12}$, r und Y die gleiche Bedeutung wie oben haben, bzw. mit Hydroxylaminabkömmlingen der Formel

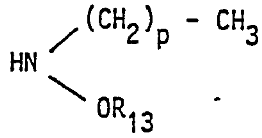

worin p und $R_{13}$ die gleiche Bedeutung wie oben haben, die entsprechenden Amide bzw. Hydroxamsäuren gewinnen, die andererseits natürlich in an sich bekannter Weise auch aus den freien Carbonsäuren ($R_5$ = COOH) unter Zuhilfenahme wasserabspaltender Mittel wie Dicyclohexylcarbodiimid, Dimethylformamid/Thionylchlorid und dergleichen oder durch intermediäre Bildung reaktiver funktioneller Derivate des Carbonsäurerestes, wie z. B. gemischter Anhydride, Säurehalogenide usw., zugänglich sind.

Enthält in einer auf einem der obigen Wege gewonnenen Verbindung der Formel I der Rest $R_5$ eine basische Gruppe, so läßt sich diese leicht durch Neutralisation mit einer geeigneten Säure in an sich bekannter Weise in ein pharmazeutisch verträgliches Salz mit dieser Säure überführen. Erfolgte diese Salzbildung in einem Lösungsmittel, in dem das entstehende Salz löslich ist, so erfolgt dessen Isolierung zweckmäßig durch Gefriertrocknung, obwohl natürlich auch das Ausfällen durch Zugabe von geeigneten, mit dem Lösungsmittel mischbaren Flüssigkeiten, in denen das Salz unlöslich ist, zur Isolierung des Salzes dienen kann. Vorzugsweise löst man aber die freie Base in einem wasserfreien, wenig polaren Mittel, wie z. B. Diethylether, gibt eine etherische Lösung der Säure zu und versetzt gegebenenfalls zur Einleitung der Kristallisation beispielsweise mit n-Hexan oder Petrolether. Auch in diesem Falle ist andererseits selbstverständlich die Isolierung des Salzes durch Eindampfen, vorzugsweise im Vacuum, möglich. Geeignete Säuren für diese Salzbildung sind z. B. Salzsäure, Bromwasserstoffsäure, Schwefelsäure, Phosphorsäure, Ameisensäure, Essigsäure, Benzoesäure, Salicylsäure, Benzolsulfonsäure und weitere zur Herstellung pharmazeutisch anwendbarer Salze gebräuchliche Säuren.

Weitere Umwandlungsmöglichkeiten des Restes $R_5$ liegen für den Fachmann auf der Hand. Beispielsweise kann die freie Carboxylgruppe (insbesondere wenn $R_3$ und $R_4$ von Wasserstoff verschieden sind und $R_6$ nicht für eine Hydroxylgruppe steht) leicht durch Behandlung mit Diazomethan oder Diazoethan in die Carboxymethyl- bzw. Carboxyethylgruppe übergeführt werden.

Zweckmäßig geht man bei den im Rahmen dieser Anmeldung beschriebenen Syntheseverfahren von jeweils solchen Ausgangsmaterialien aus, in denen gegebenenfalls vorhandene Doppelbindungen bereits in der im Endprodukt gewünschten Konfiguration vorliegen. Dadurch wird die Isomerentrennung in den Fällen, in denen in der letzten Stufe eine weitere Doppelbindung eingeführt wird, diese aber nicht mit einheitlicher Konfiguration anfällt, erleichtert. Die Isomerentrennung kann in an sich bekannter Weise, z. B. durch Säulenchromatographie, erfolgen.

Sind $R_1$ und $R_2$ voneinander verschieden, so läßt sich eine gegebenenfalls gewünschte Razemattrennung in üblicher Weise durchführen. Z. B. kann man diese, wenn $R_5$ eine basische Gruppe enthält bzw. für eine freie Carboxylgruppe steht, durch Salzbildung mit optisch aktiven Säuren bzw. Basen in an sich bekannter Weise erreichen.

Die Darstellung der Ausgangsverbindungen für die oben beschriebenen Vorgehensweisen zur Herstellung der Verbindungen der Formel I erfolgt durchweg in an sich bekannter Weise. Die dafür in Betracht zu ziehenden Wege werden insbesondere auch unter Berücksichtigung der verschiedenen Bedeutungen von A bzw. B gewählt.

I Z. B. können die Verbindungen der Formel VI und daraus die der Formel IV dadurch gewonnen werden, daß man auf einen Aldehyd der Formel XII Chlormethylen-triphenyl-phosphoran (in situ erzeugt aus Chlormethyltriphenylphosphoniumchlorid durch Einwirkung von z. B. n-Butyllithium/Dimethylsulfoxid in Tetrahydrofuran) einwirken läßt und dann aus der entstandenen Verbindung z. B. mit n-Butyllithium Chlorwasserstoff abspaltet. Das erhaltene Phenylacetylenderivat wird dann durch Umsetzung z. B. mit Butyllithium, Natrium- oder Kaliumhydrid, Ethylmagnesiumbromid oder -jodid in die Verbindung der Formel VI übergeführt. Durch deren Umsetzung mit 1,6-Dibrom- oder 1,6-Dijodhexan z. B. in Diethylether oder Tetrahydrofuran, vorzugsweise in Anwesenheit eines aprotischen, dipolaren Lösungsmittels, bei etwa — 80 °C, später bei 0 °C, erhält man dann die Verbindung der Formel IV.

II Eine Verbindung der Formel V, in der A für —C≡C— steht, erhält man z. B. durch Umsetzung einer Verbindung der Formel III mit 1,6-Dibrom- oder 1,6-Dijod-hexan unter den vorstehend bei I für die Umsetzung der Verbindung der Formel VI mit einer dieser Halogenverbindungen beschriebenen Bedingungen.

III Durch Umsetzung einer Verbindung der Formel VI unter den oben bei I beschriebenen Bedingungen mit einer Verbindung der Formel

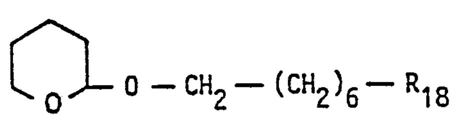

(XXV)

Abspalten der Tetrahydropyranyl-2-Gruppe und Oxydation des so erhaltenen Alkohols der Formel

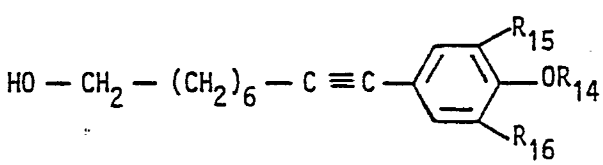

(XXVI)

mit z. B. Pyridiumchlorochromat in Dichlormethan erhält man die Verbindung der Formel VIII.

Wird in der Verbindung der Formel XXVI die Hydroxylgruppe z. B. durch Umsetzung mit Tetrabrommethan in Gegenwart von Triphenylphosphin gegen ein Bromatom ausgetauscht und das Produkt anschließend mit Triphenylphosphin zur Reaktion gebracht (beispielsweise durch 12- bis 24-stündiges Kochen in Acetonitril unter Rückfluß), so erhält man eine Verbindung der Formel IX, in der $R_{19}$ für Brom steht.

IV Setzt man eine Verbindung der Formel III mit 1,7-Dibromheptan unter den bei I genannten Bedingungen um und behandelt die entstandene Verbindung der Formel

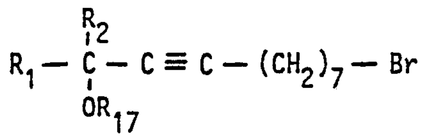 (XXVII)

mit Triphenylphosphin (z. B. Erhitzen in Acetonitril), so erhält man die entsprechende Verbindung der Formel XI, in der $R_{19}$ für Brom steht.

V Zur Herstellung einer Verbindung der Formel XIV, in der A für —C≡C— steht, setzt man zunächst eine Verbindung der Formel III, in der $R_{17}$ nicht für die Tetrahydropyranyl-2-gruppe stehen sollte, mit einer Verbindung der Formel XXV unter den bei I beschriebenen Bedingungen um und spaltet die Tetrahydropyranyl-2-Gruppe ab, wobei man einen Alkohol der Formel

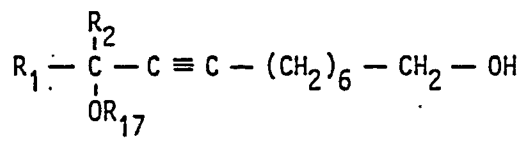 (XXVIII)

erhält. Dieser liefert bei Oxydation (z. B. mit Pyridiniumdichromat in Dimethylformamid oder Dichlormethan) die Verbindung der Formel XIV.

VI Eine Verbindung der Formel XV, in der A für —C≡C— und $R_{20}$ für Wasserstoff steht, läßt sich durch Umsetzung einer Verbindung der Formel

$$HC{\equiv}C{-}(CH_2)_6{-}C{\equiv}C{-}Me \qquad (XXIX)$$

mit einer Verbindung der Formel XIX in wasserfreien Ethern wie Tetrahydrofuran bei Temperaturen von etwa — 80 °C bis + 65 °C erhalten.

VII Zur Herstellung von Verbindungen der Formel XVIII geht man von dem Aldehyd der Formel VIII aus und erhält daraus durch Umsetzung mit Chlormethylen-triphenylphosphoran, gefolgt von Chlorwasserstoffabspaltung aus dem erhaltenen Produkt entsprechend der bei I beschriebenen Methodik und schließliche Behandlung der so erhaltenen Verbindung der Formel XXIII mit Butyllithium, Natrium- oder Kaliumhydrid oder z. B. Ethylmagnesiumbromid oder -jodid die gewünschte Verbindung der Formel XVIII.

Die Verbindung der Formel XXIII ist andererseits auch aus der Verbindung der Formel IV durch Umsetzung mit Lithiumacetylid-Ethylendiamin-Komplex in Dimethylsulfoxid oder Dimethylformamid bei etwa 5° bis 25 °C zugänglich.

Weitere Wege zur Herstellung von Ausgangsverbindungen für das erfindungsgemäße Verfahren ergeben sich für den Fachmann in naheliegender Weise. Darüberhinaus sind die Ausgangsmaterialien zum Teil auch literaturbekannt oder im Handel zugänglich.

Die folgenden Beispiele erläutern das erfindungsgemäße Verfahren sowie die Herstellung von neuen Ausgangsmaterialien und Zwischenprodukten weiter. Bei ihrer Durchführung wurde auf die Erzielung maximaler Ausbeuten kein Wert gelegt. Alle Temperaturangaben sind unkorrigiert.

Die Produkte liegen in Form von Ölen vor, soweit im Einzelfall keine anderen Angaben gemacht werden.

Die $^1H$—NMR-Spektren wurden bei 60 MHz aufgenommen. Die chemische Verschiebung der spektroskopischen Resonanzdaten wurde in ppm gemessen.

Die in den Beispielen verwendete n-Butyllithiumlösung enthielt — gelöst in n-Hexan — 1,6 Mol n-Butyllithium/Liter. Es können aber selbstverständlich auch Lösungen anderer Konzentrationen und/oder mit anderen Lösungsmitteln als n-Hexan eingesetzt werden.

Soweit nicht anders angegeben, handelt es sich bei dem in den Beispielen benutzten Ether um den Diethylether, bei dem Petrolether um denjenigen mit einem Siedebereich von 50°-70 °C.

Für die Chromatographie bzw. Säulenchromatographie wurde als stationäre Phase — sofern nicht anders erwähnt — Kieselgel 60 (0,040-0,063 mm) der Firma Macherey-Nagel benutzt. Für die HPLC wurde

ein von der gleichen Firma unter der Bezeichnung « Nucleosil C 18 (10 µm) » vertriebenes Kieselgel als stationäre Phase eingesetzt.

Die dünnschichtchromatographische Verfolgung des Reaktionsverlaufs wurde mit « HPTLC Fertigplatten, Kieselgel 60 F 254 » der Firma E. Merck, Darmstadt, durchgeführt. Das benutzte Fließmittel wird in den Beispielen jeweils mit « (DC : ...) » angegeben.

Die Mischungsverhältnisse der Laufmittel für alle chromatographischen Untersuchungen sind stets in Volumen/Volumen angegeben.

In den Beispielen anfallende Enantiomerengemische wurden — soweit nicht anders erwähnt — nicht aufgetrennt. Die angegebenen Daten beziehen sich in solchen Fällen also auf die Enantiomerengemische.

Beispiel 1

1-(4'-Hydroxy-3'-methoxycarbonylphenyl)-11-hydroxy-undeca-1,9-diin

a) Undeca-2,10-diin-1-ol

13,42 g Deca-1,9-diin in 300 ml absolutem Tetrahydrofuran werden bei —40 °C unter Rühren und Überleiten von trockenem Stickstoff innerhalb einer Stunde tropfenweise mit 59,4 ml n-Butyllithiumlösung versetzt. Man rührt eine Stunde nach und läßt dabei die Temperatur auf 0 °C ansteigen, versetzt dann mit 6,0 g Paraformaldehyd und erhitzt zum Rückfluß. (DC : Petrolether/Ether — 3 : 2). Nach Reaktionsende wird mit gesättigter Ammoniumchloridlösung zersetzt, mehrmals mit Ether extrahiert, der Extrakt über Natriumsulfat getrocknet und im Vakuum eingedampft. Durch Säulenchromatographie des Rückstandes mit Petrolether/Ether (3 : 2) erhält man 8,27 g der Titelverbindung als eine im Kühlschrank allmählich erstarrende Flüssigkeit.

$^1$H—NMR ($CDCl_3$) : 1,27-1,80 (m, 9 H) ; 1,85-2,00 (t, 1 H) ; 2,00-2,40 (m, 4 H) ; 4,10-4,30 (t, 2 H).

b) 1-(4'-Hydroxy-3'-methoxycarbonylphenyl)-11-hydroxy-undeca-1,9-diin

1,15 g des Produktes aus Beispiel 1a und 1,95 g 5-Jodsalicylsäuremethylester werden in 8 ml über Kaliumhydroxid destilliertem Triethylamin gelöst und unter Rühren mit 0,066 g Kupfer-(I)-jodid und 0,146 g Bis(triphenylphosphin)-palladium(II)-chlorid versetzt. (DC : Petrolether/Ether — 1 : 1). Nach Reaktionsende wird mit Ether verdünnt, filtriert und das Filtrat im Vakuum eingedampft. Durch Säulenchromatographie des Rückstandes mit n-Hexan/Essigsäureethylester (3 : 2) werden 1,54 g der Titelverbindung als im Kühlschrank langsam zu weißen Kristallen erstarrendes Öl erhalten.

$^1$H—NMR ($CDCl_3$) : 1,30-1,93 (m, 8 H) ; 2,05-2,60 (m, 4 H) ; 3,93 (s, 3 H) ; 4,06-4,33 (m, 2 H) ; 6,67-7,93 (m, 3 H).

Beispiel 2

1-(3'-Carboxy-4'-hydroxyphenyl)-11-hydroxy-undeca-1,9-diin

Zu 0,20 g des Produktes aus Beispiel 1, in 2 ml Methanol und 2 ml Tetrahydrofuran gelöst, gibt man 3,24 ml einer wässrigen Lösung von Lithiumhydroxid (1 Mol/l). (DC : Petrolether/Ether/Eisessig : 3 : 6 : 0,1). Nach 20 Stunden wird im Vakuum eingedampft, der Rückstand mit 3 ml Wasser verdünnt und unter Eiswasserkühlung mit Salzsäure auf pH 3 angesäuert. Man extrahiert dreimal mit Ether, wäscht mit Wasser, trocknet mit Natriumsulfat und dampft ein, wobei man 0,172 g der Titelverbindung erhält.

$^1$H—NMR ($CDCl_3$) : 1,07-1,93 (m, 8 H) ; 2,00-2,70 (m, 4 H) ; 3,97-4,30 (m, 2 H) ; 6,63-7,93 (m, 3 H).

Beispiel 3

1-(3'-Carbamoyl-4'-hydroxyphenyl)-11-hydroxy-undeca-1,9-diin

0,180 g des in Beispiel 1 erhaltenen Esters werden in 5 ml einer methanolischen Lösung von Ammoniak (11 % $NH_3$) gelöst. (DC : Petrolether/Ether/Eisessig — 3 : 6 : 0,1). Nach Reaktionsende wird im Vakuum eingedampft. Durch Säulenchromatographie des Rückstandes mit Petrolether/Ether/Eisessig (3 : 6 : 0,1) erhält man 0,132 g der Titelverbindung in Form weißer Kristalle.

$^1$H—NMR ($CDCl_3$) : 1,07-1,97 (m, 8 H) ; 2,01-2,67 (m, 4 H) ; 3,90-4,23 (m, 2 H) ; 6,56-7,77 (m, 3 H).

Beispiel 4

1-(4'-Acetoxy-3'-methoxycarbonylphenyl)-11-acetoxy-undeca-1,9-diin

0,10 g des in Beispiel 1 erhaltenen Esters werden in 1 ml absolutem Tetrahydrofuran und 0,255 ml absolutem Pyridin gelöst und unter Eiswasserkühlung tropfenweise mit 0,268 ml Essigsäureanhydrid versetzt. Man läßt auf Raumtemperatur erwärmen und dampft nach 20 Stunden im Vakuum ein. Durch

Säulenchromatographie des Rückstandes mit Petrolether/Essigsäureethylester (3 : 2) erhält man 0,120 g der Titelverbindung.

$^1H$—NMR ($CDCl_3$) : 1,20-1,93 (m, 8 H) ; 1,97-2,70 (m, 4 H) ; 2,10 (s, 3 H) ; 2,33 (s, 3 H) ; 3,87 (s, 3 H) ; 4,47-4,73 (m, 2 H) ; 6,80-8,03 (m, 3 H).

## Beispiel 5

11-Hydroxy-1-(4'-hydroxy-3'-methoxyphenyl)-undeca-1,9-diin

a) 3-Methoxy-4-(tetrahydropyranyl-2'-oxy)-benzaldehyd

Zu einer Lösung von 20 g Vanillin und 14,28 ml 3,4-Dihydro-2H-pyran in 320 ml Dichlormethan gibt man unter Rühren bei + 2 °C in 3 Portionen 0,20 g p-Toluolsulfonsäure-monohydrat. Nach 60 Minuten wird das Reaktionsgemisch mit 3 g wasserfreiem Kaliumcarbonat versetzt, 30 Minuten im Eiswasserbad verrührt, dann werden 20 ml Wasser hinzugefügt. Die Dichlormethanphase wird mit gesättigter Natriumchloridlösung zweimal gewaschen, über Natriumsulfat getrocknet und eingedampft. Das Rohprodukt wird durch Säulenchromatographie mit Petrolether/Ether (2 : 1) gereinigt. Man erhält 22,26 g der Titelverbindung als Öl, das im Kühlschrank allmählich zu weißen Kristallen erstarrt, die bei 43 °C schmelzen.

$^1H$—NMR ($CDCl_3$) : 1,33-2,27 (m, 6 H) ; 3,27-4,10 (m, 2 H) ; 3,90 (s, 3 H) ; 5,30-5,56 (m, 1 H) ; 7,02-7,43 (m, 3 H) ; 9,70 (s, 1 H).

b) 4-(2'-Chlorvinyl)-2-methoxy-1-(tetrahydropyranyl-2'-oxy)-benzol

Zu einer Suspension von 20 g Chlormethyl-triphenylphosphoniumchlorid in 70 ml absolutem Tetrahydrofuran werden unter Rühren und Überleiten von trocknem Stickstoff bei 20 °C portionsweise 6,48 g Kaliumtert-butylat gegeben. Nach 30-minütigem Rühren gibt man innerhalb von 15 Minuten eine Lösung von 9,01 g des Produktes aus Beispiel 5a in 15 ml absolutem Tetrahydrofuran tropfenweise zu und verdünnt nach weiteren 30 Minuten mit Essigsäureethylester. Dann wird je einmal mit gesättigten Lösungen von Natriumhydrogencarbonat bzw. Natriumchlorid gewaschen, über Natriumsulfat getrocknet und eingedampft. Durch Säulenchromatographie des Rohproduktes mit Petrolether/Ether (2 : 1) erhält man 8,22 g der Titelverbindung.

$^1H$—NMR ($CDCl_3$) : 1,33-2,30 (m, 6 H) ; 3,30-4,27 (m, 2 H) ; 3,87-3,90 (s,s 3 H) ; 5,20-5,56 (m, 1 H) ; 5,95-7,43 (m, 5 H).

c) 3-Methoxy-4-(tetrahydropyranyl-2'-oxy)-phenylacetylen

5,47 g des in Beispiel 5b erhaltenen Produktes werden in 28 ml absolutem Tetrahydrofuran gelöst und bei 0 °C tropfenweise mit 27,8 ml n-Butyllithiumlösung versetzt. Man rührt noch 3 Stunden bei 0 °C, zersetzt dann mit gesättigter Ammoniumchloridlösung und extrahiert mit Ether. Der Extrakt wird mit gesättigter Natriumchloridlösung gewaschen, über Natriumsulfat getrocknet und eingedampft. Säulenchromatographie des Rückstandes mit Petrolether/Ether (2 : 1) liefert 3,81 g der Titelverbindung.

$^1H$—NMR ($CDCl_3$) : 1,40-2,20 (m, 6 H) ; 2,95 (s, 1 H) ; 3,37-4,16 (m, 2 H) ; 3,87 (s, 3 H) ; 5,20-5,50 (m, 1 H) ; 6,80-7,10 (m, 3 H).

d) 1-[3'-Methoxy-4'-(tetrahydropyranyl-2"-oxy)-phenyl]-11-(tetrahydropyranyl-2'-oxy)-undeca-1,9-diin

1,31 g des Produktes aus Beispiel 5c werden in 9,5 ml absolutem Tetrahydrofuran gelöst und bei — 78 °C bis — 70 °C unter Rühren und Überleiten von trocknem Stickstoff tropfenweise mit 3,51 ml n-Butyllithiumlösung versetzt. Man rührt noch 60 Minuten im Kältebad und gibt dann tropfenweise eine Lösung von 1,72 g 1-Brom-9-(tetrahydropyranyl-2'-oxy)-non-7-in in 5,3 ml absolutem Hexamethylphosphorsäuretriamid zu. Nach etwa 4 Stunden wird das Kältebad entfernt. Sobald das Reaktionsgemisch 0 °C erreicht hat, wird mit gesättigter Ammoniumchloridlösung zersetzt und dreimal mit Ether extrahiert. Die vereinigten etherischen Extrakte werden zweimal mit gesättigter Ammoniumchloridlösung und einmal mit Wasser gewaschen, über Natriumsulfat getrocknet und dann im Vakuum eingedampft. Durch Säulenchromatographie des Rückstandes mit Petrolether/Ether (2 : 1) erhält man 1,72 g der Titelverbindung.

$^1H$—NMR ($CDCl_3$) : 1,07-2,07 (m, 20 H) ; 2,10-2,60 (m, 4 H) ; 3,27-4,07 (m, 4 H) ; 3,86 (s, 3 H) ; 4,13-4,37 (m, 2 H) ; 4,67-4,91 (m, 1 H) ; 5,33-5,51 (m, 1 H) ; 6,67-7,07 (m, 3 H).

e) Das in Beispiel 5d als eine der Ausgangsverbindungen verwendete 1-Brom-9-(tetrahydropyranyl-2'-oxy)-non-7-in wird wie folgt erhalten :

5,0 g 3-(Tetrahydropyranyl-2'-oxy)-prop-1-in werden in 70 ml absolutem Tetrahydrofuran gelöst und bei — 78 °C bis — 70 °C unter Rühren und Überleiten von trocknem Stickstoff tropfenweise mit 22,31 ml

n-Butyllithiumlösung versetzt. Man rührt noch 60 Minuten im Kältebad und gibt dann tropfenweise innerhalb von 5 Minuten 16,3 ml 1,6-Dibromhexan, anschließend innerhalb von 25 Minuten 30 ml absolutes Hexamethylphosphorsäuretriamid zu. Nach 4 Stunden wird das Kältebad entfernt und analog Beispiel 5d aufgearbeitet, wobei aber die Etherextrakte mit gesättigter Natriumchloridlösung gewaschen werden. Das Rohprodukt liefert durch Säulenchromatographie mit n-Hexan/Ether (10 : 1) 8,23 g der Titelverbindung.

$^1$H—NMR ($CDCl_3$) : 1,16-2,43 (m, 16 H) ; 3,20-4,05 (m, 4 H) ; 4,10-4,33 (m, 2 H) ; 4,61-4,90 (m, 1 H).

f) 11-Hydroxy-(4'-hydroxy-3'-methoxyphenyl)-undeca-1,9-diin

1,71 g des in Beispiel 5d erhaltenen Produktes und 0,08 g Pyridiniumtoluol-4-sulfonat werden in 34 ml absolutem Ethanol gelöst und unter trocknem Stickstoff 3 Stunden bei 55 °C bis 60 °C Badtemperatur gerührt und dann im Vakuum bei 20° bis 25 °C Badtemperatur eingedampft. Der Rückstand liefert durch Säulenchromatographie mit Petrolether/Essigsäureethylester (3 : 4) 0,726 g der Titelverbindung, die langsam in bei 71 °C schmelzende Kristalle übergeht.

$^1$H—NMR ($CDCl_3$) : 1,16-1,83 (m, 8 H) ; 1,91-2,53 (m, 4 H) ; 3,87 (s, 3 H) ; 4,20-4,37 (m, 2 H) ; 6,60-7,01 (m, 3 H).

Beispiel 6

11-Acetoxy-1-(4'-acetoxy-3'-methoxyphenyl)-undeca-1,9-diin

0,20 g des Produktes aus Beispiel 5 werden in 2 ml absolutem Tetrahydrofuran mit 0,60 ml absolutem Pyridin und 0,63 ml Essigsäureanhydrid analog Beispiel 4 umgesetzt, wobei man 0,273 g der Titelverbindung erhält.

$^1$H—NMR ($CDCl_3$) : 1,20-2,53 (m, 12 H) ; 2,05 (s, 3 H) ; 2,25 (s, 3 H) ; 3,70 (s, 3 H) ; 4,43-4,60 (m, 2 H) ; 6,60-7,10 (m, 3 H).

Beispiel 7

1-(3',4'-Dihydroxyphenyl)-11-hydroxy-undeca-1,9-diin

a) 3,4-Bis-(tetrahydropyranyl-2'-oxy)-benzaldehyd

Eine Suspension von 25 g 3,4-Dihydroxybenzaldehyd in 125 ml Dichlormethan wird unter der katalytischen Wirkung von 0,05 g p-Toluolsulfonsäure-monohydrat analog Beispiel 5a mit 39,5 ml 3,4-Dihydro-2H-pyran umgesetzt und anschließend aufgearbeitet. Nach Säulenchromatographie mit Petrolether/Ether (3 : 2) erhält man 38,42 g der Titelverbindung, die im Kühlschrank nach einiger Zeit zum Teil erstarrt.

$^1$H—NMR ($CDCl_3$) : 1,33-2,27 (m, 12 H) ; 3,20-4,23 (m, 4 H) ; 5,23-5,63 (m, 2 H) ; 6,93-7,70 (m, 3 H) ; 9,70 (s, 1 H).

b) 1,2-Bis-(tetrahydropyranyl-2'-oxy)-4-(2'-chlorvinyl)-benzol

Man verfährt analog Beispiel 5b und erhält aus 20,0 g Chlormethyltriphenylphosphoniumchlorid, 6,46 g Kalium-tert-butylat und 11,8 g des Produktes aus Beispiel 7a nach Säulenchromatographie mit Petrolether/Ether (2 : 1) 10,56 g der Titelverbindung.

$^1$H—NMR ($CDCl_3$) : 1,40-2,40 (m, 12 H) ; 3,27-4,27 (m, 4 H) ; 5,20-5,63 (m, 2 H) ; 5,97-7,56 (m, 5 H).

c) 3,4-Bis-(tetrahydropyranyl-2'-oxy)-phenylacetylen

Man verfährt analog Beispiel 5c und erhält aus 4,0 g der in Beispiel 7b hergestellten Chlorvinylverbindung und 16,24 ml n-Butyllithiumlösung nach Säulenchromatographie mit Petrolether/Ether (5 : 1) 2,96 g der Titelverbindung, die allmählich zu einer weißen kristallinen Masse mit einem Schmelzbereich von 58°-70 °C erstarrt.

$^1$H—NMR ($CDCl_3$) : 1,37-2,33 (m, 12 H) ; 2,90 (s, 1 H) ; 3,33-4,23 (m, 4 H) ; 5,23-5,56 (m, 2 H) ; 6,83-7,33 (m, 3 H).

d) 1-[3',4'-Bis-(tetrahydropyranyl-2''-oxy)-phenyl]-11-(tetrahydropyranyl-2'-oxy)-undeca-1,9-diin

Man verfährt analog Beispiel 5d und erhält aus 5,0 g des Produktes aus Beispiel 7c, 10,3 ml n-Butyllithiumlösung, 4,63 g 1-Jod-9-(tetrahydropyranyl-2'-oxy)-non-7-in und 15 ml absolutem Hexamethylphosphorsäuretriamid nach Säulenchromatographie mit Petrolether/Ether (7 : 2) 5,14 g der Titelverbindung.

$^1$H—NMR ($CDCl_3$) : 1,20-2,55 (m, 30 H) ; 3,27-4,25 (m, 6 H) ; 4,10-4,33 (m, 2 H) ; 4,70-4,87 (m, 1 H) ; 5,27-5,50 (m, 2 H) ; 6,83-7,27 (m, 3 H).

e) Das im Beispiel 7d verwendete 1-Jod-9-(tetrahydropyranyl-2'-oxy)-non-7-in ist wie folgt zugänglich :

4,20 g 1-Brom-9-(tetrahydropyranyl-2'-oxy)-non-7-in, gelöst in 30 ml absolutem Aceton, versetzt man bei Raumtemperatur mit einer Lösung von 6,23 g Natriumjodid in 30 ml absolutem Aceton, läßt die Mischung unter einer Stickstoffatmosphäre über Nacht stehen, dampft im Vakuum ein und nimmt den Rückstand in Ether/Wasser auf. Die Etherphase wird abgetrennt, mit Wasser und gesättigter Natriumchloridlösung gewaschen, über Natriumsulfat getrocknet und im Vakuum eingedampft, wobei man 4,66 g der Titelverbindung erhält.

$^1$H—NMR ($CDCl_3$) : 1,20-2,43 (m, 16 H) ; 3,03-3,33 (t, 2 H) ; 3,20-4,05 (m, 2 H) ; 4,13-4,33 (m, 2 H) ; 4,67-4,87 (m, 1 H).

f) 1-(3',4'-Dihydroxyphenyl)-11-hydroxy-undeca-1,9-diin

Man verfährt analog Beispiel 5f und erhält aus 5,10 g des Produktes aus Beispiel 7d, 100 ml absolutem Ethanol und 0,24 g Pyridinium-toluol-4-sulfonat nach Säulenchromatographie mit Petrolether/Ether (1 : 3) und Umkristallisation aus n-Hexan/Essigsäureethylester 1,91 g der Titelverbindung in Form weißer Kristalle, die bei 81 °C-83 °C schmelzen.

$^1$H—NMR ($CDCl_3$) : 1,25-2,00 (m, 9 H) ; 2,03-2,60 (m, 4 H) ; 4,20-4,40 (m, 2 H) ; 6,57-7,00 (m, 3 H).

## Beispiel 8

11-Acetoxy-1-(3',4'-diacetoxyphenyl)-undeca-1,9-diin

Durch Acetylierung des in Beispiel 7 erhaltenen Produktes unter Anwendung der in Beispiel 4 beschriebenen Arbeitsweise erhält man die Titelverbindung.

$^1$H—NMR ($CDCl_3$) : 1,20-1,83 (m, 8 H) ; 1,93-2,50 (m, 13 H) ; 4,40-4,60 (m, 2 H) ; 6,73-7,20 (m, 3 H).

## Beispiel 9

1-(3',4'-Dihydroxyphenyl)-11-hydroxy-undeca-1Z,9Z-dien

0,545 g des in Beispiel 7 erhaltenen Produktes, gelöst in 20 ml absolutem Ethanol, der 0,1 ml frisch destilliertes Chinolin enthält, werden bei Raumtemperatur und Atmosphärendruck über 0,136 g Palladium auf Calciumcarbonat (5 % Pd ; mit Blei vergifteter « Lindlar-Katalysator ») hydriert. Nach Aufnahme der theoretischen Menge Wasserstoff wird vom Katalysator abfiltriert und das Filtrat im Vakuum eingedampft. Der Rückstand wird durch HPLC mit Methanol/Wasser (60 : 40) gereinigt, wobei man 0,416 g der Titelverbindung erhält.

$^1$H—NMR ($CDCl_3$) : 1,15-1,75 (m, 8 H) ; 1,70-2,53 (m, 4 H) ; 4,07-4,33 (m, 2 H) ; 5,23-6,37 (m, 4 H) ; 6,47-6,90 (m, 3 H).

## Beispiel 10

Man verfährt wie in Beispiel 9 und erhält so aus dem Produkt des Beispiels 5 das 11-Hydroxy-1-(4'-hydroxy-3'-methoxyphenyl)-undeca-1Z,9Z-dien.

$^1$H—NMR ($CDCl_3$) : 1,10-1,70 (m, 8 H) ; 1,72-2,50 (m, 4 H) ; 3,77 (m, 3 H) ; 3,80-4,13 (m, 2 H) ; 5,13-6,30 (m, 4 H) ; 5,53 (s, 1 H) ; 6,37-6,73 (m, 3 H).

## Beispiel 11

1-(3',4'-Dimethoxyphenyl)-11-hydroxy-undeca-1,9-diin

a) 1-(3',4'-Dimethoxyphenyl)-11-(tetrahydropyranyl-2'-oxy)-undeca-1,9-diin

Man verfährt analog Beispiel 5d und erhält aus 0,75 g 3,4-Dimethoxyphenylacetylen, 2,88 ml n-Butyllithiumlösung, 1,29 g 1-Jod-9-(tetrahydropyranyl-2'-oxy)-non-7-in sowie 4 ml absolutem Hexamethylphosphorsäuretriamid nach Säulenchromatographie mit Petrolether/Ether (2 : 1) 1,17 g der Titelverbindung.

$^1$H—NMR ($CDCl_3$) : 1,25-1,93 (m, 14 H) ; 2,03-2,53 (m, 4 H) ; 3,30-4,00 (m, 2 H) ; 3,83 (s, 6 H) ; 4,13-4,27 (m, 2 H) ; 4,67-4,87 (m, 1 H) ; 6,57-7,00 (m, 3 H).

b) 1-(3',4'-Dimethoxyphenyl)-11-hydroxy-undeca-1,9-diin

Man arbeitet analog Beispiel 5f und erhält aus 1,15 g des Produktes aus Beispiel 11a, 30 ml absolutem Ethanol und 0,075 g Pyridiniumtoluol-4-sulfonat nach Säulenchromatographie mit n-Hexan/Ether (1 : 1) 0,78 g der Titelverbindung in Form weißer Kristalle, die bei 39°-41 °C schmelzen.

$^1$H—NMR ($CDCl_3$) : 1,25-1,85 (m, 9 H) ; 2,05-2,55 (m, 4 H) ; 3,83 (s, 6 H) ; 4,07-4,33 (m, 2 H) ; 6,57-7,00 (m, 3 H).

Beispiel 12

11-Hydroxy-1-(3',4'-methylendioxyphenyl)-undeca-1,9-diin

a) 1-(3',4'-Methylendioxyphenyl)-11-(tetrahydropyranyl)-2'-oxy)-undeca-1,9-diin

Man verfährt analog Beispiel 5d und erhält aus 1,61 g 3,4-Methylendioxyphenylacetylen, 6,9 ml n-Butyllithiumlösung, 3,08 g 1-Jod-9-(tetrahydropyranyl-2'-oxy)-non-7-in sowie 10 ml absolutem Hexamethylphosphorsäuretriamid nach Säulenchromatographie mit Petrolether/Ether (10 : 1) 2,60 g der Titelverbindung.

$^1$H—NMR ($CDCl_3$) : 1,20-1,90 (m, 14 H) ; 2,03-2,53 (m, 4 H) ; 3,23-4,00 (m, 2 H) ; 4,13-4,30 (m, 2 H) ; 4,65-4,85 (m, 1 H) ; 5,85 (s, 2 H) ; 6,50-6,93 (m, 3 H).

b) 11-Hydroxy-1-(3',4'-methylendioxyphenyl)-undeca-1,9-diin

Man arbeitet analog Beispiel 5f und erhält aus 2,51 g des in Beispiel 12a angefallenen Produktes, 70 ml absolutem Ethanol und 0,17 g Pyridinium-toluol-4-sulfonat nach Säulenchromatographie mit n-Hexan/Ether (3 : 2) 1,41 g der Titelverbindung in Form weißer Kristalle, die bei 45°-46 °C schmelzen.

$^1$H—NMR ($CDCl_3$) : 1,25-1,80 (m, 9 H) ; 2,03-2,53 (m, 4 H) ; 4,07-4,33 (m, 2 H) ; 5,85 (s, 2 H) ; 6,50-6,93 (m, 3 H).

Beispiel 13

1-(3',4'-Dihydroxyphenyl)-11-hydroxy-11-methyl-dodeca-1,9-diin

a) 1-Brom-9-methyl-9-(tetrahydropyranyl-2'-oxy)-dec-7-in

5,05 g 3-Methyl-3-(tetrahydropyranyl-2'-oxy)-but-1-in, 18,8 ml n-Butyllithium-Lösung, 13,9 ml 1,6-Dibromhexan und 10 ml absolutes Hexamethylphosphorsäuretriamid werden analog Beispiel 5e zur Reaktion gebracht. (DC : Petrolether/Ether — 20 : 1). Nach 6 Stunden wird das Kältebad entfernt. Sobald das Reaktionsgemisch 0 °C erreicht hat, wird analog Beispiel 5e aufgearbeitet. Säulenchromatographie mit Petrolether/Ether (20 : 1) liefert 6,08 g der Titelverbindung.

$^1$H—NMR ($CDCl_3$) : 1,20-2,37 (m, 22 H) ; 3,23-4,13 (m, 4 H) ; 4,90-5,10 (m, 1 H).

b) 1-[3',4'-Bis-(tetrahydropyranyl-2''-oxy)-phenyl]-11-methyl-11-(tetrahydropyranyl-2'-oxy)-dodeca-1,9-diin

Man verfährt analog Beispiel 5d und erhält aus 1,51 g 3,4-Bis-(tetrahydropyranyl-2'-oxy)-phenylacetylen, 3,13 ml n-Butyllithiumlösung, 1,33 g des in Beispiel 13a angefallenen Produktes sowie 4,5 ml absolutem Hexamethylphosphorsäuretriamid nach Säulenchromatographie mit Petrolether/Ether (5 : 1) 1,81 g der Titelverbindung.

$^1$H—NMR ($CDCl_3$) : 1,25-2,50 (m, 36 H) ; 3,27-4,17 (m, 6 H) ; 4,90-5,10 (m, 1 H) ; 5,23-5,43 (m, 2 H) ; 6,87-7,13 (m, 3 H).

c) 1-(3',4'-Dihydroxyphenyl)-11-hydroxy-11-methyl-dodeca-1,9-diin

Man arbeitet analog Beispiel 5f und erhält aus 1,75 g des Produktes aus Beispiel 13b, 30 ml absolutem Ethanol und 0,08 g Pyridiniumtoluol-4-sulfonat nach Säulenchromatographie mit Petrolether/Ether (1 : 3) und Umkristallisation aus n-Hexan/Essigsäureethylester 0,69 g der Titelverbindung in Form weißer Kristalle, die bei 71 °C bis 73 °C schmelzen.

$^1$H—NMR ($CDCl_3$) : 1,25-1,73 (m, 14 H) ; 1,97-2,50 (m, 5 H) ; 5,40 (s, 1 H) ; 5,90 (s, 1 H) ; 6,60-6,90 (m, 3 H).

Beispiel 14

1-(3',4'-Dihydroxyphenyl)-11-hydroxy-hexadeca-1,9-diin

a) 1-[3',4'-Bis-(tetrahydropyranyl-2''-oxy)-phenyl]-8-brom-oct-1-in

In eine Lösung von 3,33 g 3,4-Bis-(tetrahydropyranyl-2'-oxy)-phenylacetylen in 20 ml absolutem Tetrahydrofuran gibt man tropfenweise bei —78° bis —70 °C unter trocknem Stickstoff innerhalb von 45 Minuten 6,9 ml n-Butyllithiumlösung, rührt noch 1 Stunde im Kältebad und versetzt dann mit 5,1 ml 1,6-Dibromhexan, anschließend tropfenweise mit 7 ml absolutem 1,3-Dimethyl-3,4,5,6-tetrahydro-2(1H)-pyrimidinon. Man rührt 20 Stunden, läßt auf 0 °C erwärmen und arbeitet analog Beispiel 5e auf. Durch Säulenchromatographie mit Toluol/Diisopropylether (10 : 1) erhält man 3,55 g der Titelverbindung.

$^1$H—NMR ($CDCl_3$) : 1,20-2,56 (m, 22 H) ; 3,23-4,23 (m, 6 H) ; 5,25-5,53 (m, 2 H) ; 6,73-7,20 (m, 3 H).

b) 1-[3′,4′-Bis-(tetrahydropyranyl-2″-oxy)-phenyl]-11-(tert-butyldiphenylsilyloxy)-hexadeca-1,9-diin

Man arbeitet nach dem im Beispiel 5d beschriebenen Verfahren und erhält aus 1,82 g 3-(tert-Butyldiphenylsilyloxy)-oct-1-in, 3,13 ml n-Butyllithiumlösung, 1,86 g des Produktes aus Beispiel 14a sowie 4,5 ml absolutem Hexamethylphosphorsäuretriamid nach Säulenchromatographie mit Petrolether/Ether (4 : 1) 2,26 g der Titelverbindung.

$^1$H—NMR ($CDCl_3$) : 0,67-2,50 (m, 35 H) ; 1,07 (s, 9 H) ; 3,33-4,45 (m, 5 H) ; 5,23-5,43 (m, 2 H) ; 6,85-7,80 (m, 13 H).

c) Zur Darstellung des in Beispiel 14b als eine der Ausgangsverbindungen verwendeten 3-(tert-Butyldiphenylsilyloxy)-oct-1-in gibt man bei 0 °C zu einer Lösung von 2,03 g Oct-1-in-3-ol und 1,32 g Imidazol in 20 ml absolutem Dimethylformamid unter Rühren und Oberleiten von trocknem Stickstoff tropfenweise 5 ml tert-Butyldiphenylchlorsilan, rührt 3 Stunden bei Raumtemperatur nach, verdünnt mit 100 ml Dichlormethan und wäscht nacheinander je einmal mit Wasser, gesättigter Natriumhydrogencarbonatlösung sowie gesättigter Natriumchloridlösung. Die organische Phase wird über Natriumsulfat getrocknet und im Vakuum eingedampft. Durch Säulenchromatographie des Rückstandes mit Petrolether/ Toluol (8 : 1) erhält man 5,34 g der Titelverbindung.

$^1$H—NMR ($CDCl_3$) : 0,63-1,87 (m, 11 H) ; 1,10 (s, 9 H) ; 2,28 (d, 1 H) ; 4,13-4,43 (m, 1 H) ; 7,15-7,80 (m, 10 H).

d) 1-[3′,4′-Bis-(tetrahydropyranyl-2″-oxy)-phenyl]-11-hydroxy-hexadeca-1,9-diin

Man versetzt bei 0°-5 °C eine Lösung von 2,23 g des in Beispiel 14b erhaltenen Produktes in 30 ml absolutem Tetrahydrofuran unter Rühren und Oberleiten von trocknem Stickstoff tropfenweise mit 9 ml einer Lösung von Tetra-n-butylammoniumfluorid in Tetrahydrofuran (1 Mol/l) und läßt dann auf Raumtemperatur erwärmen. (DC : Petrolether/Ether — 3 : 2). Nach vier Stunden ist die Umsetzung vollständig. Man versetzt mit gesättigter Ammoniumchloridlösung und arbeitet analog Beispiel 5e auf. Durch Säulenchromatographie des Rohproduktes mit n-Hexan/Ether (3 : 2) erhält man 1,42 g der Titelverbindung.

$^1$H—NMR ($CDCl_3$) : 0,67-2,53 (m, 36 H) ; 3,33-4,43 (m, 5 H) ; 5,20-5,43 (m, 2 H) ; 6,80-7,15 (m, 3 H).

e) 1-(3′-4′-Dihydroxyphenyl)-11-hydroxy-hexadeca-1,9-diin

Man Verfährt analog Beispiel 5f und erhält aus 1,40 g des Produktes aus Beispiel 14d, 30 ml absolutem Ethanol und 0,07 g Pyridiniumtoluol-4-sulfonat nach Säulenchromatographie mit Petrolether/Ether (1 : 3) 0,73 g der Titelverbindung in Form Von Kristallen, die bei 46°-48 °C schmelzen.

$^1$H—NMR ($CDCl_3$) : 0,67-1,93 (m, 19 H) ; 2,00-2,50 (m, 4 H) ; 4,17-4,57 (m, 3 H) ; 6,50-7,00 (m, 3 H).

## Beispiel 15

1-(3′,4′-Dihydroxyphenyl)-11-hydroxy-11-phenyl-undeca-1,9-diin

a) 1-[3′,4′-Bis-(tetrahydropyranyl-2″-oxy)-phenyl]-deca-1,9-diin

0,237 g Lithiumacetylid-Ethylendiaminkomplex (95 %) werden mit 2,1 ml absolutem Dimethylsulfoxid übergossen und unter trocknem Stickstoff 30 Minuten bei Raumtemperatur gerührt. In das auf + 8°C abgekühlte Gemisch gibt man vorsichtig bei einer Temperatur von 8°-9 °C tropfenweise eine Lösung von 1,0 g 1-[3′,4′-Bis-(tetrahydropyranyl-2″-oxy)-phenyl]-8-brom-oct-1-in in 1,5 ml absolutem Dimethylsulfoxid. Man entfernt das Kühlbad und rührt 3 Stunden bei Raumtemperatur nach. Dann wird mit gesättigter Ammoniumchloridlösung zersetzt und analog Beispiel 5e aufgearbeitet. Nach Säulenchromatographie des Rohproduktes mit Petrolether/Ether (4 : 1) erhält man 0,689 g der Titelverbindung.

$^1$H—NMR ($CDCl_3$) : 1,30-2,55 (m, 25 H) ; 3,33-4,20 (m, 4 H) ; 5,23-5,47 (m, 2 H) ; 6,83-7,15 (m, 3 H).

b) 1-[3′,4′-Bis-(tetrahydropyranyl-2″-oxy)-phenyl]-11-oxo-11-phenylundeca-1,9-diin

0,64 g des Produktes aus Beispiel 15a werden in 10 ml absolutem Triethylamin gelöst und unter Rühren und Überleiten von trocknem Stickstoff zunächst mit 0,18 ml Benzoylchlorid, dann mit 0,032 g Bis-(triphenylphosphin)-palladium(II)-chlorid sowie 0,015 g Kupfer-(I)-jodid versetzt. (DC : Petrolether/Ether — 2 : 1). Nach beendeter Umsetzung wird mit 50 ml Ether verdünnt, filtriert und das Filtrat im

Vakuum eingedampft. Der ölige Rückstand liefert durch Säulenchromatographie mit Petrolether/Ether (3 : 1) 0,66 g der Titelverbindung.

$^1$H—NMR ($CDCl_3$) : 1,25-2,10 (m, 20 H) ; 2,15-2,67 (m, 4 H) ; 3,30-4,20 (m, 4 H) ; 5,23-5,47 (m, 2 H) ; 6,80-8,17 (m, 8 H).

c) 1-[3',4'-Bis-(tetrahydropyranyl-2''-oxy)-phenyl]-11-hydroxy-11-phenyl-undeca-1,9-diin

0,64 g der in Beispiel 15b erhaltenen Verbindung werden in 3 ml einer Lösung von Cer(III)-chlorid in Methanol (0,4 Mol/l) gelöst und bei Raumtemperatur unter Rühren und Überleiten von trocknem Stickstoff in drei Portionen mit insgesamt 0,047 g Natriumborhydrid versetzt. Nach 30 Minuten wird durch tropfenweise Zugabe von 1,2 ml Pufferlösung-pH 7 zersetzt. Man gibt 10 ml Dichlormethan und 1 ml gesättigte Kaliumnatriumtartratlösung zu, trennt die organische Phase ab und extrahiert die wässrige Schicht zweimal mit je 10 ml Dichlormethan. Die vereinigten organischen Phasen werden je einmal mit gesättigter Natriumhydrogencarbonat- bzw. gesättigter Natriumchloridlösung gewaschen und über Natriumsulfat getrocknet. Aus dem durch Eindampfen im Vakuum erhaltenen Rohprodukt gewinnt man durch Säulenchromatographie mit Petrolether/Ether (1 : 1) 0,60 g der Titelverbindung.

$^1$H—NMR ($CDCl_3$) : 1,25-2,53 (m, 25 H) ; 3,33-4,20 (m, 4 H) ; 5,23-5,50 (m, 3 H) ; 6,83-7,57 (m, 8 H).

d) 1-(3',4'-Dihydroxyphenyl)-11-hydroxy-11-phenyl-undeca-1,9-diin

Nach dem im Beispiel 5f beschriebenen Verfahren erhält man aus 0,58 g des Produktes aus Beispiel 15c, 12,5 ml absolutem Ethanol und 0,03 g Pyridinium-toluol-4-sulfonat nach Säulenchromatographie mit n-Hexan/Ether (1 : 2) 0,283 g der Titelverbindung in Form von bei 93°-95 °C schmelzenden Kristallen.

$^1$H—NMR ($CDCl_3$) : 1,23-1,83 (m, 9 H) ; 2,07-2,53 (m, 4 H) ; 5,20-5,70 (m, 3 H) ; 6,50-7,57 (m, 8 H).

Beispiel 16

11-Cyclohexyl-1-(3',4'-dihydroxyphenyl)-11-hydroxy-undeca-1,9-diin

a) 1-[3',4'-Bis-(tetrahydropyranyl-2''-oxy)-phenyl]-11-cyclohexyl-11-hydroxy-undeca-1,9-diin

Eine Lösung von 1,09 g des in Beispiel 15a erhaltenen Produktes in 10 ml absolutem Tetrahydrofuran wird bei —70 °C unter Rühren und Überleiten von trocknem Stickstoff tropfenweise mit 1,66 ml n-Butyllithiumlösung versetzt. Man rührt 30 Minuten nach, wobei man die Temperatur auf —10 °C ansteigen läßt und gibt dann tropfenweise 0,48 ml Cyclohexanaldehyd zu. Man hält noch je eine Stunde bei —10 °C, dann bei Raumtemperatur. Anschließend wird mit 5 ml gesättigter Ammoniumchloridlösung zersetzt und analog Beispiel 5e aufgearbeitet. Nach Säulenchromatographie des Rohproduktes mit Petrolether/Ether (2 : 1) erhält man 0,94 g der Titelverbindung.

$^1$H—NMR ($CDCl_3$) : 0,70-2,50 (m, 36 H) ; 3,26-4,20 (m, 5 H) ; 5,23-5,43 (m, 2 H) ; 6,83-7,15 (m, 3 H).

b) 11-Cyclohexyl-1-(3',4'-dihydroxyphenyl)-11-hydroxy-undeca-1,9-diin

Nach der im Beispiel 5f beschriebenen Verfahrensweise erhält man aus 0,91 g des in Beispiel 16a angefallenen Produktes, 18 ml absolutem Ethanol und 0,04 g Pyridinium-toluol-4-sulfonat nach Säulenchromatographie mit n-Hexan/Ether (2 : 5) 0,44 g der Titelverbindung.

$^1$H—NMR ($CDCl_3$) : 0,70-2,50 (m, 24 H) ; 3,93-4,20 (m, 1 H) ; 5,43 (s, 1 H) ; 5,87 (s, 1 H) ; 6,50-6,90 (m, 3 H).

Beispiel 17

11-Hydroxy-1-(3',4',5'-trimethoxyphenyl)-undeca-1,9-diin

a) 5-(2'-Chlorvinyl)-1,2,3-trimethoxy-benzol

In eine Suspension von 11,94 g Chlormethyl-triphenylphosphoniumchlorid in 85 ml absolutem Dimethylsulfoxid und 170 ml absolutem Tetrahydrofuran läßt man bei 0 °C unter Rühren und Überleiten von trocknem Stickstoff 21,5 ml n-Butyllithiumlösung eintropfen, rührt 30 Minuten nach, versetzt die entstandene Lösung tropfenweise mit 5,48 g 3,4,5-Trimethoxybenzaldehyd, gelöst in 20 ml absolutem Tetrahydrofuran, rührt noch 30 Minuten bei 0 °C und läßt dann auf Raumtemperatur erwärmen. Nach etwa 4 Stunden wird analog Beispiel 5e aufgearbeitet. Durch Säulenchromatographie des Rohproduktes mit Petrolether/Ether (2 : 1) erhält man 4,64 g der Titelverbindung.

$^1$H—NMR ($CDCl_3$) : 3,83 (s, 9 H) ; 6,05-6,93 (m, 4 H).

b) 3,4,5-Trimethoxyphenylacetylen

Unter Anwendung der in Beispiel 5c beschriebenen Verfahrensweise erhält man aus 4,60 g des

Produktes aus Beispiel 17a und 28,2 ml n-Butyllithiumlösung 3,47 g Rohprodukt. Umkristallisation aus n-Hexan liefert 3,09 g der Titelverbindung in Form weißer Kristalle, die bei 68°-70 °C schmelzen.

$^1H$—NMR ($CDCl_3$) : 2,97 (s, 1 H) ; 3,77 (s, 9 H), 6,60 (s, 2 H).

c) 11-(Tetrahydropyranyl-2'-oxy)-1-(3',4',5'-trimethoxyphenyl)-undeca-1,9-diin

Man verfährt analog Beispiel 5d und erhält aus 1,73 g des Produktes aus Beispiel 17b, 5,65 ml n-Butyllithiumlösung, 2,10 g 1-Jod-9-(tetrahydropyranyl-2'-oxy)-non-7-in sowie 7,5 ml absolutem Hexamethylphosphorsäuretriamid nach Säulenchromatographie mit Petrolether/Ether (2 : 1) 1,94 g der Titelverbindung.

$^1H$—NMR ($CDCl_3$) : 1,30-1,93 (m, 14 H) ; 2,05-2,53 (m, 4 H) ; 3,27-4,03 (m, 2 H) ; 3,80 (s, 9 H) ; 4,13-4,27 (m, 2 H) ; 4,67-4,83 (m, 1 H) ; 6,53 (s, 2 H).

d) 11-Hydroxy-1-(3',4',5'-trimethoxyphenyl)-undeca-1,9-diin

Man verfährt analog Beispiel 5f und erhält aus 1,91 g des in Beispiel 17c angefallenen Produktes, 40 ml absolutem Ethanol und 0,09 g Pyridinium-toluol-4-sulfonat nach Säulenchromatographie mit n-Hexan/Ether (1 : 1) 1,38 g der Titelverbindung.

$^1H$—NMR ($CDCl_3$) : 1,33-1,83 (m, 9 H) ; 2,05-2,57 (m, 4 H) ; 3,80 (s, 9 H) ; 4,10-4,30 (m, 2 H) ; 6,53 (s, 2 H).

Beispiel 18

1-(3',5'-Dimethoxy-4'-hydroxyphenyl)-11-hydroxy-undeca-1,9-diin

a) 4-(tert-Butyldimethylsilyloxy)-3,5-dimethoxy-benzaldehyd

Unter den in Beispiel 14c beschriebenen Bedingungen setzt man 2,05 g 3,5-Dimethoxy-4-hydroxybenzaldehyd und 0,91 g Imidazol in 15 ml absolutem Dimethylformamid mit 2,05 g tert-Butyldimethylchlorsilan um. Das Rohprodukt wird durch Chromatographie an Kieselgel 60 (0,063-0,200 mm) mit Petrolether/Ether (1 : 1) gereinigt, wobei man 2,91 g der Titelverbindung in Form von Kristallen, die bei 68 °C schmelzen, erhält.

$^1H$—NMR ($CDCl_3$) : 0,20 (s, 6 H) ; 1,03 (s, 9 H) ; 3,83 (s, 6 H) ; 7,00 (s, 2 H) ; 9,67 (s, 1 H).

b) 2-(tert-Butyldimethylsilyloxy)-5-(2'-chlorvinyl)-1,3-dimethoxy-benzol

Man verfährt analog Beispiel 17a und erhält aus 2,90 g des in Beispiel 18a angefallenen Produktes, 4,08 g Chlormethyltriphenylphosphoniumchlorid sowie 7,4 ml n-Butyllithiumlösung nach Säulenchromatographie mit Petrolether/Ether (3 : 1) 2,74 g der Titelverbindung.

$^1H$—NMR ($CDCl_3$) : 0,13 (s, 6 H) ; 1,03 (s, 9 H) ; 3,75 (s, 6 H) ; 5,97-6,83 (m, 4 H).

c) 4-(tert-Butyldimethylsilyloxy)-3,5-dimethoxy-phenylacetylen

Man verfährt analog Beispiel 5c und erhält aus 2,72 g des Produktes aus Beispiel 18b und 11,65 ml n-Butyllithiumlösung nach chromatographischer Reinigung an Kieselgel 60 (0,063-0,200 mm) mit Petrolether/Ether (4 : 1) 1,89 g der Titelverbindung in Form von Kristallen, die bei 35°-38 °C schmelzen.

$^1H$—NMR ($CDCl_3$) : 0,15 (s, 6 H) ; 1,03 (s, 9 H) ; 2,95 (s, 1 H) ; 3,75 (s, 6 H) ; 6,63 (s, 2 H).

d) 1-[4'-(tert-Butyldimethylsilyloxy)-3',5'-dimethoxyphenyl]-11-(tetrahdropyranyl-2'-oxy)-undeca-1,9-diin

Man verfährt analog Beispiel 5d und erhält aus 1,85 g des in Beispiel 18c angefallenen Produktes, 3,95 ml n-Butyllithiumlösung, 1,48 g 1-Jod-9-(tetrahydropyranyl-2'-oxy)-non-7-in sowie 5,3 ml absolutem Hexamethylphosphorsäuretriamid nach Säulenchromatographie mit Petrolether/Ether (3 : 1) 1,22 g der Titelverbindung.

$^1H$—NMR ($CDCl_3$) : 0,13 (s, 6 H) ; 1,03 (s, 9 H) ; 1,23-1,97 (m, 14 H) ; 2,00-2,50 (m, 4 H) ; 3,25-3,95 (m, 2 H) ; 3,75 (s, 6 H) ; 4,13-4,30 (m, 2 H) ; 4,67-4,87 (m, 1 H) ; 6,53 (s, 2 H).

e) 1-(3',5'-Dimethoxy-4'-hydroxyphenyl)-11-hydroxy-undeca-1,9-diin

1,19 g des in Beispiel 18d erhaltenen Produktes werden unter trocknem Stickstoff mit 25 ml einer methanolischen Lösung von Chlorwasserstoff (3 %) übergossen und bei Raumtemperatur gerührt, wobei die eingesetzte Verbindung allmählich in Lösung geht. (DC : n-Hexan/Aceton — 3 : 2). Nach Ende der Umsetzung wird das Reaktionsgemisch vorsichtig in 50 ml gesättigte Natriumhydrogencarbonatlösung gegossen. Man extrahiert mehrfach mit je 30 ml Ether, wäscht die Extrakte je einmal mit gesättigter

Natriumhydrogencarbonat- bzw. Natriumchloridlösung und trocknet über Natriumsulfat. Das durch Eindampfen im Vakuum erhaltene Rohprodukt wird zunächst durch Säulenchromatographie mit n-Hexan/Aceton (3 : 2) vorgereinigt. Durch HPLC mit Methanol/Wasser (70 : 30) erhält man dann 0,428 g der Titelverbindung.

$^1$H—NMR ($CDCl_3$) : 1,23-1,83 (m, 9 H) ; 2,00-2,53 (m, 4 H) ; 3,80 (s, 6 H) ; 4,05-4.30 (m, 2 H) ; 5,45 (s, 1 H) ; 6,53 (s, 2 H).

Beispiel 19

11-Hydroxy-1-(3'-hydroxy-4'-methoxyphenyl)-undeca-1,9-diin

a) 4-Methoxy-3-(tetrahydropyranyl-2'-oxy)-benzaldehyd

20,0 g Isovanillin, suspendiert in 320 ml Dichlormethan, werden in Gegenwart von 0,20 g p-Toluolsulfonsäuremonohydrat analog Beispiel 5a mit 14,28 ml 3,4-Dihydro-2H-pyran umgesetzt und anschließend aufgearbeitet. Durch Säulenchromatographie des Rohproduktes mit Petrolether/Ether (1 : 1) erhält man 24,85 g der Titelverbindung.

$^1$H—NMR ($CDCl_3$) : 1,40-2,27 (m, 6 H) ; 3,26-4,10 (m, 2 H) ; 3,87 (s, 3 H) ; 5,20-5,47 (m, 1 H) ; 6,60-7,56 (m, 3 H) ; 9,54 (s, 1 H).

b) 4-(2'-Chlorvinyl)-2-(tetrahydropyranyl-2'-oxy)-anisol

Man verfährt analog Beispiel 5b und erhält aus 20,0 g Chlormethyltriphenylphosphoniumchlorid, 6,46 g Kalium-tert-butylat und 9,0 g des in Beispiel 19a erhaltenen Produktes nach Säulenchromatographie mit Petrolether/Ether (1 : 1) 8,43 g der Titelverbindung.

$^1$H—NMR ($CDCl_3$) : 1,37-2,10 (m, 6 H) ; 3,30-4,13 (m, 2 H) ; 3,82-3,83 (s,s 3 H) ; 5,11-5,37 (m, 1 H) ; 5,80-7,43 (m, 5 H).

c) 4-Methoxy-3-(tetrahydropyranyl-2'-oxy)-phenylacetylen

Man verfährt analog Beispiel 5c und erhält aus 2,92 g der in Beispiel 19b hergestellten Chlorvinylverbindung und 14,73 ml n-Butyllithiumlösung nach Säulenchromatographie mit Petrolether/Essigsäureethylester (2 : 1) 2,02 g der Titelverbindung.

$^1$H—NMR ($CDCl_3$) : 1,37-2,05 (m, 6 H) ; 2,87 (s, 1 H) ; 3,25-4,05 (m, 2 H) ; 3,80 (s, 3 H) ; 5,07-5,33 (m, 1 H) ; 6,40-7,20 (m, 3 H).

d) 1-[4'-Methoxy-3'-(tetrahydropyranyl-2''-oxy)-phenyl]-11-(tetrahydropyranyl-2'-oxy)-undeca-1,9-diin

Nach dem im Beispiel 5d beschriebenen Verfahren erhält man aus 1,90 g des Produktes aus Beispiel 19c, 5,11 ml n-Butyllithiumlösung, 2,47 g 1-Brom-9-(tetrahydropyranyl-2'-oxy)-non-7-in sowie 5,70 ml 1,3-Dimethyl-3,4,5,6-tetrahydro-2-(1H)-pyrimidinon nach Säulenchromatographie mit Petrolether/Ether (2 : 1) 3,14 g der Titelverbindung.

$^1$H—NMR ($CDCl_3$) : 1,20-2,53 (m, 24 H) ; 3,16-4,10 (m, 4 H) ; 3,83 (s, 3 H) ; 4,13-4,27 (m, 2 H) ; 4 56-4,80 (m, 1 H) ; 5,16-5,37 (m, 1 H) ; 6,43-7,07 (m, 3 H).

e) 11-Hydroxy-1-(3'-hydroxy-4'-methoxyphenyl)-undeca-1,9-diin

Man verfährt analog Beispiel 5f und erhält aus 3,0 g des Produktes aus Beispiel 19d, 60 ml absolutem Ethanol und 0,150 g Pyridiniumtoluol-4-sulfonat nach Säulenchromatographie mit Essigsäureethylester/Petrolether (4 : 3) 1,61 g der Titelverbindung in Form farbloser Kristalle, die bei 77 °C bis 78 °C schmelzen.

$^1$H—NMR ($CDCl_3$) : 1,20-1,87 (m, 8 H) ; 1,98-2,53 (m, 4 H) ; 3,80 (s, 3 H) ; 3,93-4,23 (m, 2 H) ; 6,50-7,02 (m, 3 H).

Beispiel 20

Man verfährt analog Beispiel 1, verwendet jedoch statt des 5-Jodsalicylsäuremethylesters
a) 5-Jodsalicylsäure-n-propylester bzw.
b) 5-Jodsalicylsäurepiperidid und erhält so

a) 1-(4'-Hydroxy-3'-n-propoxycarbonyl-phenyl)-11-hydroxy-undeca-1,9-diin

$^1$H—NMR ($CDCl_3$) : 0,77-2,53 (m, 17 H) ; 3,77-4,37 (m, 4 H) ; 6,40-7,53 (m, 3 H) ; 10,47 (s, 1 H)
bzw.

b) 1-(4'-Hydroxy-3'-(N,N-pentamethylencarbamoyl)-phenyl]-11-hydroxy-undeca-1,9-diin.

$^1$H—NMR ($CDCl_3$) : 1,25-1,95 (m, 15 H) ; 1,95-2,50 (m, 4 H) ; 3,35-3,70 (m, 4 H) ; 4,05-4,23 (m, 2 H) ; 6,57-7,23 (m, 3 H).

Beispiel 21

Man verfährt wie in Beispiel 3, verwendet aber statt des Ammoniaks 2-Aminoethanol und erhält so aus dem in Beispiel 1 dargestellten Ester das 1-[3'-(2"-Hydroxyethyl-carbamoyl)-4'-hydroxyphenyl]-11-hydroxy-undeca-1,9-diin.
$^1$H—NMR ($CDCl_3$) : 1,16-2,53 (m, 12 H) ; 3,27-4,23 (m, 6 H) ; 6,52-6,83 (m, 1 H) ; 6,84-7,33 (m, 3 H) ; 10,77 (s, 1 H).

Beispiel 22

Unter Verwendung der entsprechenden Ausgangsmaterialien erhält man nach den beschriebenen, insbesondere den in den Beispielen 1-21 näher erläuterten Verfahrensweisen :

a) 11-Cyclohexyl-1-(4'-hydroxy-3'-methoxyphenyl)-11-hydroxy-undeca-1,9-diin ;

$^1$H—NMR ($CDCl_3$) : 0,71-2,73 (m, 23 H) ; 3,90-4,27 (m, 1 H) ; 3,91 (s, 3 H) ; 5,63 (s, 1 H) ; 6,60-7,07 (m, 3 H).

b) 11-Cyclohexyl-1-(3',5'-dimethoxy-4'-hydroxyphenyl)-11-hydroxy-undeca-1,9-diin ;

$^1$H—NMR ($CDCl_3$) : 0,90-1,97 (m, 20 H) ; 1,95-2,47 (m, 4 H) ; 3,80 (s, 6 H) ; 3,80-4,17 (m, 1 H) ; 5,43 (s, 1 H) ; 6,47 (s, 2 H).

c) 11-Cyclohexyl-1-(3',4'-dihydroxyphenyl)-11-hydroxy-undec-9E-en-1-in

$^1$H—NMR ($CDCl_3$) : 0,85-2,53 (m, 24 H) ; 3,57-3,93 (m, 1 H) ; 5,10-5,80 (m, 3 H) ; 6,13 (s, 1 H) ; 6,47-6,83 (m, 3 H)
und daraus durch Hydrierung analog Beispiel 9

d) 11-Cyclohexyl-1-(3',4'-dihydroxyphenyl)-11-hydroxy-undeca-1Z,9E-dien ;

$^1$H—NMR ($CDCl_3$) : 0,85-2,45 (m, 23 H) ; 3,50-3,83 (m, 1 H) ; 5,05-6,80 (m, 7 H).

e) 1-(4'-Hydroxy-5'-methoxy-3'-methoxycarbonyl-phenyl)-11-hydroxy-undeca-1,9-diin ;

$^1$H—NMR ($CDCl_3$) : 1,23-2,53 (m, 12 H) ; 3,80 (s, 3 H), 3,87 (s, 3 H) ; 3,93-4,23 (m, 2 H) ; 6,60-6,83 (d, 1 H) ; 7,10-7,33 (d, 1 H) ; 10,60 (s, 1 H).

Beispiel 23

1-(3',4'-Dihydroxyphenyl)-11-hydroxy-undec-1Z-en-9-in

a) 1-(tert-Butyldiphenylsilyloxy)-prop-2-in

2,9 ml 2-Propin-1-ol, 15,6 ml tert-Butyldiphenylchlorsilan und 4,1 g Imidazol werden analog Beispiel 14c umgesetzt. Durch Chromatographie des Rohproduktes über Kieselgel 60 (0,063-0,200 mm) mit Petrolether/Diisopropylether (20 : 1) erhält man 13,3 g der Titelverbindung in Form von bei 55-58 °C schmelzenden Kristallen.
$^1$H—NMR ($CDCl_3$) : 1,07 (s, 9 H) ; 2,30-2,43 (t, 1 H) ; 4,20-4,37 (d, 2 H) ; 7,15-7,75 (m, 10 H).

b) 1-Brom-10-(tert-butyldiphenylsilyloxy)-dec-8-in

Man verfährt analog Beispiel 5e und erhält unter Verwendung von 7,36 g des Produktes aus Beispiel 23a, 15,63 ml n-Butyllithiumlösung, 12,8 ml 1,7-Dibromheptan und 22 ml absolutem Hexamethylphosphorsäuretriamid nach Säulenchromatographie mit n-Hexan/Toluol (3 : 1) 6,96 g der Titelverbindung.

$^1$H—NMR ($CDCl_3$) : 1,07 (s, 9 H) ; 1,10-2,27 (m, 12 H) ; 3,20-3,50 (t, 2 H) ; 4,17-4,35 (m, 2 H) ; 7,17-7,75 (m, 10 H).

c) [10-(tert-Butyldiphenylsilyloxy)-dec-8-in-1-yl]-triphenylphosphoniumbromid

Eine Lösung von 5,66 g des Produktes aus Beispiel 23b und 3,45 g Triphenylphosphin in 36 ml absolutem Acetonitril wird 6 Tage zum Rückfluß erhitzt. Dann dampft man im Vakuum ein, schüttelt den Rückstand fünfmal mit Diethylether aus und trocknet über Phosphorpentoxid. Es fallen 7,44 g der Titelverbindung in Form eines hygroskopischen Feststoffes an.

$^1$H—NMR ($CDCl_3$) : 1,07 (s, 9 H) ; 1,05-1,83 (m, 10 H) ; 1,85-2,15 (m, 2 H) ; 3,40-3,95 (m, 2 H) ; 4,10-4,30 (m, 2 H) ; 7,00-7,87 (m, 25 H).

d) 1-[3',4'-Bis-(tert-butyldimethylsilyloxy)-phenyl]-11-(tert-butyldiphenylsilyloxy)-undec-1Z-en-9-in

Zu einer Lösung von 5,50 g des Produktes aus Beispiel 23c in 40 ml absolutem Dimethylsulfoxid und 80 ml absolutem Tetrahydrofuran gibt man bei —10 °C unter Rühren und Überleiten von trockenem Stickstoff tropfenweise 4,7 ml n-Butyllithiumlösung. Man rührt 15 Minuten nach und tropft dann eine Lösung von 1,84 g 3,4-Bis-(tert-butyldimethylsilyloxy)-benzaldehyd in 5 ml absolutem Tetrahydrofuran hinzu. Nach einstündigem Rühren bei —10 °C läßt man auf Raumtemperatur erwärmen und zersetzt nach weiteren 4 Stunden mit 20 ml gesättigter Ammoniumchloridlösung. Das bei der Aufarbeitung (analog Beispiel 5e) erhaltene Rohprodukt wird durch Säulenchromatographie mit Petrolether/Toluol (8 : 1) gereinigt, wobei man 1,94 g der Titelverbindung erhält.

$^1$H—NMR ($CDCl_3$) : 0,20 (s, 12 H) ; 0,70-1,55 (m, 35 H) ; 1,90-2,45 (m, 4 H) ; 4,13-4,30 (m, 2 H) ; 4,90-6,25 (m, 2 H) ; 6,50-6,80 (m, 3 H) ; 7,05-7,65 (m, 10 H).

e) Zur Darstellung des im Beispiel 23d verwendeten 3,4-Bis-(tert-butyldimethylsilyloxy)-benzaldehyds setzt man analog Beispiel 14c 2,14 g 3,4-Dihydroxybenzaldehyd, 5,83 g tert-Butyldimethylchlorsilan und 2,58 g Imidazol um und erhält nach Chromatographie mit Petrolether/Ether (6 : 1) 3,57 g der Titelverbindung in Form von Kristallen, die bei 44°-46 °C schmelzen.

$^1$H—NMR ($CDCl_3$) : 0,20 (s, 12 H) ; 0,90 (s, 18 H) ; 6,60-7,20 (m, 3 H) ; 9,47 (s, 1 H).

f) 1-(3',4'-Dihydroxyphenyl)-11-hydroxy-undec-1Z-en-9-in

Man verfährt analog Beispiel 14d und erhält so aus 3,52 g des Produktes aus Beispiel 23d und 42,8 ml einer Lösung von Tetra-n-butylammoniumfluorid in Tetrahydrofuran (1 Mol/l) nach Säulenchromatographie mit Petrolether/Essigsäureethylester (1 : 1) und HPLC-Reinigung mit Methanol/Wasser (6 : 4) 1,29 g der Titelverbindung.

$^1$H—NMR ($CDCl_3$) : 1,10-1,67 (m, 8 H) ; 1,90-2,45 (m, 5 H) ; 4,07-4,30 (m, 2 H) ; 5,15-6,83 (m, 7 H).

Beispiel 24

1-(4'-Hydroxy-3'-methoxyphenyl)-11-hydroxy-hexadeca-1,9-diin

2,70 g des Produktes aus Beispiel 5c setzt man analog Beispiel 13a mit 1,6-Dibromhexan um, läßt das erhaltene 8-Brom-1-[3'-methoxy-4'-(tetrahydropyranyl-2"-oxy)-phenyl]-oct-1-in analog Beispiel 15a mit Lithiumacetylid-Ethylendiaminkomplex reagieren, setzt anschließend analog Beispiel 16a mit n-Hexanal um und spaltet schließlich aus dem so gewonnenen 11-Hydroxy-1-[3'-methoxy-4'-(tetrahydropyranyl-2"-oxy)-phenyl]-hexadeca-1,9-diin analog Beispiel 5f die Schutzgruppe ab. Durch Säulenchromatographie des Rohproduktes mit Ether/Petrolether (1 : 1) erhält man die reine Titelverbindung.

$^1$H—NMR ($CDCl_3$) : 0,56-2,55 (m, 23 H) ; 3,77 (s, 3 H) ; 3,97-4,40 (m 1 H) ; 5,43 (s, 1 H) ; 6,51-6,77 (m, 3 H).

Beispiel 25

11-Cyclohexyl-1-(3',5'-dimethoxy-4'-hydroxyphenyl)-11-hydroxy-undec-9E-en-1-in

a) 4-(tert-Butyldiphenylsilyloxy)-3,5-dimethoxy-phenylacetylen

Unter Verwendung von 3,5-Dimethoxy-4-hydroxybenzaldehyd und tert-Butyldiphenylchlorsilan sowie den übrigen in Beispielen 18a-c genannten Ausgangsmaterialien und den dort beschriebenen Vorgehensweisen erhält man die Titelverbindung in Form von bei 83°-85 °C schmelzenden Kristallen.

$^1$H—NMR ($CDCl_3$) : 1,07 (s, 9 H) ; 2,83 (s, 1 H) ; 3,33 (s, 6 H) ; 6,40 (s, 2 H) ; 6,97-7,60 (m, 10 H).

b) 1-[4'-(tert-Butyldiphenylsilyloxy)-3',5'-dimethoxyphenyl]-9-(tetrahydropyranyl-2'-oxy)-non-1-in

Man verfährt analog Beispiel 5d und erhält aus 14,58 g des Produktes aus Beispiel 25a, 21,9 ml n-Butyllithiumlösung, 6,98 g 1-Brom-7-(tetrahydropyranyl-2'-oxy)-heptan sowie 55 ml absolutem Hexamethylphosphorsäuretriamid nach Säulenchromatographie mit Petrolether/Ether (5 : 1) 10,27 g der Titelverbindung.

$^1$H—NMR ($CDCl_3$) : 0,75-1,90 (m, 16 H) ; 1,07 (s, 9 H) ; 2,10-2,47 (m, 2 H) ; 3,07-3,97 (m, 4 H) ; 3,35 (s, 6 H) ; 4,37-4,57 (m, 1 H) ; 6,30 (s, 2 H) ; 6,97-7,65 (m, 10 H).

c) 1-[4'-(tert-Butyldiphenylsilyloxy)-3',5'-dimethoxyphenyl]-9-hydroxy-non-1-in

Aus 10,22 g des in Beispiel 25b angefallenen Produktes, 130 ml absolutem Ethanol und 0,38 g Pyridinium-toluol-4-sulfonat erhält man analog Beispiel 5f nach Säulenchromatographie mit Petrolether/Essigsäureethylester (1 : 1) 7,98 g der Titelverbindung.

$^1$H—NMR ($CDCl_3$) : 1,07 (s, 9 H) ; 1,15-1,80 (m, 11 H) ; 2,10-2,50 (m, 2 H) ; 3,33 (s, 6 H) ; 3,35-3,70 (m, 2 H) ; 6,30 (s, 2 H) ; 6,97-7,65 (m, 10 H).

d) 1-[4'-(tert-Butyldiphenylsilyloxy)-3',5'-dimethoxyphenyl]-non-1-in-9-al

Eine Lösung von 1,20 g des in Beispiel 25c erhaltenen Produktes in 22,5 ml absolutem Dichlormethan wird bei Raumtemperatur unter Rühren und Überleiten von trocknem Stickstoff mit 0,37 g wasserfreiem Natriumacetat, danach portionsweise mit 0,75 g Pyridinium-chlorochromat versetzt. Die Reaktion ist nach ca. 2 Stunden beendet. (DC : Petrolether/Ether — 1 : 1). Man filtriert, engt das Filtrat auf ein Volumen von etwa 2 ml ein und chromatographiert mit Petrolether/Ether (3 : 2), wobei 0,87 g der Titelverbindung erhalten werden.

$^1$H—NMR ($CDCl_3$) : 1,07 (s, 9 H) ; 1,15-1,83 (m, 8 H) ; 2,10-2,57 (m, 4 H) ; 3,33 (s, 6 H) ; 6,30 (s, 2 H) ; 6,97-7,67 (m, 10 H) ; 9,57-9,70 (t, 1 H).

e) 1-[4'-(tert-Butyldiphenylsilyloxy)-3',5'-dimethoxyphenyl]-11-cyclohexyl-11-oxo-undec-9E-en-1-in

Eine Lösung von 0,45 g (2-Cyclohexyl-2-oxoethyl)-phosphonsäuredimethylester in 15 ml absolutem Tetrahydrofuran wird tropfenweise unter Rühren und Überleiten von trocknem Stickstoff so mit 1,15 ml n-Butyllithiumlösung versetzt, daß die Innentemperatur unter 5 °C bleibt. Man rührt 15 Minuten nach und gibt dann tropfenweise bei derselben Temperatur eine Lösung von 0,85 g des Produktes aus Beispiel 25d in 7,5 ml absolutem 1,2-Dimethoxyethan zu. Man rührt das Reaktionsgemisch noch 2 Stunden, wobei man auf Raumtemperatur erwärmen läßt, zersetzt anschließend mit gesättigter Ammoniumchloridlösung und arbeitet analog Beispiel 5e auf. Durch Säulenchromatographie des Rohproduktes mit Petrolether/Ether (5 : 1) erhält man 0,78 g der Titelverbindung.

$^1$H—NMR ($CDCl_3$) : 0,90-2,50 (m, 23 H) ; 1,07 (s, 9 H) ; 3,33 (s, 6 H) ; 5,83-6,95 (m, 2 H) ; 6,30 (s, 2 H) ; 7,00-7,67 (m, 10 H).

f) 1-[4'-(tert-Butyldiphenylsilyloxy)-3',5'-dimethoxyphenyl]-11-cyclohexl-11-hydroxy-undec-9E-en-1-in

Man verfährt analog Beispiel 15c und erhält aus 0,74 g des Produktes aus Beispiel 25e in 3 ml einer Lösung von Cer(III)-chlorid in Methanol (0,4 Mol/l) mit 0,049 g Natriumborhydrid nach Säulenchromatographie mit Petrolether/Ether (2 : 1) 0,70 g der Titelverbindung.

$^1$H—NMR ($CDCl_3$) : 0,80-2,45 (m, 24 H) ; 1,07 (s, 9 H) ; 3,33 (s, 6 H) ; 3,50-3,83 (m, 1 H) ; 5,05-5,60 (m, 2 H) ; 6,30 (s, 2 H) ; 6,97-7,67 (m, 10 H).

g) 11-Cyclohexyl-1-(3',5'-dimethoxy-4'-hydroxyphenyl)-11-hydroxy-undec-9E-en-1-in

Man verfährt analog Beispiel 14d, arbeitet aber bei Raumtemperatur und erhält so aus 0,64 g des Produktes aus Beispiel 25f mit 3 ml einer Lösung von Tetra-n-butylammoniumfluorid in Tetrahydrofuran nach Säulenchromatographie mit Petrolether/Essigsäureethylester (3 : 2) 0,36 g der Titelverbindung.

$^1$H—NMR ($CDCl_3$) : 0,75-2,53 (m, 24 H) ; 3,55-3,90 (m, 1 H) ; 3,80 (s, 6 H) ; 5,07-5,70 (m, 3 H) ; 6,47 (s, 2 H).

Beispiel 26

1-(3',4'-Dimethoxyphenyl)-11-hydroxy-undec-1E-en-9-in und das entsprechende 1Z-Isomer

a) 10-(tert-Butyldiphenylsilyloxy)-dec-8-in-1-al

Unter Verwendung von 8,87 g des Produktes aus Beispiel 23a, 18,8 ml n-Butyllithiumlösung, 6,73 g 1-Brom-7-(tetrahydropyranyl-2'-oxy)-heptan und 28,5 ml absolutem Hexamethylphosphorsäuretriamid erhält man analog Beispiel 5d 8,92 g 1-(tert-Butyldiphenylsilyloxy)-10-(tetrahydropyranyl-2'-oxy)-dec-2-in aus dem analog Beispiel 5f die Schutzgruppe abgespalten wird. Das so gewonnene 1-(tert-Butyldiphenylsilyloxy)-10-hydroxy-dec-2-in wird dann analog Beispiel 25d zur Titelverbindung oxydiert, die durch Chromatographie mit Petrolether/Ether (2 : 1) gereinigt wird.

$^1H$—NMR ($CDCl_3$) : 1,07 (s, 9 H) ; 1,10-1,77 (m, 8 H) ; 1,93-2,53 (m, 4 H) ; 4,10-4,27 (t, 2 H) ; 7,05-7,63 (m, 10 H) ; 9,40-9,55 (t, 1 H).

b) 11-(tert-Butyldiphenylsilyloxy)-1-(3',4'-dimethoxyphenyl)-undec-1E,Z-en-9-in

Zu einer Suspension von 1,571 g 3,4-Dimethoxybenzyltriphenylphosphoniumchlorid in 8 ml absolutem Tetrahydrofuran gibt man bei 2 bis 5 °C unter Rühren und Überleiten von trocknem Stickstoff portionsweise 0,392 g Kalium-tert.-butylat. Man rührt 30 Minuten und gibt dann tropfenweise innerhalb von 5 Minuten eine Lösung von 0,841 g des Produktes aus Beispiel 26a in 3 ml absolutem Tetrahydrofuran zu und verdünnt nach weiteren 60 Minuten mit etwa 15 ml Essigsäureethylester. Das Gemisch wird je einmal mit gesättigten Lösungen von Ammoniumchlorid bzw. Natriumchlorid gewaschen, dann über Natriumsulfat getrocknet und eingedampft. Nach Säulenchromatographie mit Ether/Petrolether (1 : 10) erhält man 0,698 g der Titelverbindung.

$^1H$—NMR ($CDCl_3$) : 0,12-2,43 (m, 21 H) ; 3,77 (s, 6 H) ; 4,03-4,23 (m, 2 H) ; 5,11-6,73 (m, 5 H) ; 6,91-7,67 (m, 10 H).

c) 1-(3',4'-Dimethoxyphenyl)-11-hydroxy-undec-1E,Z-en-9-in und die entsprechenden 1E- bzw. 1Z-Isomeren

Man arbeitet analog Beispiel 14d und erhält so aus 0,452 g des Produktes aus Beispiel 26b mit 1,26 ml einer Lösung von Tetra-n-butylammoniumfluorid in Tetrahydrofuran nach Säulenchromatographie mit Ether/Petrolether (2 : 1) 0,223 g der Titelverbindung.

$^1H$—NMR ($CDCl_3$) : 1,13-2,51 (m, 12 H) ; 3,80 (s, 6 H) ; 3,94-4,23 (m, 2 H) ; 5,10-6,37 (m, 2 H) ; 6,43-6,73 (m, 3 H).

Das bei der Synthese der Titelverbindung anfallende Isomerengemisch läßt sich durch HPLC mit Methanol/Wasser (7 : 3) unter Gewinnung des 1E- bzw. des 1Z-Isomeren auftrennen :

1E-Isomer :

$^1H$—NMR ($CDCl_3$) : 1,10-2,50 (m, 12 H) ; 3,73 (s, 3 H) ; 3,77 (s, 3 H) ; 3,87-4,27 (m, 2 H) ; 5,53-6,33 (m, 2 H, $^3J$ : 15 Hz) ; 6,40-6,75 (m, 3 H).

1Z-Isomer :

$^1H$—NMR ($CDCl_3$) : 1,10-2,50 (m, 12 H) ; 3,73 (s, 6 H) ; 3,90-4,20 (m, 2 H) ; 5,10-6,30 (m, 2 H ; $^3J$ : 11 Hz) ; 6,37-6,70 (m, 3 H).

Beispiel 27

1-(3',5'-Dimethoxy-4'-hydroxyphenyl)-11-hydroxy-undec-9Z-en-1-in

a) 9-Brom-1-[4'-(tert-butyldiphenylsilyloxy)-3',5'-dimethoxyphenyl]-non-1-in

Zu einer Lösung von 6,11 g des Produktes aus Beispiel 25c und 5,33 g Triphenylphosphin in 230 ml absolutem Dichlormethan gibt man bei 0 °C unter Rühren und Überleiten von trocknem Stickstoff portionsweise 6,74 g Tetrabrommethan. Nach zweistündigem Rühren wird das Gemisch im Vakuum eingedampft und der Rückstand mit 240 ml Petrolether und 12 ml Ether versetzt. Man filtriert, dampft das Filtrat ein und reinigt den Rückstand durch Säulenchromatographie mit Petrolether/Ether (20 : 1), wobei man 6,47 g der Titelverbindung erhält.

$^1H$—NMR ($CDCl_3$) : 1,07 (s, 9 H) ; 1,20-2,03 (m, 10 H) ; 2,15-2,50 (m, 2 H) ; 3,17-3,47 (m, 2 H) ; 3,33 (s, 6 H) ; 6,30 (s, 2 H) ; 7,00-7,65 (m, 10 H).

b) 2-(tert-Butyldiphenylsilyloxy)-acetaldehyd

Man setzt unter den in Beispiel 14c beschriebenen Bedingungen 5,15 g Glykolaldehyd und 26,78 ml tert.-Butyldiphenylchlorsilan in Gegenwart von 7,61 g Imidazol und 26 ml absolutem Dimethylformamid um und erhält nach Säulenchromatographie mit Ether/Petrolether (1 : 2) 21,26 g der Titelverbindung.

$^1H$—NMR ($CDCl_3$) : 1,10 (s, 9 H) ; 4,07 (d, 2 H) ; 6,73-7,56 (m, 10 H) ; 9,30 (t, 1 H).

c) 11-(tert.-Butyldiphenylsilyloxy)-1-[4'-(tert.-butyldiphenylsilyloxy)3',5'-dimethoxyphenyl]-undec-9Z-en-1-in

6,38 g des Produktes aus Beispiel 27a und 3,13 g Triphenylphosphin setzt man analog Beispiel 23c um und bringt dann 3,42 g des erhaltenen Produktes mit 2,5 ml n-Butyllithiumlösung und 0,90 g des in

Beispiel 27b dargestellten Aldehyds analog Beispiel 23d zur Reaktion. Nach Säulenchromatographie mit Petrolether/Ether (20 : 1) erhält man 1,74 g der Titelverbindung.

$^1H$—NMR ($CDCl_3$) : 0,75-2,45 (m, 30 H) ; 3,33 (s, 6 H) ; 4,05-4,23 (m, 2 H) ; 5,13-5,63 (m, 2 H) ; 6,30 (s, 2 H) ; 6,97-7,67 (m, 20 H).

d) 1-(3',5'-Dimethoxy-4'-hydroxyphenyl)-11-hydroxy-undec-9Z-en-1-in

Durch Abspalten der Schutzgruppe aus 1,71 g des in Beispiel 27c erhaltenen Produktes mit 12,9 ml einer Lösung von Tetra-n-butylammoniumfluorid in Tetrahydrofuran (1 Mol/l) analog Beispiel 14d erhält man nach Säulenchromatographie mit Petrolether/Aceton (1 : 1) 0,625 g der bei 74°-76 °C schmelzenden Titelverbindung.

$^1H$—NMR ($CDCl_3$) : 0,95-2,50 (m, 13 H) ; 3,80 (s, 6 H) ; 3,93-4,23 (m, 2 H) ; 5,15-5,70 (m, 3 H) ; 6,50 (s, 2 H).

**Patentansprüche** (für die Vertragsstaaten : BE, CH, DE, FR, GB, IT, LI, LU, NL, SE)

1. Phenolderivate der allgemeinen Formel

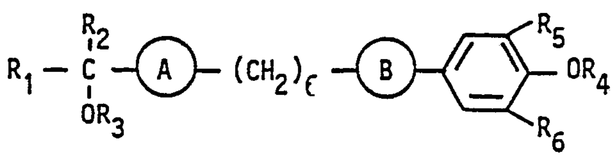

(I)

in der

A und B gleich oder verschieden sind und jeweils für eine der Gruppen —C≡C—, cis-CH=CH— oder trans-CH=CH— stehen,

$R_1$ Wasserstoff oder einen geradkettigen Alkylrest mit 1 bis 6 Kohlenstoffatomen, oder eine 5- bis 7-gliedrige Cycloalkylgruppe oder eine Gruppe der Formel $—(CH_2)_m—O—R_7$, worin m für eine der Zahlen 1, 2 oder 3 und $R_7$ für Methyl oder Ethyl steht, oder eine Gruppe der Formel

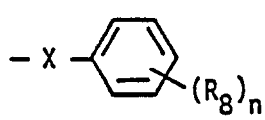

worin X für eine einfache Bindung, eine $—CH_2$-Gruppe oder für eine $—CH_2O$-Gruppe steht und $R_8$ ein Wasserstoff-, Chlor- oder Fluoratom oder eine Methyl-, Methoxy- oder Trifluormethylgruppe bedeutet und n für eine der Zahlen 1 oder 2 steht, bedeutet,

$R_2$ für Wasserstoff, Methyl oder Ethyl steht,

$R_3$ Wasserstoff oder einen Acetyl- oder Propionylrest bedeutet,

$R_4$ Wasserstoff, einen Acetyl- oder Propionylrest oder einen geradkettigen oder verzweigten Alkylrest $R_9$ mit 1 bis 4 Kohlenstoffatomen darstellt,

$R_5$ für eine Gruppe der Formel $—COOR_{10}$, worin $R_{10}$ ein Wasserstoffatom, ein pharmazeutisch verträgliches, insbesondere ein einwertiges Kation, einen geradkettigen oder verzweigten Alkylrest mit 1 bis 6 Kohlenstoffatomen oder die Gruppe $—(CH_2)_2—N—[(CH_2)_p—CH_3]_2$ ist, in der p für Null oder eine der Zahlen 1 bis 3 steht, sowie von pharmazeutisch verträglichen Salzen dieser basischen Ester mit Säuren, oder für eine Gruppe der Formel

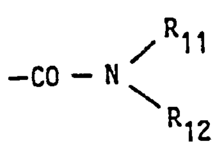

worin $R_{11}$ und $R_{12}$ gleich oder verschieden sind und für Wasserstoff, den Alkylrest $R_9$ oder für 2-Hydroxyethyl stehen oder einer dieser Reste eine Hydroxylgruppe, der andere Wasserstoff bedeutet, oder $R_{11}$ und $R_{12}$ zusammengenommen die Gruppe $—(CH_2)_q—$ darstellen, in der q für eine der Zahlen 4, 5 oder 6 steht, oder für eine Gruppe der Formel

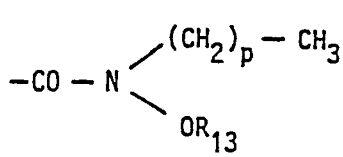

worin $R_{13}$ für Wasserstoff, $—(CH_2)_p—CH_3$, Carboxymethyl, Acetyl oder Propionyl steht und p jeweils die gleiche Bedeutung wie oben hat, oder für eine Gruppe der Formel $—CO—NH—(CH_2)_r—N(CH_3)_2$, worin r für eine der Zahlen 2 oder 3 steht, und deren pharmazeutisch verträgliche Salze mit Säuren, oder für eine Gruppe der Formel

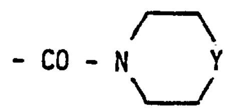

in der Y ein Sauerstoffatom oder die Gruppe $>N—CH_3$ bedeutet und gegebenenfalls deren pharmazeutisch verträgliche Salze mit Säuren, oder für einen Nitrilrest oder für eine Hydroxy-, Acetyloxy- oder Propionyloxygruppe, eine Alkoxygruppe $OR_9$, in der $R_9$ die gleiche Bedeutung wie oben hat, oder — zusammengenommen mit $OR_4$ — für die Methylendioxygruppe steht und

$R_6$ Wasserstoff, eine Hydroxy-, Acetyloxy- oder Propionyloxygruppe, den Alkylrest $R_9$ oder eine Alkoxygruppe $OR_9$, worin $R_9$ jeweils die gleiche Bedeutung wie oben hat, darstellt.

2. Phenolderivate der Formel

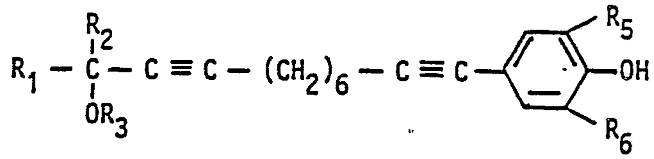

(I')

worin $R_1$ bis $R_3$ sowie $R_5$ und $R_6$ die gleiche Bedeutung wie in Anspruch 1 haben.

3. Phenolderivate der Formel

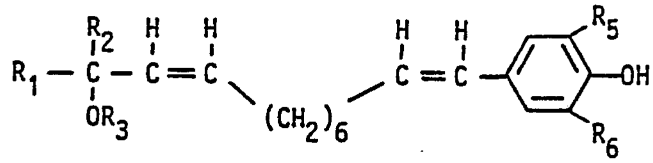

(I'')

worin $R_1$ bis $R_3$ sowie $R_5$ und $R_6$ die gleiche Bedeutung wie in Anspruch 1 haben.

4. Phenolderivate der Formel

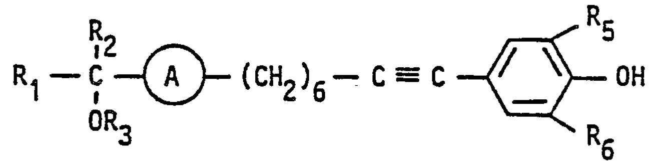

(I''')

worin $R_1$ bis $R_3$ sowie $R_5$, $R_6$ und A die gleiche Bedeutung wie in Anspruch 1 haben.

5. Phenolderivate der allgemeinen Formel

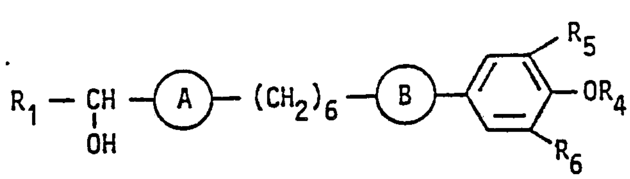

worin $R_1$, $R_4$ bis $R_6$, A und B die gleiche Bedeutung wie in Anspruch 1 haben.

6. Phenolderivate der Formel

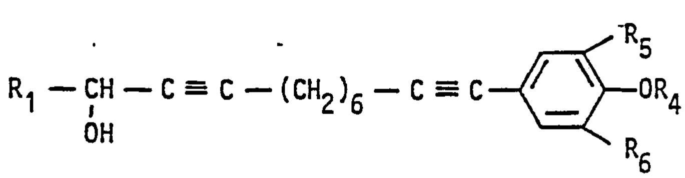

in der $R_1$ ein Wasserstoffatom, einen geradkettigen Alkylrest mit 1 bis 6 Kohlenstoffatomen oder einen Phenyl- oder Cyclohexylrest bedeutet und $R_4$ bis $R_6$ die gleiche Bedeutung wie in Anspruch 1 haben.

7. Phenolderivate gemäß Ansprüchen 1 bis 6, in denen $R_6$ für ein Wasserstoffatom oder eine Methoxygruppe steht.

8. Phenolderivate gemäß Ansprüchen 1 bis 7, in denen $R_5$ für eine der Gruppen —OH, —$OR_9$,

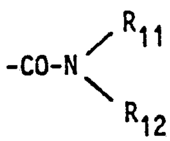
,
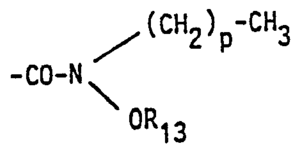
oder
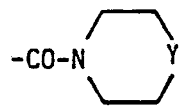

steht, worin $R_9$, $R_{11}$ bis $R_{13}$, p und Y die gleiche Bedeutung wie in Anspruch 1 haben.

9. Phenolderivate gemäß Ansprüchen 1 bis 8, in denen $R_5$ für eine der Gruppen —OH, —$OCH_3$ oder

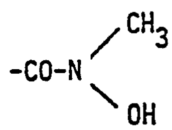

steht.

10. Arzneimittel, dadurch gekennzeichnet, daß es als Wirkstoff wenigstens eines der Phenolderivate entsprechend Ansprüchen 1 bis 9 enthält und zur parenteralen, oralen, intranasalen, perkutanen oder rektalen Applikation geeignet ist.

11. Arzneimittel gemäß Anspruch 10, dadurch gekennzeichnet, daß es als Wirkstoff pro Einzeldosis 0,01 bis 50 mg eines Phenolderivates entsprechend Ansprüchen 1 bis 9 enthält.

12. Arzneimittel gemäß Ansprüchen 10 und 11, dadurch gekennzeichnet, daß es zur parenteralen Applikation geeignet ist und 0,01 bis 10 mg eines Phenolderivates entsprechend Ansprüchen 1 bis 9 enthält.

13. Arzneimittel gemäß Ansprüchen 10 und 11, dadurch gekennzeichnet, daß es zur oralen Applikation geeignet ist und 0,1 bis 50 mg eines Phenolderivates entsprechend Ansprüchen 1 bis 9 enthält.

14. Arzneimittel gemäß Anspruch 13 zur oralen Applikation, dadurch gekennzeichnet, daß es in Form von Tabletten, Dragees oder Kapseln, gegebenenfalls mit verzögerter Wirkstofffreigabe, vorliegt.

15. Arzneimittel gemäß Anspruch 10 zur intranasalen, oralen oder peroralen Verabreichung eines Phenolderivates entsprechend Ansprüchen 1 bis 9 in Spray-Form.

16. Arzneimittel gemäß Anspruch 10 zur perkutanen Applikation, bestehend aus einem auf die Haut aufzubringenden, ein Phenolderivat entsprechend Ansprüchen 1 bis 9 in gelöster Form, vorzugsweise unter Zusatz von die Hautpenetration fördernden Mitteln, enthaltendem Reservoir.

17. Verfahren zur Herstellung der Arzneimittel gemäß Ansprüchen 10 bis 16, dadurch gekennzeichnet, daß man die Phenolderivate entsprechend Ansprüchen 1 bis 9 in üblicher Weise mit Trägermaterialien, Lösungsmitteln bzw. Verdünnungsstoffen, sowie gegebenenfalls Bindemitteln, Tablettensprengstoffen und anderen üblichen Hilfsstoffen verarbeitet und aus dem erhaltenen Gemisch die Einzeldosierungsformen fertigt.

18. Verfahren zur Herstellung der Phenolderivate der Formel I aus Anspruch 1, dadurch gekennzeichnet, daß man

A) eine Verbindung der allgemeinen Formel II

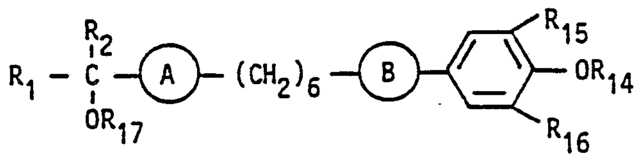
(II)

in der A, B, $R_1$ und $R_2$ die gleiche Bedeutung wie in Formel I haben, $R_{14}$ den Alkylrest $R_9$ oder eine unter milden Bedingungen abspaltbare Schutzgruppe darstellt, $R_{15}$ für die Gruppe $OR_{14}$ steht oder die gleiche Bedeutung wie $R_5$ mit der Maßgabe hat, daß darin $R_{10}$ nicht für ein Kation stehen kann und die in $R_5$ gegebenenfalls vorhandenen basischen Gruppen nicht in Salzform vorliegen können oder $R_{15}$ zusammengenommen mit der Gruppe $OR_{14}$ für eine Methylendioxygruppe steht, $R_{16}$ ein Wasserstoffatom oder eine der Gruppen $R_9$ oder $OR_{14}$ bedeutet und $R_{17}$ eine unter milden Bedingungen abspaltbare Schutzgruppe ist,

1) durch Reaktion einer Verbindung der Formel

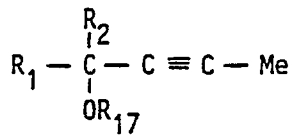

(III)

worin $R_1$, $R_2$ und $R_{17}$ die gleiche Bedeutung wie oben haben und Me für ein Lithium-, Natrium- oder Kaliumatom oder einen der Reste —MgBr oder —MgI steht, mit einer Verbindung der Formel

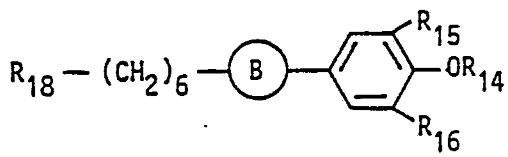

(IV)

in der B und $R_{14}$ bis $R_{16}$ die gleiche Bedeutung wie oben haben und $R_{18}$ für ein Brom- oder ein Jodatom steht, bei Temperaturen von etwa —80° bis +75 °C in einem inerten, aprotischen Lösungsmittel, gegebenenfalls in Gegenwart katalytischer Mengen eines Kupfersalzes, oder

2) durch Umsetzung einer Verbindung der Formel IV, in der $R_{15}$ für die Gruppe $OR_{14}$ steht, mit einer Verbindung der Formel

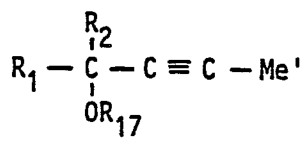

(IIIa)

worin $R_1$, $R_2$ und $R_{17}$ die gleiche Bedeutung wie oben haben und Me' für ein Lithium-, Natrium- oder Kaliumatom steht, in flüssigem Ammoniak bei Temperaturen von etwa —80 °C bis —35 °C, oder

3) durch Reaktion einer Verbindung der Formel

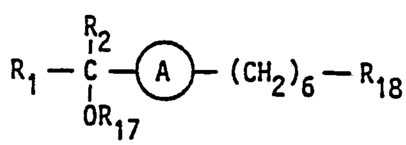

(V)

in der A, $R_1$, $R_2$, $R_{17}$ und $R_{18}$ die gleiche Bedeutung wie oben haben, mit einer Verbindung der Formel

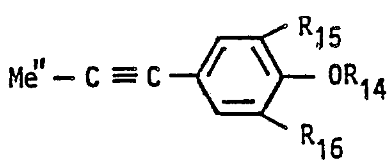

(VI)

worin $R_{14}$ bis $R_{16}$ die gleiche Bedeutung wie oben haben und Me" für eine der oben definierten Gruppen Me oder Me' steht, unter den vorstehend bei 1 bzw. 2 genannten Bedingungen, oder

4) (wenn in der Verbindung der Formel II die Gruppe A für —CH=CH— steht)

i) durch Behandlung einer Verbindung der Formel

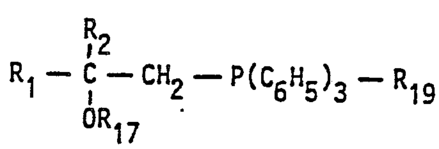

(VII)

in der $R_1$, $R_2$ und $R_{17}$ die gleiche Bedeutung wie oben haben und $R_{19}$ für ein Chlor-, Brom- oder Jodatom steht, in inerten Lösungsmitteln mit einer starken, wasserfreien Base, und Umsetzung des durch Abspaltung von $HR_{19}$ gebildeten Phosphorans mit einem Aldehyd der Formel

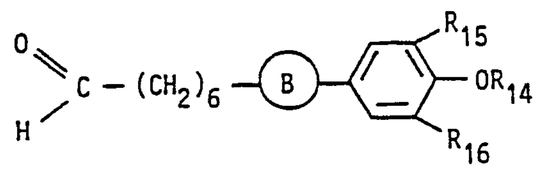

(VIII)

worin B und $R_{14}$ bis $R_{16}$ die gleiche Bedeutung wie oben haben, bei Temperaturen von etwa — 80 °C bis + 30 °C, oder

ii) durch Reaktion des durch Baseneinwirkung auf die Verbindung der Formel

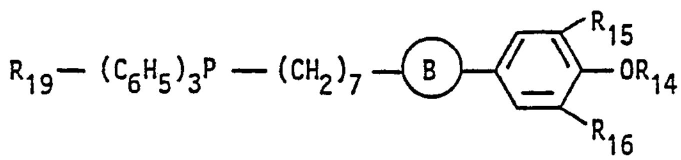

(IX)

worin B, $R_{14}$ bis $R_{16}$ und $R_{19}$ die gleiche Bedeutung wie oben haben, unter $HR_{19}$-Abspaltung gebildeten Phosphorans mit einem Aldehyd der Formel

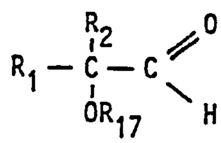

(X)

worin $R_1$, $R_2$ und $R_{17}$ die gleiche Bedeutung wie oben haben, bei Temperaturen von etwa — 80 °C bis + 30 °C in Gegenwart eines inerten Lösungsmittels, oder

5) (wenn in der Verbindung der Formel II die Gruppe B für —CH=CH— steht)

i) durch Reaktion eines aus der Verbindung der Formel

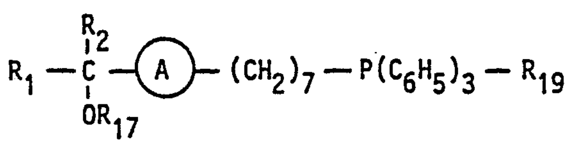

(XI)

worin A, $R_1$, $R_2$, $R_{17}$ und $R_{19}$ die gleiche Bedeutung wie oben haben durch Baseneinwirkung und $HR_{19}$-Abspaltung gebildeten Phosphorans mit einem Aldehyd der Formel

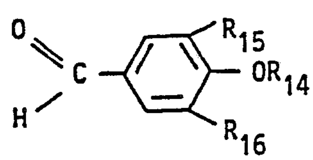

(XII)

worin $R_{14}$ bis $R_{16}$ die gleiche Bedeutung wie oben haben bei Temperaturen von etwa — 80 °C bis + 30 °C in Gegenwart eines inerten Lösungsmittels, oder

ii) durch Umsetzung des durch Baseneinwirkung auf die Verbindung der Formel

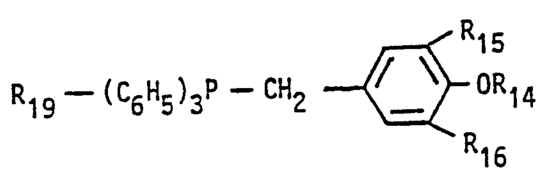

(XIII)

worin $R_{14}$ bis $R_{16}$ und $R_{19}$ die gleiche Bedeutung wie oben haben erhaltenen Phosphorans mit einem Aldehyd der Formel

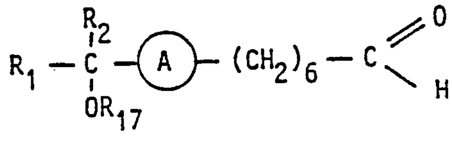

(XIV)

in der A, $R_1$, $R_2$ und $R_{17}$ die gleiche Bedeutung wie oben haben bei Temperaturen von etwa —80 °C bis +30 °C in Gegenwart eines inerten Lösungsmittels herstellt und aus der so erhaltenen Verbindung der Formel II in an sich bekannter Weise die in $R_{14}$ bis $R_{17}$ enthaltenen Schutzgruppen abspaltet und gegebenenfalls — wenn wenigstens einer der Reste $R_3$ und $R_4$ bzw. $R_5$ und $R_6$ einen Acetyl- oder Propionylrest darstellen bzw. enthalten soll — eine oder mehrere Acetyl- oder Propionylgruppe(n) einführt, oder

B) eine Verbindung der Formel

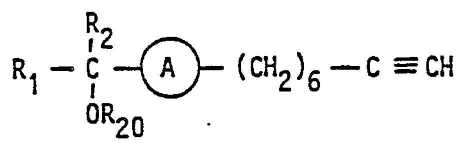

(XV)

worin A, $R_1$ und $R_2$ die gleiche Bedeutung wie oben haben und $R_{20}$ für ein Wasserstoffatom steht oder die gleiche Bedeutung wie $R_{17}$ hat in Gegenwart eines bei etwa —10 °C bis +80 °C flüssigen sekundären oder tertiären Amins und katalytischer Mengen eines komplexen Palladiumkatalysators sowie gegebenenfalls katalytischer Mengen von Kupfer-(I)-jodid bei etwa 0 °C bis 75 °C mit einer Verbindung der Formel

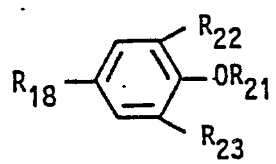

(XVI)

worin $R_{18}$ die gleiche Bedeutung wie oben hat, $R_{21}$ ein Wasserstoffatom, den Alkylrest $R_9$ oder eine unter milden Bedingungen abspaltbare Schutzgruppe bedeutet, $R_{22}$ für die Gruppe $OR_{21}$ steht oder die gleiche Bedeutung wie $R_5$ mit der Maßgabe hat, daß darin $R_{10}$ nicht für ein Kation stehen kann und die in $R_5$ gegebenenfalls vorhandenen basischen Gruppen nicht in Salzform vorliegen können, oder $R_{22}$ zusammengenommen mit der Gruppe $OR_{21}$ für eine Methylendioxygruppe steht und $R_{23}$ die gleiche Bedeutung wie $R_{16}$ hat oder für eine Hydroxylgruppe steht, zur Reaktion bringt und aus der erhaltenen Verbindung der Formel

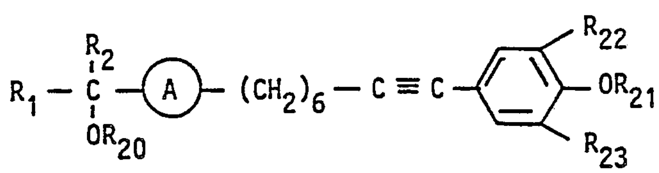

(XVII)

die gegebenenfalls in $R_{20}$ bis $R_{23}$ enthaltenen Schutzgruppen abspaltet und gegebenenfalls — wenn wenigstens einer der Reste $R_3$ und $R_4$ bzw. $R_5$ und $R_6$ einen Acetyl- oder Propionylrest darstellen bzw. enthalten soll — eine oder mehrere Acetyl- oder Propionylgruppe(n) einführt, oder

C) eine Verbindung der Formel

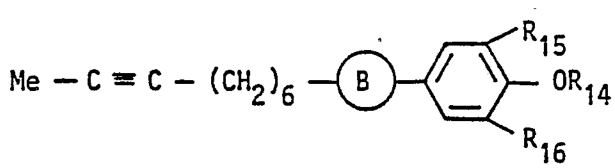

(XVIII)

worin Me, B und $R_{14}$ bis $R_{16}$ die gleiche Bedeutung wie oben haben in Gegenwart eines indifferenten Lösungsmittels bei etwa —80 °C bis +30 °C mit einer Verbindung der Formel

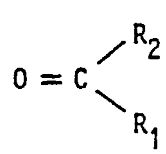

(XIX)

in der $R_1$ und $R_2$ die gleiche Bedeutung wie oben haben zu einer Verbindung der Formel

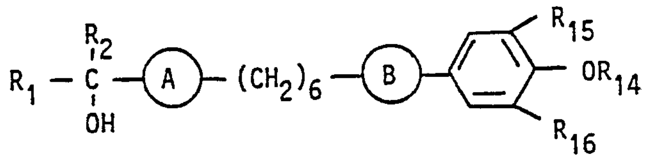

(XX)

worin A, B, $R_1$, $R_2$ und $R_{14}$ bis $R_{16}$ die gleiche Bedeutung wie oben haben umsetzt und aus dem Umsetzungsprodukt die gegebenenfalls in $R_{14}$ bis $R_{16}$ enthaltenen Schutzgruppen abspaltet sowie — wenn wenigstens einer der Reste $R_3$ und $R_4$ bzw. $R_5$ und $R_6$ einen Acetyl- oder Propionylrest darstellen bzw. enthalten soll — eine oder mehrere Acetyl- oder Propionylgruppen einführt, oder

D) eine Verbindung der Formel

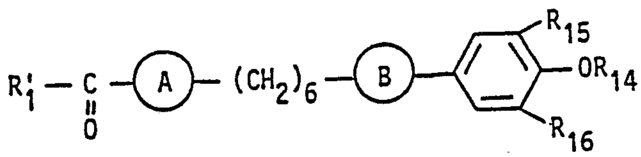

(XXI)

in der A, B und $R_{14}$ bis $R_{16}$ die gleiche Bedeutung wie oben haben und $R'_1$ mit Ausnahme von Wasserstoff die gleiche Bedeutung wie $R_1$ hat

1) durch Behandlung einer Verbindung der Formel

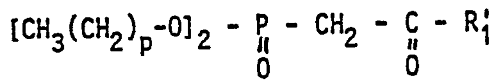

(XXII)

worin p und $R'_1$ die gleiche Bedeutung wie oben haben, in einem inerten Lösungsmittel mit einer wasserfreien Base und anschließende Umsetzung mit einer Verbindung der Formel VIII bei Temperaturen von etwa — 10 °C bis + 35 °C, oder

2) durch Umsetzung einer Verbindung der Formel

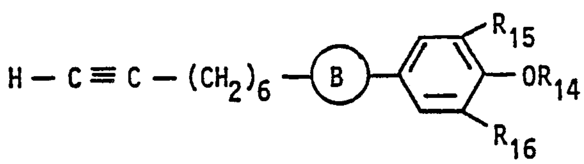

(XXIII)

in der B und $R_{14}$ bis $R_{16}$ die gleiche Bedeutung wie oben haben, in Gegenwart eines oberhalb etwa + 80 °C siedenden tertiären Amins und katalytischer Mengen eines komplexen Palladiumkatalysators sowie gegebenenfalls katalytischer Mengen von Kupfer(I)-jodid bei etwa 0 °C bis 75 °C mit einer Verbindung der Formel

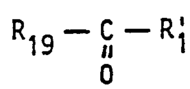

(XXIV)

worin $R'_1$ und $R_{19}$ die gleiche Bedeutung wie oben haben, herstellt und diese Verbindung der Formel XXI durch Reduktion mit einem Metallborhydrid oder durch Reaktion mit einer Verbindung der Formel $R'_2$-Me, worin Me die gleiche Bedeutung wie oben hat und $R'_2$ mit Ausnahme von Wasserstoff die gleiche Bedeutung wie $R_2$ hat, anschließende Abspaltung von in $R_{14}$ bis $R_{16}$ vorliegenden Schutzgruppen und gegebenenfalls — wenn wenigstens einer der Reste $R_3$ und $R_4$ bzw. $R_5$ und $R_6$ einen Acetyl- oder Propionylrest darstellen bzw. enthalten soll — Einführung einer oder mehrerer Acetyl- oder Propionylgruppen in die entsprechende Verbindung der Formel I (in der $R_1$ und gegebenenfalls $R_2$ von Wasserstoff verschieden sind) überführt, und in den nach A, B, C oder D erhaltenen Verbindungen die Reste $R_5$ bzw. $R_{15}$ oder $R_{22}$ in die gewünschte Gruppe $R_5$ überführt und/oder gegebenenfalls darin vorliegende Dreifachbindungen katalytisch zu Doppelbindungen hydriert.

19. Verfahren gemäß Anspruch 18, dadurch gekennzeichnet, daß die durch $R_{14}$, $R_{17}$, $R_{20}$ oder $R_{21}$ dargestellten bzw. die in $R_{15}$ und $R_{22}$ enthaltenen Schutzgruppen gleiche oder verschiedene Vertreter

aus der den Tetrahydropyranyl-2-rest, den tert-Butyldimethylsilyl- und den tert-Butyldiphenylsilylrest umfassenden Gruppe sind.

20. Verfahren gemäß Ansprüchen 18 und 19, dadurch gekennzeichnet, daß man bei den Verfahrensweisen A1 und A3 in Gegenwart eines dipolaren aprotischen Lösungsmittels und eines Kupfer-(I)-salzes arbeitet.

21. Verfahren gemäß Ansprüchen 18 und 19, dadurch gekennzeichnet, daß bei den Verfahrensweisen A4 und A5 für die Phosphoranbildung durch $HR_{19}$-Abspaltung als wasserfreie Base n-Butyllithium, Kalium-tert-butylat oder Natrium-bis-(trimethylsilyl)-amid eingesetzt wird.

22. Verfahren gemäß Ansprüchen 18 und 19, dadurch gekennzeichnet, daß bei den Verfahrensweisen B und $D_2$ als komplexer Palladiumkatalysator Bis-(triphenylphosphin)-palladium-(II)-chlorid oder -acetat oder Tetrakis-(triphenylphosphin)-palladium eingesetzt werden und in Gegenwart eines Trialkylamins mit 2 oder 3 Kohlenstoffatomen in jedem der Alkylreste gearbeitet wird.

**Patentansprüche** (für den Vertragsstaat AT)

1. Verfahren zur Herstellung von Phenolderivaten der allgemeinen Formel

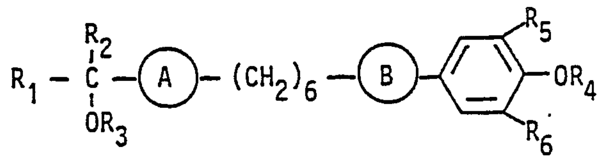

(I)

in der

A und B gleich oder verschieden sind und jeweils für eine der Gruppen —C≡C—, cis-CH=CH— oder trans-CH=CH— stehen,

$R_1$ Wasserstoff oder einen geradkettigen Alkylrest mit 1 bis 6 Kohlenstoffatomen, oder eine 5- bis 7-gliedrige Cycloalkylgruppe oder eine Gruppe der Formel $—(CH_2)_m—O—R_7$, worin m für eine der Zahlen 1, 2 oder 3 und $R_7$ für Methyl oder Ethyl steht, oder eine Gruppe der Formel

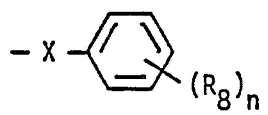

worin X für eine einfache Bindung, eine $—CH_2$-Gruppe oder für eine $—CH_2O$-Gruppe steht und $R_8$ ein Wasserstoff-, Chlor- oder Fluoratom oder eine Methyl-, Methoxy- oder Trifluormethylgruppe bedeutet und n für eine der Zahlen 1 oder 2 steht, bedeutet,

$R_2$ für Wasserstoff, Methyl oder Ethyl steht,

$R_3$ Wasserstoff oder einen Acetyl- oder Propionylrest bedeutet,

$R_4$ Wasserstoff, einen Acetyl- oder Propionylrest oder einen gerad-kettigen oder verzweigten Alkylrest $R_9$ mit 1 bis 4 Kohlenstoffatomen darstellt,

$R_5$ für eine Gruppe der Formel $—COOR_{10}$, worin $R_{10}$ ein Wasserstoffatom, ein pharmazeutisch verträgliches, insbesondere ein einwertiges Kation, einen geradkettigen oder verzweigten Alkylrest mit 1 bis 6 Kohlenstoffatomen oder die Gruppe $—(CH_2)_2—N—[(CH_2)_p—CH_3]_2$ ist, in der p für Null oder eine der Zahlen 1 bis 3 steht, sowie von pharmazeutisch verträglichen Salzen dieser basischen Ester mit Säuren, oder für eine Gruppe der Formel

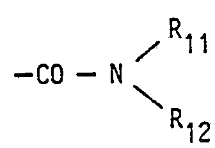

worin $R_{11}$ und $R_{12}$ gleich oder verschieden sind und für Wasserstoff, den Alkylrest $R_9$ oder für 2-Hydroxyethyl stehen oder einer dieser Reste eine Hydroxylgruppe, der andere Wasserstoff bedeutet, oder $R_{11}$ und $R_{12}$ zusammengenommen die Gruppe $—(CH_2)_q—$ darstellen, in der q für eine der Zahlen 4, 5 oder 6 steht, oder für eine Gruppe der Formel

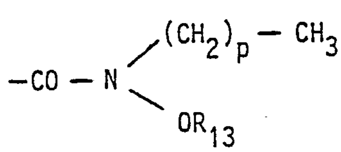

worin $R_{13}$ für Wasserstoff, $—(CH_2)_p—CH_3$, Carboxymethyl, Acetyl oder Propionyl steht und p jeweils die gleiche Bedeutung wie oben hat, oder für eine Gruppe der Formel $—CO—NH—(CH_2)_r—N(CH_3)_2$, worin r für eine der Zahlen 2 oder 3 steht, und deren pharmazeutisch verträgliche Salze mit Säuren, oder für eine Gruppe der Formel

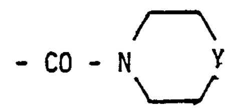

in der Y ein Sauerstoffatom oder die Gruppe $>N—CH_3$ bedeutet und gegebenenfalls deren pharmazeutisch verträgliche Salze mit Säuren, oder für einen Nitrilrest oder für eine Hydroxy-, Acetyloxy- oder Propionyloxygruppe, eine Alkoxygruppe $OR_9$, in der $R_9$ die gleiche Bedeutung wie oben hat, oder — zusammengenommen mit $OR_4$ — für die Methylendioxygruppe steht und

$R_6$ Wasserstoff, eine Hydroxy-, Acetyloxy- oder Propionyloxygruppe, den Alkylrest $R_9$ oder eine Alkoxygruppe $OR_9$, worin $R_9$ jeweils die gleiche Bedeutung wie oben hat, darstellt, dadurch gekennzeichnet, daß man

A) eine Verbindung der allgemeinen Formel II

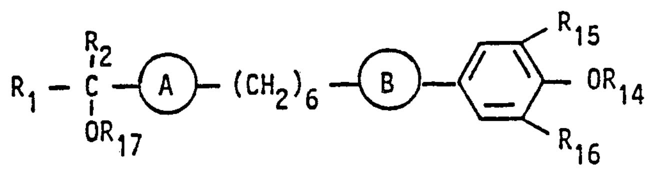

(II)

in der A, B, $R_1$ und $R_2$ die gleiche Bedeutung wie in Formel I haben, $R_{14}$ den Alkylrest $R_9$ oder eine unter milden Bedingungen abspaltbare Schutzgruppe darstellt, $R_{15}$ für die Gruppe $OR_{14}$ steht oder die gleiche Bedeutung wie $R_5$ mit der Maßgabe hat, daß darin $R_{10}$ nicht für ein Kation stehen kann und die in $R_5$ gegebenenfalls vorhandenen basischen Gruppen nicht in Salzform vorliegen können oder $R_{15}$ zusammengenommen mit der Gruppe $OR_{14}$ für eine Methylendioxygruppe steht, $R_{16}$ ein Wasserstoffatom oder eine der Gruppen $R_9$ oder $OR_{14}$ bedeutet und $R_{17}$ eine unter milden Bedingungen abspaltbare Schutzgruppe ist,

1) durch Reaktion einer Verbindung der Formel

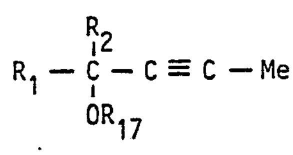

(III)

worin $R_1$, $R_2$ und $R_{17}$ die gleiche Bedeutung wie oben haben und Me für ein Lithium-, Natrium- oder Kaliumatom oder einen der Reste —MgBr oder —MgI steht, mit einer Verbindung der Formel

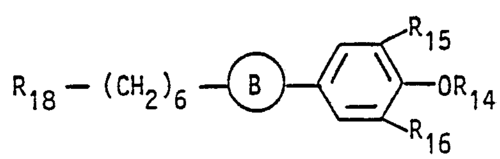

(IV)

in der B und $R_{14}$ bis $R_{16}$ die gleiche Bedeutung wie oben haben und $R_{18}$ für ein Brom- oder ein Jodatom steht, Bei Temperaturen von etwa —80° bis +75 °C in einem inerten, aprotischen Lösungsmittel, gegebenenfalls in Gegenwart katalytischer Mengen eines Kupfersalzes, oder

2) durch Umsetzung einer Verbindung der Formel IV, in der $R_{15}$ für die Gruppe $OR_{14}$ steht, mit einer Verbindung der Formel

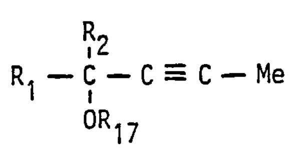

(IIIa)

worin $R_1$, $R_2$ und $R_{17}$ die gleiche Bedeutung wie oben haben und Me' für ein Lithium-, Natrium- oder Kaliumatom steht, in flüssigem Ammoniak bei Temperaturen von etwa — 80 °C bis — 35 ° C, oder

3) durch Reaktion einer Verbindung der Formel

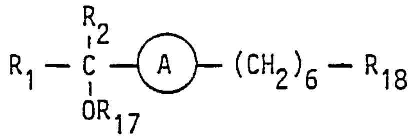

(V)

in der A, $R_1$, $R_2$ und $R_{17}$ und $R_{18}$ die gleiche Bedeutung wie oben haben, mit einer Verbindung der Formel

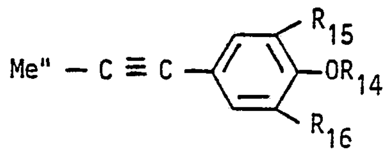

(VI)

worin $R_{14}$ bis $R_{16}$ die gleiche Bedeutung wie oben haben und Me″ für eine der oben definierten Gruppen Me oder Me′ steht, unter den vorstehend bei 1 bzw. 2 genannten Bedingungen, oder

4) (wenn in der Verbindung der Formel II die Gruppe A für —CH=CH— steht)

i) durch Behandlung einer Verbindung der Formel

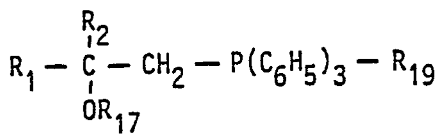

(VII)

in der $R_1$, $R_2$ und $R_{17}$ die gleiche Bedeutung wie oben haben und $R_{19}$ für ein Chlor-, Brom- oder Jodatom steht, in inerten Lösungsmitteln mit einer starken, wasserfreien Base, und Umsetzung des durch Abspaltung von $HR_{19}$ gebildeten Phosphorans mit einem Aldehyd der Formel

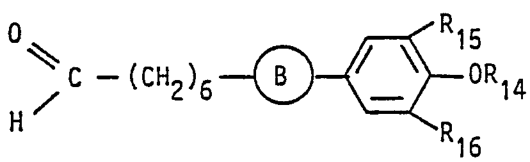

(VIII)

worin B und $R_{14}$ bis $R_{16}$ die gleiche Bedeutung wie oben haben, bei Temperaturen von etwa —80 °C bis + 30 °C, oder

ii) durch Reaktion des durch Baseneinwirkung auf die Verbindung der Formel

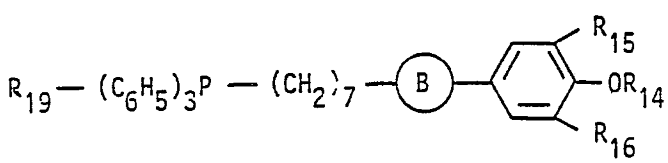

(IX)

worin B, $R_{14}$ bis $R_{16}$ und $R_{19}$ die gleiche Bedeutung wie oben haben, unter $HR_{19}$-Abspaltung gebildeten Phosphorans mit einem Aldehyd der Formel

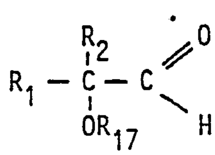

(X)

worin $R_1$, $R_2$ und $R_{17}$ die gleiche Bedeutung wie oben haben, bei Temperaturen von etwa —80 °C bis + 30 °C in Gegenwart eines inerten Lösungsmittels, oder

5) (wenn in der Verbindung der Formel II die Gruppe B für —CH=CH— steht)

i) durch Reaktion eines aus der Verbindung der Formel

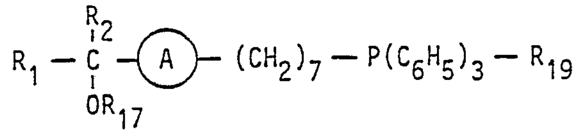

(XI)

worin A, $R_1$, $R_2$, $R_{17}$ und $R_{19}$ die gleiche Bedeutung wie oben haben durch Baseneinwirkung und $HR_{19}$-Abspaltung gebildeten Phosphorans mit einem Aldehyd der Formel

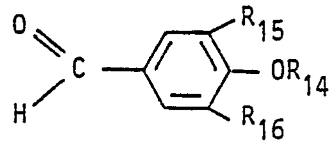

(XII)

worin $R_{14}$ bis $R_{16}$ die gleiche Bedeutung wie oben haben bei Temperaturen von etwa —80 °C bis +30 °C in Gegenwart eines inerten Lösungsmittels, oder

ii) durch Umsetzung des durch Baseneinwirkung auf die Verbindung der Formel

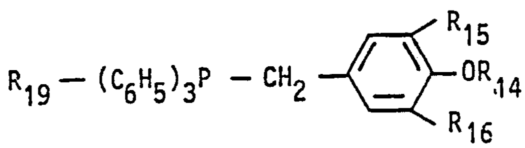

(XIII)

worin $R_{14}$ bis $R_{16}$ und $R_{19}$ die gleiche Bedeutung wie oben haben erhaltenen Phosphorans mit einem Aldehyd der Formel

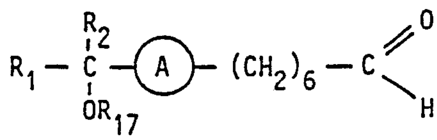

(XIV)

in der A, $R_1$, $R_2$ und $R_{17}$ die gleiche Bedeutung wie oben haben bei Temperaturen von etwa —80 °C bis +30 °C in Gegenwart eines inerten Lösungsmittels herstellt und aus der so erhaltenen Verbindung der Formel II in an sich bekannter Weise die in $R_{14}$ bis $R_{17}$ enthaltenen Schutzgruppen abspaltet und gegebenenfalls — wenn wenigstens einer der Reste $R_3$ und $R_4$ bzw. $R_5$ und $R_6$ einen Acetyl- oder Propionylrest darstellen bzw. enthalten soll — eine oder mehrere Acetyl- oder Propionylgruppe(n) einführt, oder

B) eine Verbindung der Formel

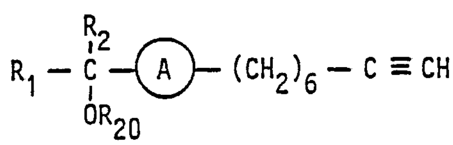

(XV)

worin A, $R_1$ und $R_2$ die gleiche Bedeutung wie oben haben und $R_{20}$ für ein Wasserstoffatom steht oder die gleiche Bedeutung wie $R_{17}$, hat, in Gegenwart eines bei etwa —10 °C bis +80 °C flüssigen sekundären oder tertiären Amins und katalytischer Mengen eines komplexen Palladiumkatalysators sowie gegebenenfalls katalytischer Mengen von Kupfer-(I)-jodid bei etwa 0 °C bis 75 °C mit einer Verbindung der Formel

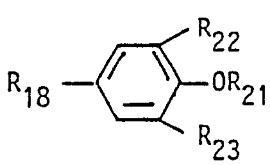

(XVI)

worin $R_{18}$ die gleiche Bedeutung wie oben hat, $R_{21}$ ein Wasserstoffatom, einen Alkylrest $R_9$ oder eine unter milden Bedingungen abspaltbare Schutzgruppe bedeutet, $R_{22}$ für die Gruppe $OR_{21}$ steht oder die gleiche Bedeutung wie $R_5$ mit der Maßgabe hat, daß darin $R_{10}$ nicht für ein Kation stehen kann und die in $R_5$ gegebenenfalls vorhandenen basischen Gruppen nicht in Salzform vorliegen können, oder $R_{22}$ zusammengenommen mit der Gruppe $OR_{21}$ für eine Methylendioxygruppe steht und $R_{23}$ die gleiche Bedeutung wie $R_{16}$ hat oder für eine Hydroxylgruppe steht, zur Reaktion bringt und aus der erhaltenen Verbindung der Formel

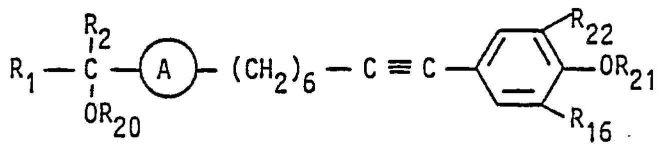

(XVII)

die gegebenenfalls in $R_{20}$ bis $R_{23}$ enthaltenen Schutzgruppen abspaltet und gegebenenfalls — wenn wenigstens einer der Reste $R_3$ und $R_4$ bzw. $R_5$ und $R_6$ einen Acetyl- oder Propionylrest darstellen bzw. enthalten soll — eine oder mehrere Acetyl- oder Propionylgruppe(n) einführt, oder

C) eine Verbindung der Formel

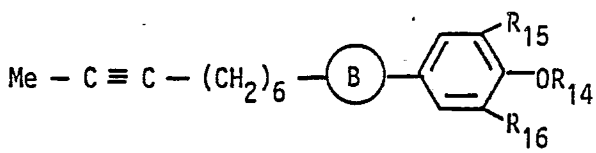

(XVIII)

worin Me, B und $R_{14}$ bis $R_{16}$ die gleiche Bedeutung wie oben haben, in Gegenwart eines indifferenten Lösungsmittels bei etwa —80 °C bis + 30 °C mit einer Verbindung der Formel

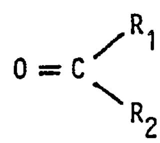

(XIX)

in der $R_1$ und $R_2$ die gleiche Bedeutung wie oben haben zu einer Verbindung der Formel

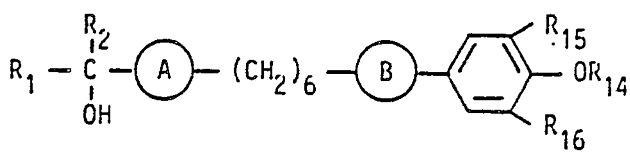

(XX)

worin A, B, $R_1$, $R_2$ und $R_{14}$ bis $R_{16}$ die gleiche Bedeutung wie oben haben umsetzt und aus dem Umsetzungsprodukt die gegebenenfalls in $R_{14}$ bis $R_{16}$ enthaltenen Schutzgruppen abspaltet sowie — wenn wenigstens einer der Reste $R_3$ und $R_4$ bzw. $R_5$ und $R_6$ einen Acetyl- oder Propionylrest darstellen bzw. enthalten soll — eine oder mehrere Acetyl- oder Propionylgruppen einführt, oder

D) eine Verbindung der Formel

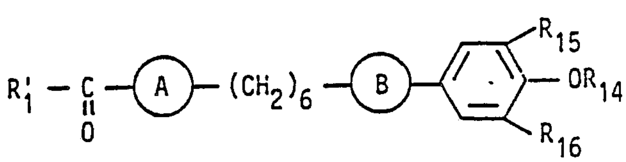

(XXI)

in der A, B und $R_{14}$ bis $R_{16}$ die gleiche Bedeutung wie oben haben und $R'_1$ mit Ausnahme von Wasserstoff die gleiche Bedeutung wie $R_1$ hat

1) durch Behandlung einer Verbindung der Formel

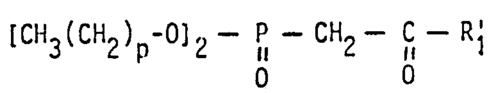

(XXII)

worin p und $R'_1$ die gleiche Bedeutung wie oben haben, in einem inerten Lösungsmittel mit einer wasserfreien Base und anschließende Umsetzung mit einer Verbindung der Formel VIII bei Temperaturen von etwa — 10 °C bis + 35 °C, oder

2) durch Umsetzung einer Verbindung der Formel

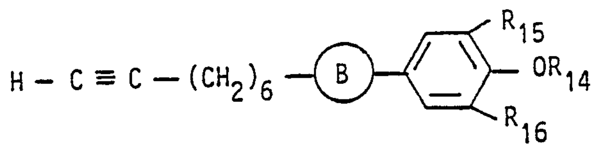
(XXIII)

in der B und $R_{14}$ bis $R_{16}$ die gleiche Bedeutung wie oben haben, in Gegenwart eines oberhalb etwa + 80 °C siedenden tertiären Amins und katalytischer Mengen eines komplexen Palladiumkatalysators sowie gegebenenfalls katalytischer Mengen von Kupfer-(I)-jodid bei etwa 0 °C bis 75 °C mit einer Verbindung der Formel

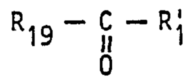
(XXIV)

worin $R'_1$ und $R_{19}$ die gleiche Bedeutung wie oben haben, herstellt und diese Verbindung der Formel XXI durch Reduktion mit einem Metallborhydrid oder durch Reaktion mit einer Verbindung der Formel $R'_2$-Me, worin Me die gleiche Bedeutung wie oben hat und $R'_2$ mit Ausnahme von Wasserstoff die gleiche Bedeutung wie $R_2$ hat, anschließende Abspaltung von in $R_{14}$ bis $R_{16}$ vorliegenden Schutzgruppen und gegebenenfalls — wenn wenigstens einer der Reste $R_3$ und $R_4$ bzw. $R_5$ und $R_6$ einen Acetyl- oder Propionylrest darstellen bzw. enthalten soll — Einführung einer oder mehrerer Acetyl- oder Propionylgruppen in die entsprechende Verbindung der Formel I (in der $R_1$ und gegebenenfalls $R_2$ von Wasserstoff verschieden sind) überführt, und in den nach A, B, C oder D erhaltenen Verbindungen die Reste $R_5$ bzw. $R_{15}$ oder $R_{22}$ in die gewünschte Gruppe $R_5$ überführt und/oder gegebenenfalls darin vorliegende Dreifachbindungen katalytisch zu Doppelbindungen hydriert.

2. Verfahren gemäß Anspruch 1, dadurch gekennzeichnet, daß die durch $R_{14}$, $R_{17}$, $R_{20}$ oder $R_{21}$ dargestellten bzw. die in $R_{15}$ und $R_{22}$ enthaltenen Schutzgruppen gleiche oder verschiedene Vertreter aus der den Tetrahydropyranyl-2-rest, den tert-Butyldimethylsilyl- und den tert-Butyldiphenylsilylrest umfassenden Gruppe sind.

3. Verfahren gemäß Ansprüchen 1 und 2, dadurch gekennzeichnet, daß man bei den Verfahrensweisen A1 und A3 in Gegenwart eines dipolaren aprotischen Lösungsmittels und eines Kupfer-(I)-salzes arbeitet.

4. Verfahren gemäß Ansprüchen 1 und 2, dadurch gekennzeichnet, daß bei den Verfahrensweisen A4 und A5 für die Phosphoranbildung durch $HR_{19}$-Abspaltung als wasserfreie Base n-Butyllithium, Kalium-tert-butylat oder Natrium-bis-(trimethylsilyl)-amid eingesetzt wird.

5. Verfahren gemäß Ansprüchen 1 und 2, dadurch gekennzeichnet, daß bei den Verfahrensweisen B und $D_2$ als komplexer Palladiumkatalysator Bis-(triphenylphosphin)-palladium-(II)-chlorid oder -acetat oder Tetrakis-(triphenylphosphin)-palladium eingesetzt werden und in Gegenwart eines Trialkylamins mit 2 oder 3 Kohlenstoffatomen in jedem der Alkylreste gearbeitet wird.

6. Verfahren gemäß Ansprüchen 1 und 5, dadurch gekennzeichnet, daß bei den Verfahrensweisen B und $D_2$ in Gegenwart eines komplexen Palladiumkatalysators und katalytischer Mengen von Kupfer-(I)-jodid gearbeitet wird.

7. Verfahren gemäß Anspruch 1, dadurch gekennzeichnet, daß man für die katalytische Hydrierung von in den Gruppen A oder B vorliegenden Dreifachbindungen zu Doppelbindungen mit Blei vergiftete Palladiumkatalysatoren einsetzt und in Gegenwart zumindest katalytischer Mengen von Pyridin oder Chinolin arbeitet.

8. Verfahren gemäß Anspruch 1, dadurch gekennzeichnet, daß man zur Herstellung von Verbindungen der Formel I, in denen $R_5$ für eine Gruppe der Formeln

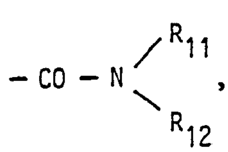 , 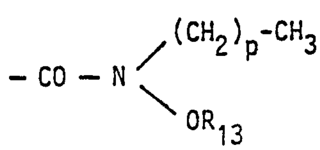 oder 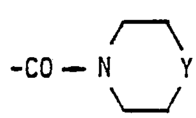

worin $R_{11}$ bis $R_{13}$, p und Y die gleiche Bedeutung wie oben haben, steht, eine Aminoverbindung der Formeln

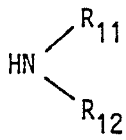
,
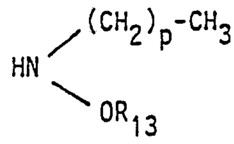
oder
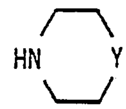

worin $R_{11}$ bis $R_{13}$, p und Y die gleiche Bedeutung wie oben haben,

a) mit einer Verbindung der Formel I, in der $R_5$ eine veresterte Carboxylgruppe ist, umsetzt, oder

b) mit einer Verbindung der Formel I, in der $R_5$ eine freie Carboxylgruppe darstellt, in Gegenwart wasserabspaltender Mittel oder nach intermediärer Bildung eines reaktiven funktionellen Derivates der Carboxylgruppe zur Reaktion bringt.

**Claims** (for the Contracting States : BE, CH, DE, FR, GB, IT, LI, LU, NL, SE)

1. Phenol derivatives of the general formula

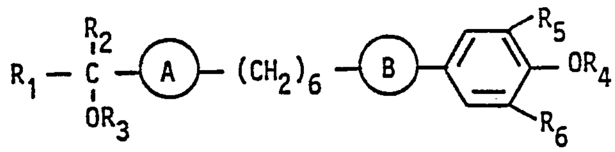
(I)

in which

A and B are identical or different and each represent one of the groups —C≡C—, cis-CH=CH— or trans-CH=CH—,

$R_1$ denotes hydrogen or a straight-chain alkyl radical with 1 to 6 carbon atoms, or a 5- to 7-membered cycloalkyl group, or a group of the formula $-(CH_2)_m-O-R_7$, wherein m represents one of the numbers 1, 2 or 3 and $R_7$ represents methyl or ethyl, or a group of the formula

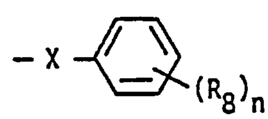

wherein X represents a single bond, a $-CH_2-$ group or a $-CH_2O-$ group and $R_8$ denotes a hydrogen, chlorine or fluorine atom or a methyl, methoxy or trifluoromethyl group and n represents one of the numbers 1 or 2,

$R_2$ represents hydrogen, methyl or ethyl,

$R_3$ denotes hydrogen or an acetyl or propionyl radical,

$R_4$ represents hydrogen, an acetyl or propionyl radical or a straight-chain or branched alkyl radical $R_9$ with 1 to 4 carbon atoms,

$R_5$ represents a group of the formula $-COOR_{10}$, wherein $R_{10}$ is a hydrogen atom, a pharmaceutically tolerated cation, in particular a monovalent cation, a straight-chain or branched alkyl radical with 1 to 6 carbon atoms or the group $-(CH_2)_2-N-[(CH_2)_p-CH_3]_2$, in which p represents zero or one of the numbers 1 to 3, and pharmaceutically tolerated salts of these basic esters with acids, or represents a group of the formula

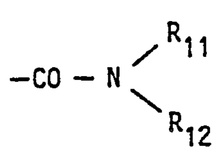

wherein $R_{11}$ and $R_{12}$ are identical or different and represent hydrogen, the alkyl radical $R_9$ or 2-hydroxyethyl, or one of these radicals denotes a hydroxyl group and the other denotes hydrogen, or $R_{11}$ and $R_{12}$ taken together represent the group $-(CH_2)_q-$, in which q represents one of the numbers 4, 5 or 6, or represents a group of the formula

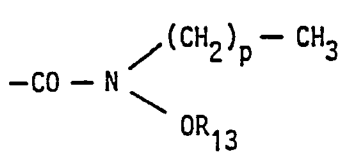

wherein $R_{13}$ represents hydrogen, $—(CH_2)_p—CH_3$, carboxymethyl, acetyl or propionyl and p in each case has the same meaning as above, or represents a group of the formula $—CO—NH—(CH_2)_r—N(CH_3)_2$, wherein r represents one of the numbers 2 or 3, or pharmaceutically tolerated salts thereof with acids, or represents a group of the formula

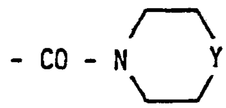

in which Y denotes an oxygen atom or the group $>N—CH_3$, or if appropriate pharmaceutically tolerated salts thereof with acids, or represents a nitrile radical, or represents a hydroxyl, acetoxy or propionyloxy group, an alkoxy group $OR_9$, in which $R_9$ has the same meaning as above, or — taken together with $OR_4$ — represents the methylenedioxy group and

$R_6$ represents hydrogen, a hydroxyl, acetoxy or propionyloxy group, the alkyl radical $R_9$ or an alkoxy group $OR_9$, wherein $R_9$ in each case has the same meaning as above.

2. Phenol derivatives of the formula

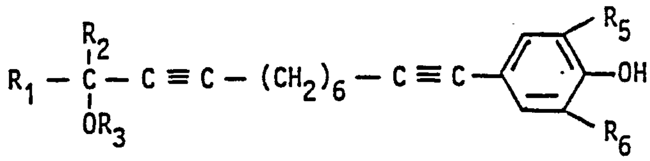

(I')

wherein $R_1$ to $R_3$ and $R_5$ and $R_6$ have the same meaning as in claim 1.

3. Phenol derivatives of the formula

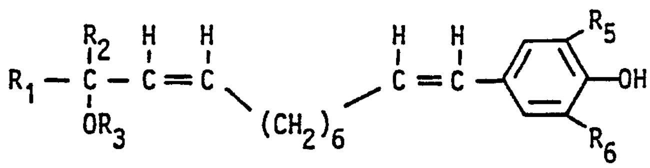

(I'')

wherein $R_1$ to $R_3$ and $R_5$ and $R_6$ have the same meaning as in claim 1.

4. Phenol derivatives of the formula

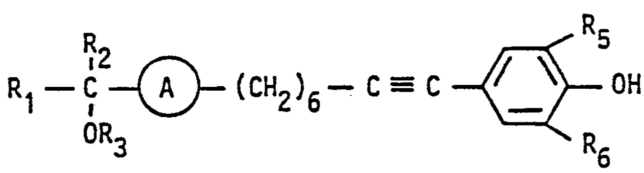

(I''')

wherein $R_1$ to $R_3$ and $R_5$, $R_6$ and A have the same meaning as in claim 1.

5. Phenol derivatives of the general formula

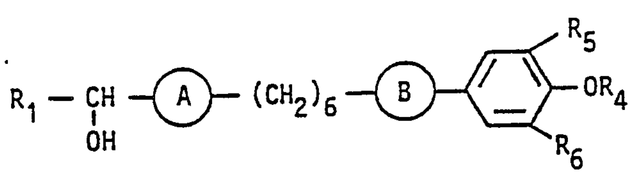

wherein $R_1$, $R_4$ to $R_6$, A and B have the same meaning as in claim 1.

6. Phenol derivatives of the formula

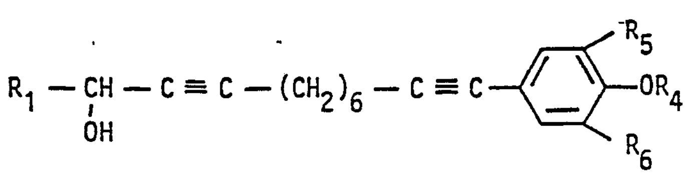

in which $R_1$ denotes a hydrogen atom, a straight-chain alkyl radical with 1 to 6 carbon atoms or a phenyl or cyclohexyl radical and $R_4$ to $R_6$ have the same meaning as in claim 1.

7. Phenol derivatives according to claims 1 to 6, in which $R_6$ represents a hydrogen atom or a methoxy group.

8. Phenol derivatives according to claims 1 to 7, in which $R_5$ represents one of the groups —OH, —$OR_9$,

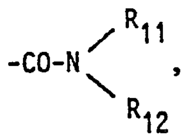 , 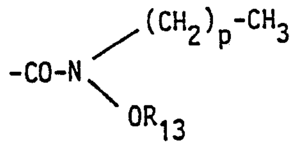 or 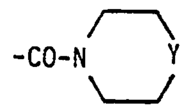

wherein $R_9$, $R_{11}$ to $R_{13}$, p and Y have the same meaning as in claim 1.

9. Phenol derivatives according to claims 1 to 8, in which $R_5$ represents one of the groups —OH, —$OCH_3$ or

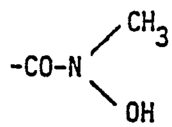

10. Medicament, characterized in that it contains as the active ingredient at least one of the phenol derivatives corresponding to claims 1 to 9 and is suitable for parenteral, oral, intranasal, percutaneous or rectal administration.

11. Medicament according to claim 10, characterized in that it contains as the active ingredient 0.01 to 50 mg of a phenol derivative corresponding to claims 1 to 9 per individual dose.

12. Medicament according to claims 10 and 11, characterized in that it is suitable for parenteral administration and contains 0.01 to 10 mg of a phenol derivative corresponding to claims 1 to 9.

13. Medicament according to claims 10 and 11, characterized in that it is suitable for oral administration and contains 0.1 to 50 mg of a phenol derivative corresponding to claims 1 to 9.

14. Medicament according to claim 13 for oral administration, characterized in that it is in the form of tablets, coated tablets or capsules, if appropriate with delayed release of the active ingredient.

15. Medicament according to claim 10 for intranasal, oral or peroral administration of a phenol derivative corresponding to claims 1 to 9 in spray form.

16. Medicament according to claim 10 for percutaneous administration, consisting of a reservoir which is to be applied to the skin and contains a phenol derivative corresponding to claims 1 to 9 in dissolved form, preferably with the addition of agents which promote penetration of the skin.

17. Process for the preparation of the medicaments according to claims 10 to 16, characterized in that the phenol derivatives corresponding to claims 1 to 9 are processed in the customary manner with excipient materials, solvents and/or diluents and if appropriate binders, tablet disintegrating agents and other customary auxiliaries and the individual dosage forms are produced from the resulting mixture.

18. Process for the preparation of the phenol derivatives of the formula I from claim 1, characterized in that

A) a compound of the general formula II

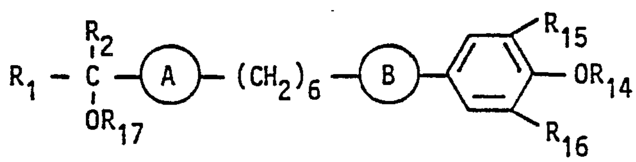 (II)

in which A, B, $R_1$ and $R_2$ have the same meaning as in formula I, $R_{14}$ represents the alkyl radical $R_9$ or a protective group which can be split off under mild conditions, $R_{15}$ represents the group $OR_{14}$ or has the same meaning as $R_5$, with the proviso that in this radical $R_{10}$ cannot represent a cation and the basic groups which may be present in $R_5$ cannot be in salt form, or $R_{15}$, taken together with the group $OR_{14}$, represents a methylenedioxy group, $R_{16}$ denotes a hydrogen atom or one of the groups $R_9$ or $OR_{14}$ and $R_{17}$ is a protective group which can be split off under mild conditions, is prepared

1) by reaction of a compound of the formula

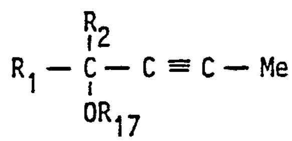 (III)

wherein $R_1$, $R_2$ and $R_{17}$ have the same meaning as above and Me represents a lithium, sodium or potassium atom or one of the radicals —MgBr or —MgI, with a compound of the formula

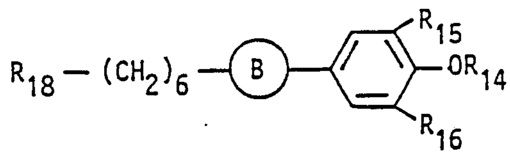

(IV)

in which B and $R_{14}$ to $R_{16}$ have the same meaning as above and $R_{18}$ represents a bromine or an iodine atom, at temperatures of about —80° to +75 °C in an inert, aprotic solvent, if appropriate in the presence of catalytic amounts of a copper salt, or

2) by reaction of a compound of the formula IV, in which $R_{15}$ represents the group $OR_{14}$, with a compound of the formula

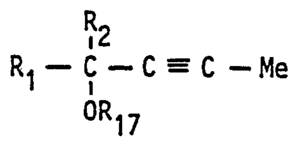

(IIIa)

wherein $R_1$, $R_2$ and $R_{17}$ have the same meaning as above and Me′ represents a lithium, sodium or potassium atom, in liquid ammonia at temperatures of about —80 °C to —35 °C, or

3) by reaction of a compound of the formula

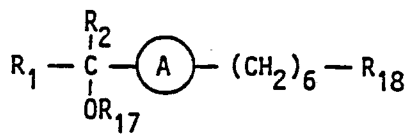

(V)

in which A, $R_1$, $R_2$, $R_{17}$ and $R_{18}$ have the same meaning as above, with a compound of the formula

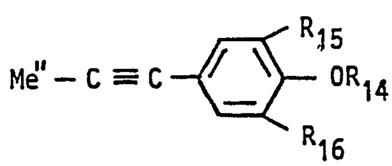

(VI)

wherein $R_{14}$ to $R_{16}$ have the same meaning as above and Me″ represents one of the groups Me or Me′ defined above, under the conditions mentioned above for 1 or 2, or

4) (if the group A in the compound of the formula II represents —CH=CH—)

i) by treatment of a compound of the formula

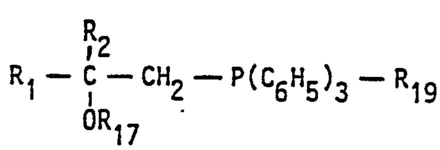

(VII)

in which $R_1$, $R_2$ and $R_{17}$ have the same meaning as above and $R_{19}$ represents a chlorine, bromine or iodine atom, with a strong anhydrous base in inert solvents, and reaction of the phosphorane formed by splitting off $HR_{19}$ with an aldehyde of the formula

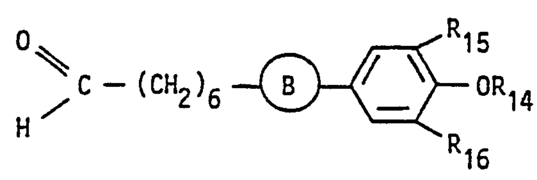

(VIII)

wherein B and $R_{14}$ to $R_{16}$ have the same meaning as above, at temperatures of about —80 °C to +30 °C, or

ii) by reaction of the phosphorane formed by the action of a base on the compound of the formula

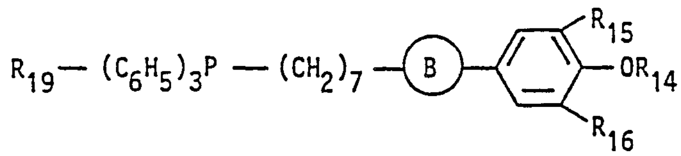

(IX)

wherein B, $R_{14}$ to $R_{16}$ and $R_{19}$ have the same meaning as above, $HR_{19}$ being split off, with an aldehyde of the formula

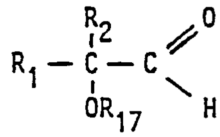

(X)

wherein $R_1$, $R_2$ and $R_{17}$ have the same meaning as above, at temperatures of about — 80 °C to + 30 °C in the presence of an inert solvent, or

5) (if the group B in the compound of the formula II represents —CH=CH—)

i) by reaction of a phosphorane formed from the compound of the formula

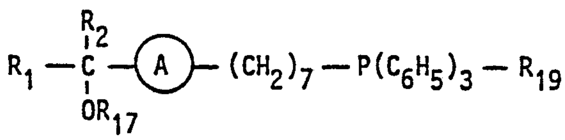

(XI)

wherein A, $R_1$, $R_2$, $R_{17}$ and $R_{19}$ have the same meaning as above by the action of a base and splitting off of $HR_{19}$, with an aldehyde of the formula

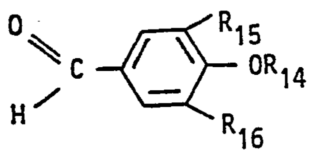

(XII)

wherein $R_{14}$ to $R_{16}$ have the same meaning as above at temperatures of about — 80 °C to + 30 °C in the presence of an inert solvent, or

ii) by reaction of the phosphorane obtained by the action of a base on the compound of the formula

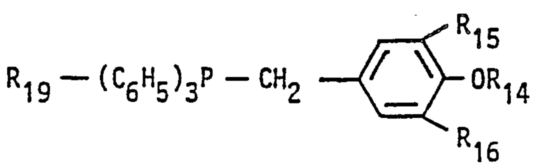

(XIII)

wherein $R_{14}$ to $R_{16}$ and $R_{19}$ have the same meaning as above with an aldehyde of the formula

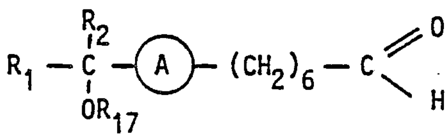

(XIV)

in which A, $R_1$, $R_2$ and $R_{17}$ have the same meaning as above at temperatures of about — 80 °C to + 30 °C in the presence of an inert solvent and the protective groups contained in $R_{14}$ to $R_{17}$ are split off from the resulting compound of the formula II in a manner which is known per se and if appropriate — if at least one of the radicals $R_3$ and $R_4$ or $R_5$ and $R_6$ is to represent or contain an acetyl or propionyl radical — one or more acetyl or propionyl group(s) is/are introduced, or

B) a compound of the formula

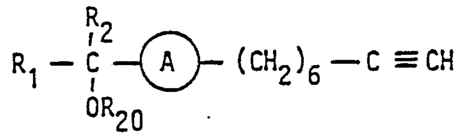

(XV)

wherein A, $R_1$ and $R_2$ have the same meaning as above and $R_{20}$ represents a hydrogen atom or has the same meaning as $R_{17}$ is reacted, in the presence of a secondary or tertiary amine which is liquid at about — 10 °C to + 80 °C and catalytic amounts of a complex palladium catalyst and if appropriate catalytic amounts of copper (I) iodide at about 0 °C to 75 °C, with a compound of the formula

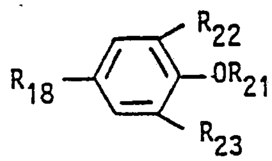

(XVI)

wherein $R_{18}$ has the same meaning as above, $R_{21}$ denotes a hydrogen atom, the alkyl radical $R_9$ or a protective group which can be split off under mild conditions, $R_{22}$ represents the group $OR_{21}$ or has the same meaning as $R_5$, with the proviso that in this radical $R_{10}$ cannot represent a cation and the basic groups which may be present in $R_5$ cannot be in salt form, or $R_{22}$, taken together with the group $OR_{21}$, represents a methylenedioxy group and $R_{23}$ has the same meaning as $R_{16}$ or represents a hydroxyl group, and the protective groups contained, if appropriate, in $R_{20}$ to $R_{23}$ are split off from the resulting compound of the formula

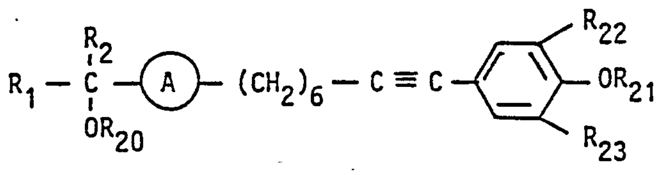

(XVII)

and if appropriate — if at least one of the radicals $R_3$ and $R_4$ or $R_5$ and $R_6$ is to represent or contain an acetyl or propionyl radical — one or more acetyl or propionyl group(s) is/are introduced, or

C) a compound of the formula

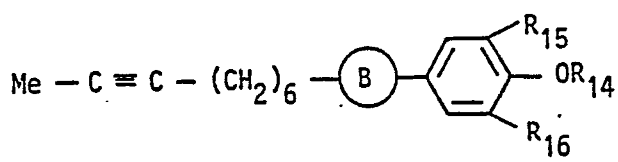

(XVIII)

wherein Me, B and $R_{14}$ to $R_{16}$ have the same meaning as above, is reacted in the presence of an inert solvent at about — 80 °C to + 30 °C with a compound of the formula

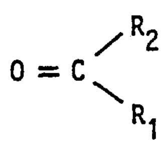

(XIX)

in which $R_1$ and $R_2$ have the same meaning as above to give a compound of the formula

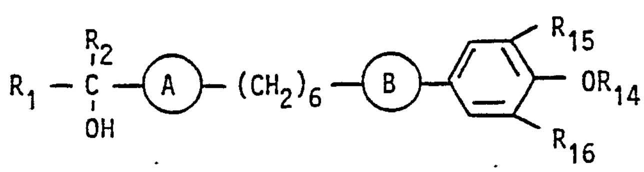

(XX)

wherein A, B, $R_1$, $R_2$ and $R_{14}$ to $R_{16}$ have the same meaning as above and the protective groups contained, if appropriate, in $R_{14}$ to $R_{16}$ are split off from the reaction product and — if at least one of the radicals $R_3$ and $R_4$ or $R_5$ and $R_6$ is to represent or contain an acetyl or propionyl radical — one or more acetyl or propionyl groups is/are introduced, or

D) a compound of the formula

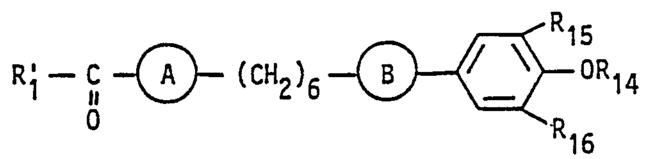

(XXI)

in which A, B and $R_{14}$ to $R_{16}$ have the same meaning as above and $R'_1$ has the same meaning as $R_1$, with the exception of hydrogen, is prepared

1) by treatment of a compound of the formula

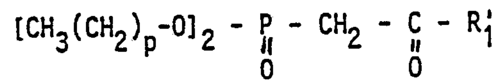

(XXII)

wherein p and $R'_1$ have the same meaning as above, with an anhydrous base in an inert solvent and subsequent reaction with a compound of the formula VIII at temperatures of about — 10 °C to + 35 °C, or

2) by reaction of a compound of the formula

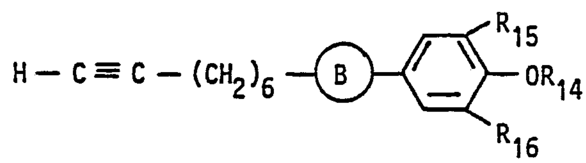

(XXIII)

in which B and $R_{14}$ to $R_{16}$ have the same meaning as above, in the presence of a tertiary amine with a boiling point above about + 80 °C and catalytic amounts of a complex palladium catalyst and if appropriate catalytic amounts of copper (I) iodide at about 0 °C to 75 °C, with a compound of the formula

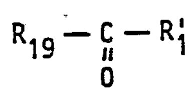

(XXIV)

wherein $R'_1$ and $R_{19}$ have the same meaning as above, and this compound of the formula XXI is converted into the corresponding compound of the formula I (in which $R_1$ and if appropriate $R_2$ are other than hydrogen) by reduction with a metal borohydride or by reaction with a compound of the formula $R'_2$-Me, wherein Me has the same meaning as above and $R'_2$ has the same meaning as $R_2$, with the exception of hydrogen, subsequent splitting off of protective groups present in $R_{14}$ to $R_{16}$ and if appropriate — if at least one of the radicals $R_3$ and $R_4$ or $R_5$ and $R_6$ is to represent or contain an acetyl or propionyl radical — introduction of one or more acetyl or propionyl groups, and the radicals $R_5$ and $R_{15}$ or $R_{22}$ in the compounds obtained according to A, B, C or D are converted into the desired group $R_5$ and/or if appropriate triple bonds present therein are hydrogenated catalytically to give double bonds.

19. Process according to claim 18, characterized in that the protective groups represented by $R_{14}$, $R_{17}$, $R_{20}$ or $R_{21}$ or contained in $R_{15}$ and $R_{22}$ are identical or different representatives from the group comprising the tetrahydropyran-2-yl radical, the tert.-butyldimethylsilyl radical and the tert.-butyldiphenylsilyl radical.

20. Process according to claims 18 and 19, characterized in that procedures A1 and A3 are carried out in the presence of a dipolar aprotic solvent and a copper (I) salt.

21. Process according to claims 18 and 19, characterized in that n-butyllithium, potassium tert.-butylate or sodium bis-(trimethylsilyl)-amide is employed as the anhydrous base for the phosphorane formation by splitting off $HR_{19}$ in procedures A4 and A5.

22. Process according to claims 18 and 19, characterized in that bis-(triphenylphosphine)-palladium (II) chloride or acetate or tetrakis-(triphenylphosphine)-palladium are employed as the complex palladium catalyst in procedures B and $D_2$ and these procedures are carried out in the presence of a trialkylamine with 2 or 3 carbon atoms in each of the alkyl radicals.

**Claims** (for the Contracting State AT)

1. Process for the preparation of phenol derivatives of the general formula

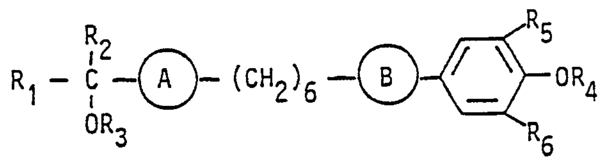

(I)

in which

A and B are identical or different and each represent one of the groups —C≡C—, cis-CH=CH— or trans-CH=CH—,

$R_1$ denotes hydrogen or a straight-chain alkyl radical with 1 to 6 carbon atoms, or a 5- to 7-membered cycloalkyl group, or a group of the formula $-(CH_2)_m-O-R_7$, wherein m represents one of the numbers 1, 2 or 3 and $R_7$ represents methyl or ethyl, or a group of the formula

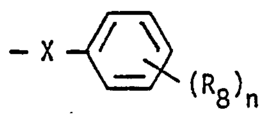
.

wherein X represents a single bond, a $-CH_2-$ group or a $-CH_2O-$ group and $R_8$ denotes a hydrogen, chlorine or fluorine atom or a methyl, methoxy or trifluoromethyl group and n represents one of the numbers 1 or 2,

$R_2$ represents hydrogen, methyl or ethyl,

$R_3$ denotes hydrogen or an acetyl or propionyl radical,

$R_4$ represents hydrogen, an acetyl or propionyl radical or a straight-chain or branched alkyl radical $R_9$ with 1 to 4 carbon atoms,

$R_5$ represents a group of the formula $-COOR_{10}$, wherein $R_{10}$ is a hydrogen atom, a pharmaceutically tolerated cation, in particular a monovalent cation, a straight-chain or branched alkyl radical with 1 to 6 carbon atoms or the group $-(CH_2)_2-N-[(CH_2)_p-CH_3]_2$, in which p represents zero or one of the numbers 1 to 3, and pharmaceutically tolerated salts of these basic esters with acids, or represents a group of the formula

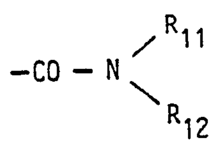

wherein $R_{11}$ and $R_{12}$ are identical or different and represent hydrogen, the alkyl radical $R_9$ or 2-hydroxyethyl, or one of these radicals denotes a hydroxyl group and the other denotes hydrogen, or $R_{11}$ and $R_{12}$ taken together represent the group $-(CH_2)_q-$, in which q represents one of the numbers 4, 5 or 6, or represents a group of the formula

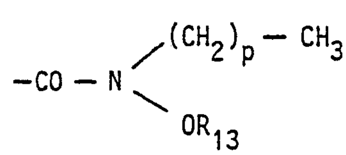

wherein $R_{13}$ represents hydrogen, $-(CH_2)_p-CH_3$, carboxymethyl, acetyl or propionyl and p in each case has the same meaning as above, or represents a group of the formula $-CO-NH-(CH_2)_r-N(CH_3)_2$, wherein r represents one of the numbers 2 or 3, or pharmaceutically tolerated salts thereof with acids, or represents a group of the formula

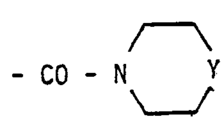

in which Y denotes an oxygen atom or the group $>N-CH_3$, or if appropriate pharmaceutically tolerated salts thereof with acids, or represents a nitrile radical, or represents a hydroxyl, acetoxy or

propionyloxy group, an alkoxy group $OR_9$, in which $R_9$ has the same meaning as above, or — taken together with $OR_4$ — represents the methylenedioxy group and

$R_6$ represents hydrogen, a hydroxyl, acetoxy or propionyloxy group, the alkyl radical $R_9$ or an alkoxy group $OR_9$, wherein $R_9$ in each case has the same meaning as above, characterized in that

A) a compound of the general formula II

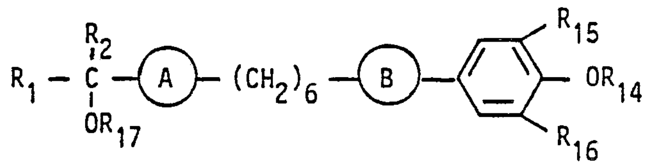

(II)

in which A, B, $R_1$ and $R_2$ have the same meaning as in formula I, $R_{14}$ represents the alkyl radical $R_9$ or a protective group which can be split off under mild conditions, $R_{15}$ represents the group $OR_{14}$ or has the same meaning as $R_5$, with the proviso that in this radical $R_{10}$ cannot represent a cation and the basic groups which may be present in $R_5$ cannot be in salt form, or $R_{15}$, taken together with the group $OR_{14}$, represents a methylenedioxy group, $R_{16}$ denotes a hydrogen atom or one of the groups $R_9$ or $OR_{14}$ and $R_{17}$ is a protective group which can be split off under mild conditions, is prepared

1) by reaction of a compound of the formula

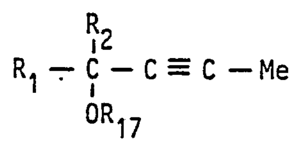

(III)

wherein $R_1$, $R_2$ and $R_{17}$ have the same meaning as above and Me represents a lithium, sodium or potassium atom or one of the radicals —MgBr or —MgI, with a compound of the formula

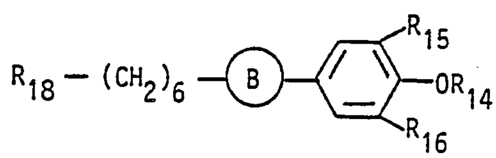

(IV)

in which B and $R_{14}$ to $R_{16}$ have the same meaning as above and $R_{18}$ represents a bromine or an iodine atom, at temperatures of about —80° to +75 °C in an inert, aprotic solvent, if appropriate in the presence of catalytic amounts of a copper salt, or

2) by reaction of a compound of the formula IV, in which $R_{15}$ represents the group $OR_{14}$, with a compound of the formula

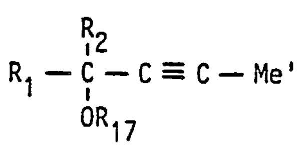

(IIIa)

wherein $R_1$, $R_2$ and $R_{17}$ have the same meaning as above and Me′ represents a lithium, sodium or potassium atom, in liquid ammonia at temperatures of about —80 °C to —35 °C, or

3) by reaction of a compound of the formula

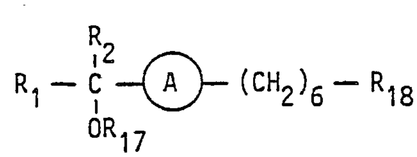

(V)

in which A, $R_1$, $R_2$, $R_{17}$ and $R_{18}$ have the same meaning as above, with a compound of the formula

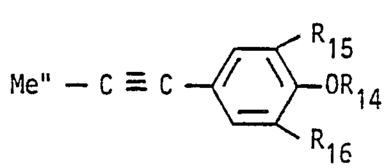

(VI)

wherein $R_{14}$ to $R_{16}$ have the same meaning as above and Me″ represents one of the groups Me or Me′ defined above, under the conditions mentioned above for 1 or 2, or

4) (if the group A in the compound of the formula II represents —CH=CH—)

i) by treatment of a compound of the formula

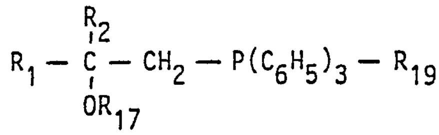

(VII)

in which $R_1$, $R_2$ and $R_{17}$ have the same meaning as above and $R_{19}$ represents a chlorine, bromine or iodine atom, with a strong anhydrous base in inert solvents, and reaction of the phosphorane formed by splitting off $HR_{19}$ with an aldehyde of the formula

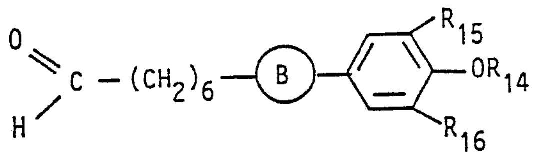

(VIII)

wherein B and $R_{14}$ to $R_{16}$ have the same meaning as above, at temperatures of about —80 °C to +30 °C, or

ii) by reaction of the phosphorane formed by the action of a base on the compound of the formula

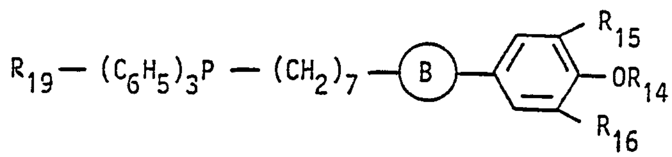

(IX)

wherein B, $R_{14}$ to $R_{16}$ and $R_{19}$ have the same meaning as above, $HR_{19}$ being split off, with an aldehyde of the formula

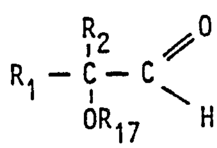

(X)

wherein $R_1$, $R_2$ and $R_{17}$ have the same meaning as above, at temperatures of about —80 °C to +30 °C in the presence of an inert solvent, or

5) (if the group B in the compound of the formula II represents —CH=CH—)

i) by reaction of a phosphorane formed from the compound of the formula

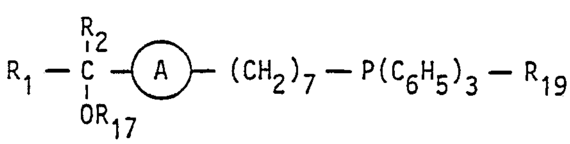

(XI)

wherein A, $R_1$, $R_2$, $R_{17}$ and $R_{19}$ have the same meaning as above by the action of a base and splitting off of $HR_{19}$, with an aldehyde of the formula

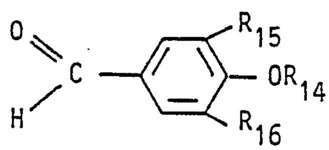

(XII)

wherein $R_{14}$ to $R_{16}$ have the same meaning as above at temperatures of about —80 °C to +30 °C in the presence of an inert solvent, or

ii) by reaction of the phosphorane obtained by the action of a base on the compound of the formula

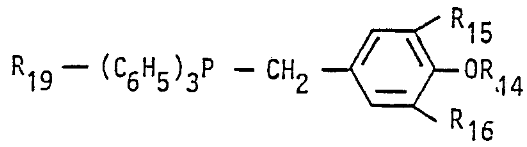

(XIII)

wherein $R_{14}$ to $R_{16}$ and $R_{19}$ have the same meaning as above with an aldehyde of the formula

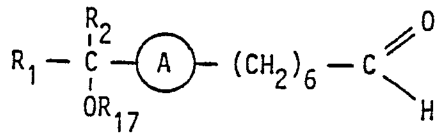

(XIV)

in which A, $R_1$ $R_2$ and $R_{17}$ have the same meaning as above at temperatures of about — 80 °C to + 30 °C in the presence of an inert solvent and the protective groups contained in $R_{14}$ to $R_{17}$ are split off from the resulting compound of the formula II in a manner which is known per se and if appropriate — if at least one of the radicals $R_3$ and $R_4$ or $R_5$ and $R_6$ is to represent or contain an acetyl or propionyl radical — one or more acetyl or propionyl group(s) is/are introduced, or

B) a compound of the formula

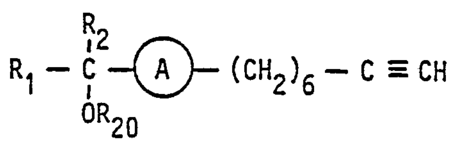

(XV)

wherein A, $R_1$ and $R_2$ have the same meaning as above and $R_{20}$ represents a hydrogen atom or has the same meaning as $R_{17}$ is reacted, in the presence of a secondary or tertiary amine which is liquid at about — 10 °C to + 80 °C and catalytic amounts of a complex palladium catalyst and if appropriate catalytic amounts of copper (I) iodide at about 0 °C to 75 °C, with a compound of the formula

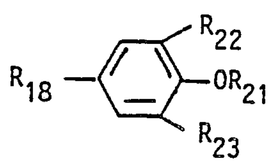

(XVI)

wherein $R_{18}$ has the same meaning as above, $R_{21}$ denotes a hydrogen atom, the alkyl radical $R_9$ or a protective group which can be split off under mild conditions, $R_{22}$ represents the group $OR_{21}$ or has the same meaning as $R_5$, with the proviso that in this radical $R_{10}$ cannot represent a cation and the basic groups which may be present in $R_5$ cannot be in salt form, or $R_{22}$, taken together with the group $OR_{21}$, represents a methylenedioxy group and $R_{23}$ has the same meaning as $R_{16}$ or represents a hydroxyl group, and the protective groups contained, if appropriate, in $R_{20}$ to $R_{23}$ are split off from the resulting compound of the formula

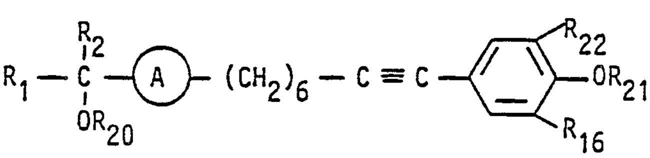

(XVII)

and if appropriate — if at least one of the radicals $R_3$ and $R_4$ or $R_5$ and $R_6$ is to represent or contain an acetyl or propionyl radical — one or more acetyl or propionyl group(s) is/are introduced, or

C) a compound of the formula

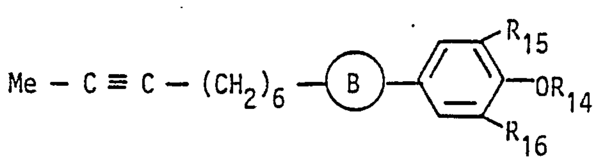

(XVIII)

wherein Me, B and $R_{14}$ to $R_{16}$ have the same meaning as above, is reacted in the presence of an inert solvent at about — 80 °C to + 30 °C with a compound of the formula

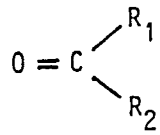

(XIX)

in which $R_1$ and $R_2$ have the same meaning as above to give a compound of the formula

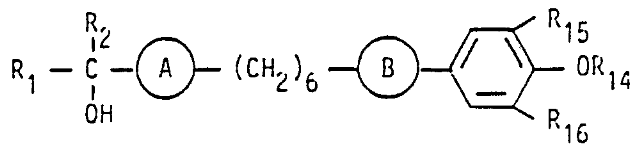

(XX)

wherein A, B, $R_1$, $R_2$ and $R_{14}$ to $R_{16}$ have the same meaning as above and the protective groups contained, if appropriate, in $R_{14}$ to $R_{16}$ are split off from the reaction product and — if at least one of the radicals $R_3$ and $R_4$ or $R_5$ and $R_6$ is to represent or contain an acetyl or propionyl radical — one or more acetyl or propionyl groups is/are introduced, or

D) a compound of the formula

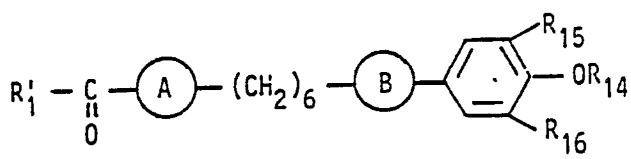

(XXI)

in which A, B and $R_{14}$ to $R_{16}$ have the same meaning as above and $R'_1$ has the same meaning as $R_1$, with the exception of hydrogen, is prepared

1) by treatment of a compound of the formula

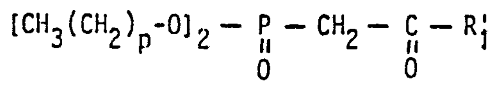

(XXII)

wherein p and $R'_1$ have the same meaning as above, with an anhydrous base in an inert solvent and subsequent reaction with a compound of the formula VIII at temperatures of about — 10 °C to + 35 °C, or

2) by reaction of a compound of the formula

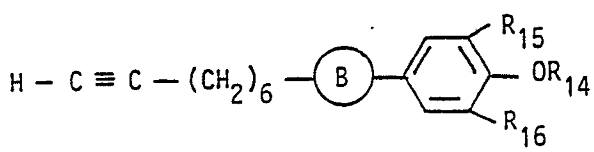

(XXIII)

in which B and $R_{14}$ to $R_{16}$ have the same meaning as above, in the presence of a tertiary amine with a boiling point above about + 80 °C and catalytic amounts of a complex palladium catalyst and if appropriate catalytic amounts of copper (I) iodide at about 0 °C to 75 °C, with a compound of the formula

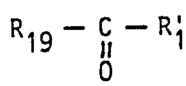

(XXIV)

wherein $R'_1$ and $R_{19}$ have the same meaning as above, and this compound of the formula XXI is converted into the corresponding compound of the formula I (in which $R_1$ and if appropriate $R_2$ are other than hydrogen) by reduction with a metal borohydride or by reaction with a compound of the formula $R'_2$-Me, wherein Me has the same meaning as above and $R'_2$ has the same meaning as $R_2$, with the exception of hydrogen, subsequent splitting off of protective groups present in $R_{14}$ to $R_{16}$ and if appropriate — if at least one of the radicals $R_3$ and $R_4$ or $R_5$ and $R_6$ is to represent or contain an acetyl or propionyl radical —

introduction of one or more acetyl or propionyl groups, and the radicals $R_5$ and $R_{15}$ or $R_{22}$ in the compounds obtained according to A, B, C or D are converted into the desired group $R_5$ and/or if appropriate triple bonds present therein are hydrogenated catalytically to give double bonds.

2. Process according to claim 1, characterized in that the protective groups represented by $R_{14}$ $R_{17}$, $R_{20}$ or $R_{21}$ or contained in $R_{15}$ and $R_{22}$ are identical or different representatives from the group comprising the tetrahydropyran-2-yl radical, the tert.-butyldimethylsilyl radical and the tert.-butyldiphenylsilyl radical.

3. Process according to claims 1 and 2, characterized in that procedures A1 and A3 are carried out in the presence of a dipolar aprotic solvent and a copper (I) salt.

4. Process according to claims 1 and 2, characterized in that n-butyllithium, potassium tert.-butylate or sodium bis-(trimethylsilyl)-amide is employed as the anhydrous base for the phosphorane formation by splitting off $HR_{19}$ in procedures A4 and A5.

5. Process according to claims 1 and 2, characterized in that bis-(triphenylphosphine)-palladium (II) chloride or acetate or tetrakis-(triphenylphosphine)-palladium are employed as the complex palladium catalyst in procedures B and $D_2$ and these procedures are carried out in the presence of a trialkylamine with 2 or 3 carbon atoms in each of the alkyl radicals.

6. Process according to claims 1 and 5, characterized in that procedures B and $D_2$ are carried out in the presence of a complex palladium catalyst and catalytic amounts of copper (I) iodide.

7. Process according to claim 1, characterized in that palladium catalysts poisoned with lead are employed for the catalytic hydrogenation of triple bonds present in the groups A or B and the hydrogenation is carried out in the presence of at least catalytic amounts of pyridine or quinoline.

8. Process according to claim 1, characterized in that, for the preparation of compounds of the formula I in which $R_5$ represents a group of the formulae

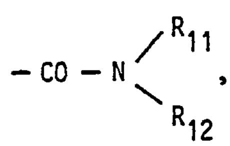 , 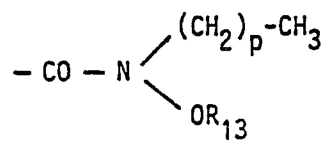 or 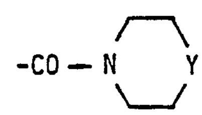

wherein $R_{11}$ to $R_{13}$, p and Y have the same meaning as above, an amino compound of the formulae

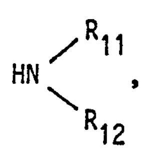 , 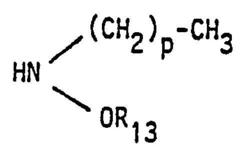 or 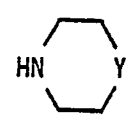

wherein $R_{11}$ to $R_{13}$, p and Y have the same meaning as above,

a) is reacted with a compound of the formula I in which $R_5$ is an esterified carboxyl group; or

b) is reacted with a compound of the formula 1 in which $R_5$ represents a free carboxyl group in the presence of dehydrating agents or after intermediate formation of a reactive functional derivative of the carboxyl group.

**Revendications** (pour les Etats contractants : BE, CH, DE, FR, GB, IT, LI, LU, NL, SE)

1. Dérivés de phénol de formule générale

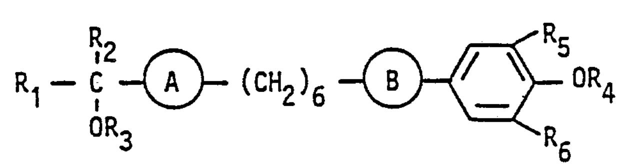

(I)

dans laquelle

A et B sont identiques ou différents et représentent chacun l'un des groupes —C≡C—, cis-CH=CH— ou trans-CH=CH—,

$R_1$ est l'hydrogène ou un reste alkyle à chaîne droite ayant 1 à 6 atomes de carbone ou un groupe cycloalkyle pentagonal à heptagonal ou un groupe de formule $—(CH_2)_m—O-R_7$, dans laquelle m est l'un des nombres 1, 2 et 3 et $R_7$ est un groupe méthyle ou éthyle, ou bien un groupe de formule

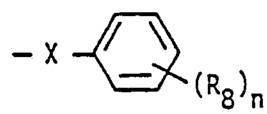

dans laquelle X est une liaison simple, un groupe —CH2— ou un groupe $—CH_2O—$ et $R_8$ est un atome d'hydrogène, de chlore ou de fluor ou un groupe méthyle, méthoxy ou trifluorométhyle et n est l'un des nombres 1 et 2,

$R_2$ est l'hydrogène, un groupe méthyle ou éthyle,

$R_3$ est l'hydrogène ou un reste acétyle ou propionyle,

$R_4$ est l'hydrogène, un reste acétyle ou propionyle ou un reste alkyle $R_9$ à chaîne droite ou à chaîne ramifiée ayant 1 à 4 atomes de carbone,

$R_5$ est un groupe de formule $—COOR_{10}$ dans laquelle $R_{10}$ est un atome d'hydrogène, un cation acceptable du point de vue pharmaceutique, notamment un cation monovalent, un reste alkyle à chaîne droite ou à chaîne ramifiée ayant 1 à 6 atomes de carbone ou le groupe $—(CH_2)_2—N— [(CH_2)_p—CH_3]_2$ dans lequel p est égal à zéro ou à l'un des nombres 1 à 3, ainsi que des sels pharmaceutiquement acceptables de ces esters basiques avec des acides, ou bien un groupe de formule

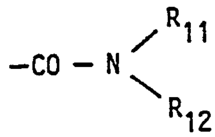

dans laquelle $R_{11}$ et $R_{12}$ sont identiques ou différents et représentent l'hydrogène, le reste alkyle $R_9$ ou un reste 2-hydroxyéthyle ou bien l'un de ces restes représente un groupe hydroxyle, l'autre représente un atome d'hydrogène, ou bien $R_{11}$ et $R_{12}$ forment conjointement le groupe $—(CH_2)_q—$ dans lequel q est l'un des nombres 4, 5 et 6, ou bien un groupe de formule

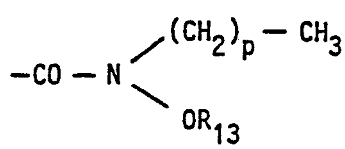

dans laquelle $R_{13}$ est l'hydrogène, un groupe $—(CH_2)_p—CH_3$, carboxyméthyle, acétyle ou propionyle et p a dans chaque cas la même définition que ci-dessus, ou bien un groupe de formule $—CO—NH—(CH_2)_r—N(CH_3)_2$, dans laquelle r est le nombre 2 ou 3, et ses sels d'acides pharmaceutiquement acceptables, ou bien un groupe de formule

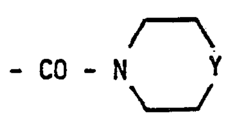

dans laquelle Y est un atome d'oxygène ou le groupe $>N—CH_3$ et le cas échéant ses sels d'acides pharmaceutiquement acceptables, ou bien un reste nitrile, ou bien un groupe hydroxy, acétyloxy ou propionyloxy, un groupe alkoxy $OR_9$ dans lequel $R_9$ a la même définition que ci-dessus, ou bien — conjointement avec $OR_4$ — le groupe méthylènedioxy et

$R_6$ est l'hydrogène, un groupe hydroxy, acetyloxy ou propionyloxy, un reste alkyle $R_9$ ou un groupe alkoxy $OR_9$ dans laquelle $R_9$ a dans chaque cas la même définition que ci-dessus.

2. Dérivés de phénol de formule

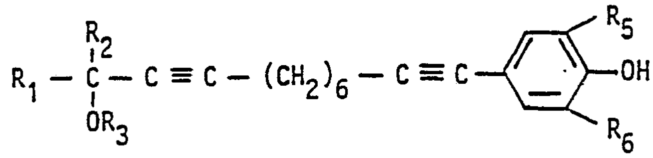

(I')

dans laquelle $R_1$ à $R_3$ ainsi que $R_5$ et $R_6$ ont la même définition que dans la revendication 1.

3. Dérivés de phénol de formule

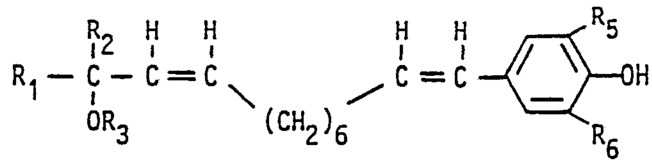 (I'')

dans laquelle $R_1$ à $R_3$ ainsi que $R_5$ et $R_6$ ont la même définition que dans la revendication 1.

4. Dérivés de phénol de formule

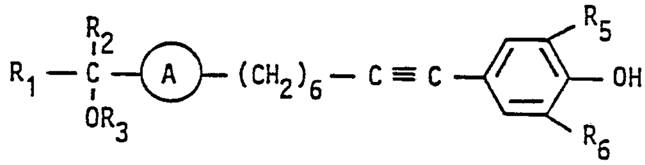 (I''')

dans laquelle $R_1$ à $R_3$ ainsi que $R_5$, $R_6$ et A ont la même définition que dans la revendication 1.

5. Dérivés de phénol de formule générale

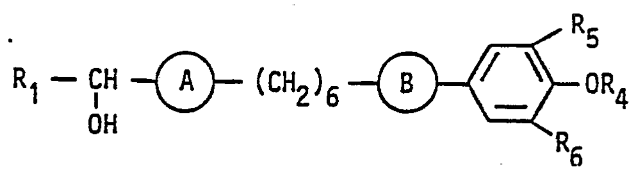

dans laquelle $R_1$, $R_4$ à $R_6$, A et B ont la même définition que dans la revendication 1.

6. Dérivés de phénol de formule

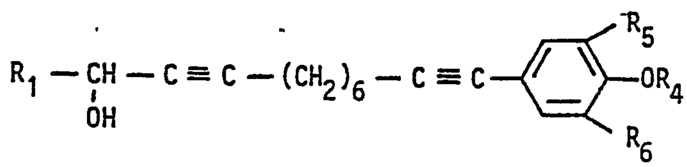

dans laquelle $R_1$ est un atome d'hydrogène, un reste alkyle à chaîne droite ayant 1 à 6 atomes de carbone ou un reste phényle ou cyclohexyle et $R_4$ à $R_6$ ont la même définition que dans la revendication 1.

7. Dérivés de phénol suivant les revendications 1 à 6, dans lesquels $R_6$ est un atome d'hydrogène ou un groupe méthoxy.

8. Dérivés de phénol suivant les revendications 1 à 7, dans lesquels $R_5$ est l'un des groupes —OH, —$OR_9$,

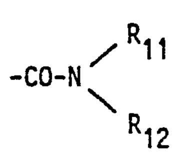 , 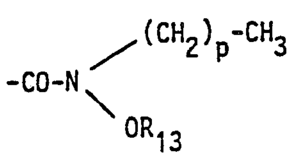 ou 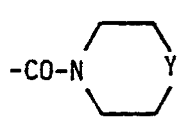

où $R_9$ $R_{11}$ à $R_{13}$ p et Y ont la même définition que dans la revendication 1.

9. Dérivés de phénol suivant les revendications 1 à 8, dans lesquels $R_5$ est l'un des groupes —OH, —$OCH_3$ ou

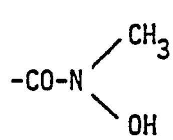

10. Médicament caractérisé en ce qu'il contient comme substance active au moins l'un des dérivés de phénol correspondant aux revendications 1 à 9 et en ce qu'il convient pour l'administration parentérale, orale, intranasale, percutanée ou rectale.

11. Médicament suivant la revendication 10, caractérisé en ce qu'il contient comme substance active, par dose individuelle, 0,01 à 50 mg d'un dérivé de phénol correspondant aux revendications 1 à 9.

12. Médicament suivant les revendications 10 et 11, caractérisé en ce qu'il convient pour l'administration parentérale et en ce qu'il contient 0,01 à 10 mg d'un dérivé de phénol correspondant aux revendications 1 à 9.

13. Médicament suivant les revendications 10 et 11, caractérisé en ce qu'il convient pour l'administration orale et en ce qu'il contient 0,1 à 50 mg d'un dérivé de phénol correspondant aux revendications 1 à 9.

14. Médicament suivant la revendication 13 pour l'administration orale, caractérisé en ce qu'il se présente sous la forme de comprimés, de dragées ou de gélules, le cas échéant avec libération retardée de la substance active.

15. Médicament suivant la revendication 10 pour l'administration intranasale, orale ou pérorale d'un dérivé de phénol correspondant aux revendications 1 à 9, sous la forme pulvérisable.

16. Médicament suivant la revendication 10, destiné à l'administration percutanée, constitué d'une réserve à appliquer sur la peau, contenant un dérivé de phénol suivant les revendications 1 à 9 sous la forme dissoute, de préférence avec addition d'agents favorisant la pénétration à travers la peau.

17. Procédé de préparation des médicaments suivant les revendications 10 à 16, caractérisé en ce qu'on met en œuvre les dérivés phénoliques correspondant aux revendications 1 à 9 de manière classique avec des excipients, des solvants ou des diluants, ainsi que le cas échéant des liants, des agents de désintégration de comprimés et d'autres substances auxiliaires classiques et on prépare les formes dosées individuelles à partir du mélange obtenu.

18. Procédé de préparation des dérivés phénoliques de formule I suivant la revendication 1, caractérisé en ce que :

A) On prépare un composé de formule générale II

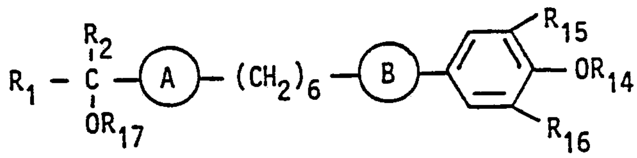

(II)

dans laquelle A, B, $R_1$ et $R_2$ ont la même définition que dans la formule I, $R_{14}$ est le reste alkyle $R_9$ ou un groupe protecteur éliminable dans des conditions douces, $R_{15}$ représente le groupe $OR_{14}$ ou a la même définition que $R_5$ sous réserve que $R_{10}$ ne puisse pas y représenter un cation et que les groupes basiques éventuellement présents dans $R_5$ ne puissent pas être sous la forme d'un sel, ou bien $R_{15}$, conjointement avec le groupe $OR_{14}$, représente un groupe méthylènedioxy, $R_{16}$ est un atome d'hydrogène ou l'un des groupes $R_9$ ou $OR_{14}$ et $R_{17}$ est un groupe protecteur éliminable dans des conditions douces,

1) par réaction d'un composé de formule

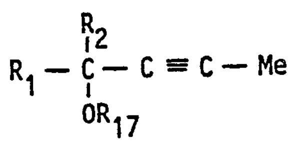

(III)

dans laquelle $R_1$, $R_2$ et $R_{17}$ ont la même définition que ci-dessus et Me est un atome de lithium, de sodium ou de potassium ou l'un des restes —MgBr ou —MgI, avec un composé de formule

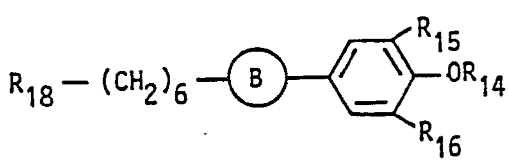

(IV)

dans laquelle B et $R_{14}$ à $R_{16}$ ont la même définition que ci-dessus et $R_{18}$ représente un atome de brome ou un atome d'iode, à des températures d'environ —80° à +75 °C dans un solvant aprotique inerte, éventuellement en présence de quantités catalytiques d'un sel de cuivre, ou bien

2) par réaction d'un compose de formule IV, dans laquelle $R_{15}$ est le groupe $OR_{14}$, avec un composé de formule

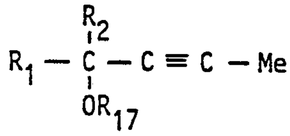

(IIIa)

dans laquelle $R_1$, $R_2$ et $R_{17}$ ont la même définition que ci-dessus et Me' représente un atome de lithium, de sodium ou de potassium, dans l'ammoniac liquide à des températures d'environ — 80 °C à — 35 °C, ou bien

3) par réaction d'un composé de formule

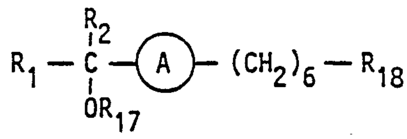

(V)

dans laquelle A, $R_1$, $R_2$, $R_{17}$ et $R_{18}$ ont la même définition que ci-dessus avec un composé de formule

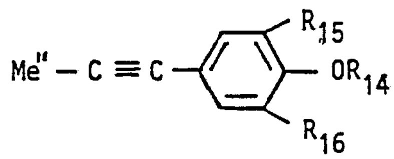

(VI)

dans laquelle $R_{14}$ à $R_{16}$ ont la même définition que ci-dessus et Me" représente l'un des groupes Me ou Me' définis ci-dessus, dans les conditions mentionnées ci-dessus en 1 et 2, ou bien

4) (lorsque, dans le composé de formule II, le groupe A est un groupe —CH=CH—)

i) par traitement d'un composé de formule

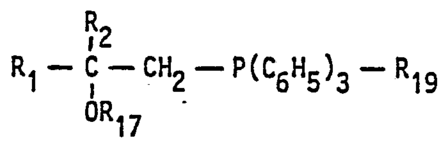

(VII)

dans laquelle $R_1$, $R_2$ et $R_{17}$ ont la même définition que ci-dessus et $R_{19}$ est un atome de chlore, de brome ou d'iode, dans des solvants inertes avec une base forte anhydre, et réaction du phosphoranne formé par élimination de $HR_{19}$ avec un aldéhyde de formule

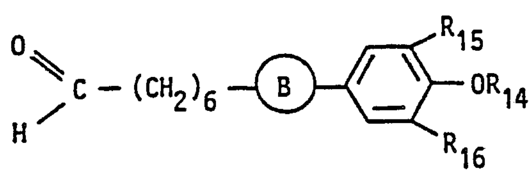

(VIII)

dans laquelle B et $R_{14}$ à $R_{16}$ ont la même définition que ci-dessus, à des températures d'environ — 80 °C à + 30 °C, ou bien

ii) par réaction du phosphoranne formé avec élimination de $HR_{19}$ par l'action d'une base sur le composé de formule

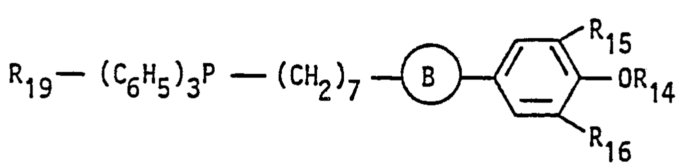

(IX)

dans laquelle B, $R_{14}$ à $R_{16}$ et $R_{19}$ ont la même définition que ci-dessus, avec un aldéhyde de formule

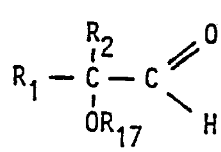

(X)

dans laquelle $R_1$, $R_2$ et $R_{17}$ ont la même définition que ci-dessus, à des températures d'environ — 80 °C à + 30 °C en présence d'un solvant inerte, ou bien

5) (lorsque, dans le composé de formule II, le groupe B est un groupe —CH=CH—)

i) par réaction d'un phosphoranne formé à partir du composé de formule

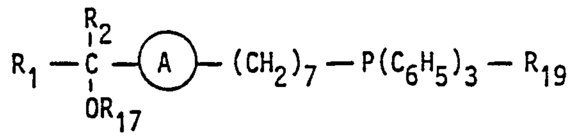

(XI)

dans laquelle A, $R_1$, $R_2$, $R_{17}$ et $R_{19}$ ont la même définition que ci-dessus, par l'action d'une base et avec élimination de $HR_19$, avec un aldéhyde de formule

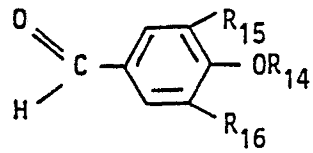

(XII)

dans laquelle $R_{14}$ à $R_{16}$ ont la même définition que ci-dessus à des températures d'environ — 80 °C à + 30 °C en présence d'un solvant inerte, ou bien

ii) par réaction du phosphoranne obtenu par l'action d'une base sur le compose de formule

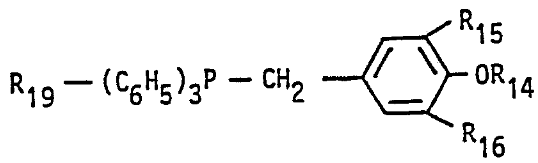

(XIII)

dans laquelle $R_{14}$ à $R_{16}$ et $R_{19}$ ont la même définition que ci-dessus, avec un aldéhyde de formule

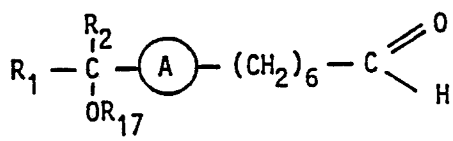

(XIV)

dans laquelle A, $R_1$, $R_2$ et $R_{17}$ ont la même définition que ci-dessus, à des températures d'environ — 80 °C à + 30 °C en présence d'un solvant inerte et on élimine du composé ainsi obtenu de formule II, d'une manière connue, les groupes protecteurs contenus dans $R_{14}$ à $R_{17}$ et le cas échéant — lorsqu'au moins l'un des restes $R_3$ et $R_4$ ou $R_5$ et $R_6$ représente un reste acétyle ou propionyle ou doit contenir un tel reste — on introduit un ou plusieurs groupes acétyle ou propionyle, ou bien

B) On fait réagir un composé de formule

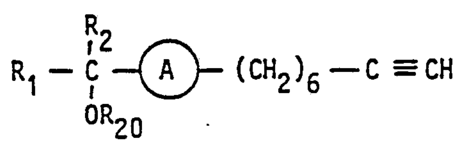

(XV)

dans laquelle A, $R_1$ et $R_2$ ont la même définition que ci-dessus et $R_{20}$ représente un atome d'hydrogène ou a la même définition que $R_{17}$ en présence d'une amine secondaire ou tertiaire liquide entre environ — 10 °C et + 80 °C et de quantités catalytiques d'un catalyseur complexe au palladium ainsi que, le cas échéant, de quantités catalytiques d'iodure de cuivre-(I) à une température d'environ 0 °C à 75 °C avec un composé de formule

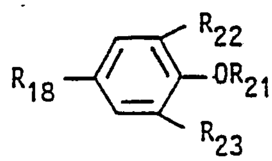

(XVI)

dans laquelle $R_{18}$ a la même définition que ci-dessus, $R_{21}$ est un atome d'hydrogène, le reste alkyle $R_9$ ou un groupe protecteur éliminable dans des conditions douces, $R_{22}$ représente le groupe $OR_{21}$ ou a la

même définition que $R_5$ sous réserve que $R_{10}$ ne puisse pas y représenter un cation et que les groupes basiques éventuellement présents dans $R_5$ ne puissent pas être sous la forme de sel, ou bien $R_{22}$ conjointement avec le groupe $OR_{21}$ représente un groupe méthylènedioxy et $R_{23}$ a la même définition que $R_{16}$ ou représente un groupe hydroxyle, et on élimine du composé obtenu de formule

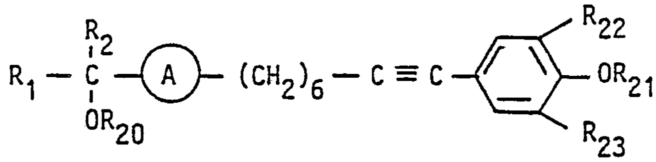

(XVII)

les groupes protecteurs éventuellement contenus dans $R_{20}$ à $R_{23}$ et le cas échéant — lorsqu'au moins l'un des restes $R_3$ et $R_4$ ou $R_5$ et $R_6$ doit représenter ou contenir un reste acétyle ou propionyle, on introduit un ou plusieurs groupes acétyle ou propionyle, ou bien

C) On transforme un composé de formule

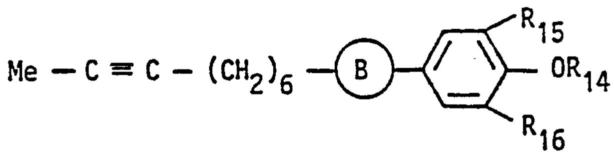

(XVIII)

dans laquelle Me, B et $R_{14}$ à $R_{16}$ ont la même définition que ci-dessus, en présence d'un solvant indifférent, à une température d'environ — 80 °C à + 30 °C, par réaction avec un composé de formule

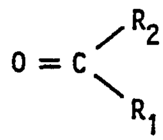

(XIX)

dans laquelle $R_1$ et $R_2$ ont la même définition que ci-dessus, en un composé de formule

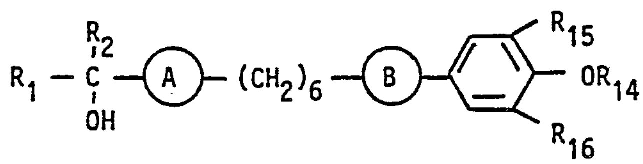

(XX)

dans laquelle A, B, $R_1$, $R_2$ et $R_{14}$ à $R_{16}$ ont la même définition que ci-dessus et on élimine du produit de réaction les groupes protecteurs éventuellement contenus dans $R_{14}$ à $R_{16}$ de même que — lorsqu'au moins l'un des restes $R_3$ et $R_4$ ou $R_5$ et $R_6$ doit représenter ou contenir un reste acétyle ou propionyle — on introduit un ou plusieurs groupes acétyle ou propionyle, ou bien

D) On prépare un composé de formule

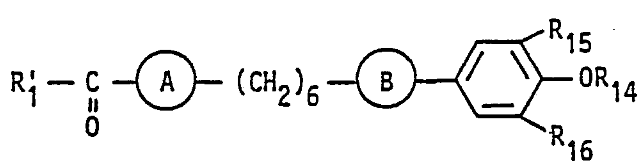

(XXI)

dans laquelle A, B et $R_{14}$ à $R_{16}$ ont la même définition que ci-dessus et $R'_1$, à l'exception de l'hydrogène, a la même définition que $R_1$

1) par traitement d'un composé de formule

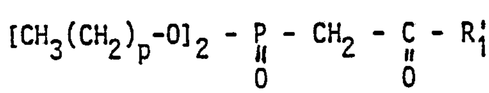

(XXII)

dans laquelle p et $R'_1$ ont la même définition que ci-dessus, dans un solvant inerte avec une base anhydre puis réaction avec un composé de formule VIII à des températures d'environ — 10 °C à + 35 °C, ou bien

2) par réaction d'un composé de formule

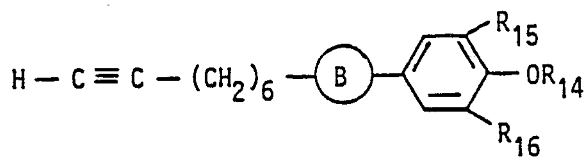

(XXIII)

dans laquelle B et $R_{14}$ à $R_{16}$ ont la même définition que ci-dessus en présence d'une amine tertiaire bouillant au-dessus d'environ +80 °C et de quantités catalytiques d'un catalyseur complexe de palladium ainsi que, le cas échéant, de quantités catalytiques d'iodure de cuivre-(I) à une température d'environ 0 °C à 75 °C avec un composé de formule

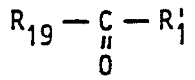

(XXIV)

dans laquelle $R'_1$ et $R_{19}$ ont la même définition que ci-dessus et on transforme ce composé de formule XXI par réduction avec un borohydrure métallique ou par réaction avec un composé de formule $R'_2$-Me, dans laquelle Me a la même définition que ci-dessus et $R'_2$, à l'exception de l'hydrogène, a la même définition que $R_2$, puis élimination de groupes protecteurs présents dans $R_{14}$ à $R_{16}$ et le cas échéant — lorsqu'au moins l'un des restes $R_3$ et $R_4$ ou $R_5$ et $R_6$ doit représenter ou contenir un reste acétyle ou propionyle-introduction d'un ou plusieurs groupes acétyle ou propionyle, en le composé correspondant de formule I (dans laquelle $R_1$ et le cas échéant $R_2$ sont différents de l'hydrogène), et dans les composés obtenus selon A, B, C et D, on transforme les restes $R_5$ et $R_{15}$ ou $R_{22}$ en le groupe $R_5$ désiré et/ou on hydrogène les triples liaisons qui y sont éventuellement présentes, par voie catalytique en doubles liaisons.

19. Procédé suivant la revendication 18, caractérisé en ce que les groupes protecteurs représentés par $R_{14}$, $R_{17}$, $R_{20}$ ou $R_{21}$ ou contenus dans $R_{15}$ et $R_{22}$ sont des représentants identiques ou différents du groupe comprenant le reste tétrahydropyrannyle-2, le reste tertio-butyldiméthylsilyle et le reste tertio-butyldiphénylsilyle.

20. Procédé suivant les revendications 18 et 19, caractérisé en ce qu'on opère, dans les procédés A1 et A3, en préférence d'un solvant aprotique dipolaire et d'un sel de cuivre-(I).

21. Procédé suivant les revendications 18 et 19, caractérisé en ce que dans les procédés A4 et A5, on utilise comme base anhydre pour la formation du phosphoranne par élimination de $HR_{19}$, le n-butyllithium, le tertio-butylate de potassium ou le bis-(triméthylsilyl)-amidure de sodium.

22. Procédé suivant les revendications 18 et 19, caractérisé en ce que dans les procédés B et $D_2$, on utilise comme catalyseur complexe de palladium le chlorure ou l'acétate de bis-(triphénylphosphine)-palladium-(II) ou le tétrakis-(triphénylphosphine)-palladium et on opère en présence d'une trialkylamine ayant 2 ou 3 atomes de carbone dans chacun des restes alkyle.

**Revendications** (pour l'Etat contractant AT)

1. Procédé de production de dérivés de phénol de formule générale

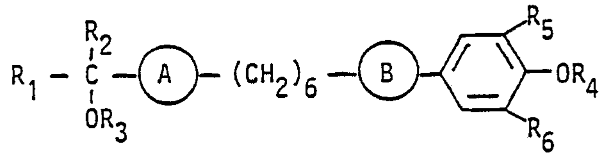

(I)

dans laquelle

A et B sont identiques ou différents et représentent chacun l'un des groupes —C≡C—, cis-CH=CH— ou trans-CH=CH—,

$R_1$ est l'hydrogène ou un reste alkyle à chaîne droite ayant 1 à 6 atomes de carbone ou un groupe cycloalkyle pentagonal à heptagonal ou un groupe de formule $—(CH_2)_m—O-R_7$, dans laquelle m est l'un des nombres 1, 2 et 3 et $R_7$ est un groupe méthyle ou éthyle, ou bien un groupe de formule

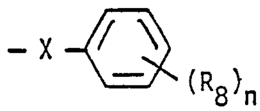

dans laquelle X est une liaison simple, un groupe —CH2— ou un groupe $—CH_2O—$ et $R_8$ est un atome d'hydrogène, de chlore ou de fluor ou un groupe méthyle, méthoxy ou trifluorométhyle et n est l'un des nombres 1 et 2,

$R_2$ est l'hydrogène, un groupe méthyle ou éthyle,

$R_3$ est l'hydrogène ou un reste acétyle ou propionyle,

$R_4$ est l'hydrogène, un reste acétyle ou propionyle ou un reste alkyle $R_9$ à chaîne droite ou à chaîne ramifiée ayant 1 à 4 atomes de carbone,

$R_5$ est un groupe de formule $—COOR_{10}$ dans laquelle $R_{10}$ est un atome d'hydrogène, un cation acceptable du point de vue pharmaceutique, notamment un cation monovalent, un reste alkyle à chaîne droite ou à chaîne ramifiée ayant 1 à 6 atomes de carbone ou le groupe $—(CH_2)_2—N— [(CH_2)_p—CH_3]_2$ dans lequel p est égal à zéro ou à l'un des nombres 1 à 3, ainsi que des sels pharmaceutiquement acceptables de ces esters basiques avec des acides, ou bien un groupe de formule

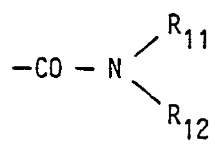

dans laquelle $R_{11}$ et $R_{12}$ sont identiques ou différents et représentent l'hydrogène, le reste alkyle $R_9$ ou un reste 2-hydroxyéthyle ou bien l'un de ces restes représente un groupe hydroxyle, l'autre représente un atome d'hydrogène, ou bien $R_{11}$ et $R_{12}$ forment conjointement le groupe $—(CH_2)_q—$ dans lequel q est l'un des nombres 4, 5 et 6, ou bien un groupe de formule

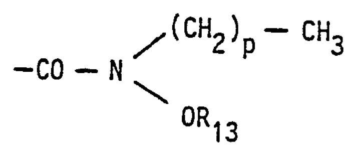

dans laquelle $R_{13}$ est l'hydrogène, un groupe $—(CH_2)_p—CH_3$, carboxyméthyle, acétyle ou propionyle et p a dans chaque cas la même définition que ci-dessus, ou bien un groupe de formule $—CO—NH—(CH_2)_r—N(CH_3)_2$, dans laquelle r est le nombre 2 ou 3, et ses sels d'acides pharmaceutiquement acceptables, ou bien un groupe de formule

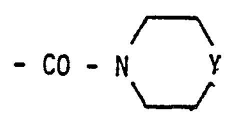

dans laquelle Y est un atome d'oxygène ou le groupe $>N—CH_3$ et le cas échéant ses sels d'acides pharmaceutiquement acceptables, ou bien un reste nitrile, ou bien un groupe hydroxy, acétyloxy ou propionyloxy, un groupe alkoxy $OR_9$ dans lequel $R_9$ a la même définition que ci-dessus, ou bien — conjointement avec $OR_4$ — le groupe méthylènedioxy et $R_6$ est l'hydrogène, un groupe hydroxy, acétyloxy ou propionyloxy, un reste alkyle $R_9$ ou un groupe alkoxy $OR_9$ dans laquelle $R_9$ a dans chaque cas la même définition que ci-dessus, caractérisé en ce que

A) On prépare un composé de formule générale II

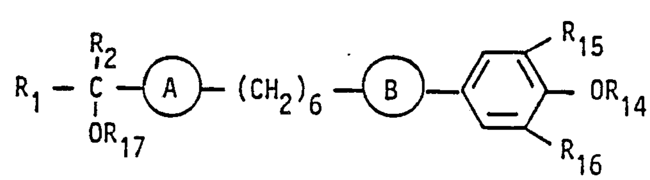

(II)

dans laquelle A, B, $R_1$ et $R_2$ ont la même définition que dans la formule I, $R_{14}$ représente le reste alkyle $R_9$ ou est un groupe protecteur éliminable dans des conditions douces, $R_{15}$ est le groupe $OR_{14}$ et a la même définition que $R_5$ sous réserve que $R_{10}$ ne puisse pas y représenter un cation et que les groupes basiques éventuellement présents dans $R_5$ ne puissent pas se présenter sous la forme de sel, ou bien $R_{15}$ forme conjointement avec le groupe $OR_{14}$ un groupe méthylènedioxy, $R_{16}$ est un atome d'hydrogène ou l'un des groupes $R_9$ ou $OR_{14}$ et $R_{17}$ est un groupe protecteur éliminable dans des conditions douces,

1) par réaction d'un composé de formule

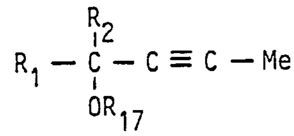

(III)

dans laquelle $R_1$, $R_2$ et $R_{17}$ ont la même définition que ci-dessus et Me représente un atome de lithium, de sodium ou de potassium ou l'un des restes —MgBr ou —MgI, avec un composé de formule

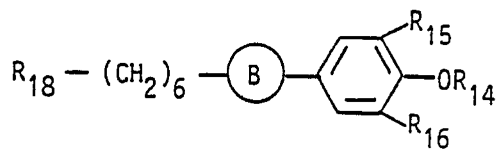

(IV)

dans laquelle B et $R_{14}$ à $R_{16}$ ont la même définition que ci-dessus et $R_{18}$ est un atome de brome ou un atome d'iode, à des températures d'environ —80 à +75 °C dans un solvant aprotique inerte, éventuellement en présence de quantités catalytiques d'un sel de cuivre, ou bien

2) par réaction d'un composé de formule IV dans laquelle $R_{15}$ représente le groupe $OR_{14}$, avec un composé de formule

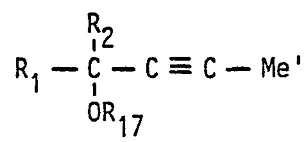

(IIIa)

dans laquelle $R_1$, $R_2$ et $R_{17}$ ont la même définition que ci-dessus et Me' représente un atome de lithium, de sodium ou de potassium, dans l'ammoniac liquide à des températures d'environ —80 °C à —35 °C, ou bien

3) par réaction d'un composé de formule

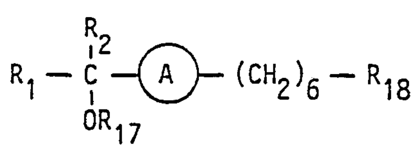

(V)

dans laquelle A, $R_1$, $R_2$ et $R_{17}$ et $R_{18}$ ont la même définition que ci-dessus, avec un composé de formule

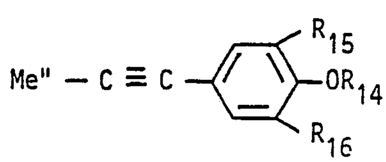

(VI)

dans laquelle $R_{14}$ à $R_{16}$ ont la même définition que ci-dessus et Me" représente l'un des groupes Me ou Me' définis ci-dessus, dans les conditions mentionnées en 1 et 2 ci-dessus, ou bien

4) (lorsque, dans le compose de formule II, le groupe A représente —CH=CH—)

i) par traitement d'un composé de formule

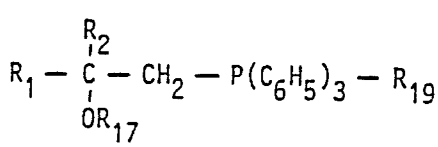

(VII)

dans laquelle $R_1$, $R_2$ et $R_{17}$ ont la même définition que ci-dessus et $R_{19}$ est un atome de chlore, de brome ou d'iode, dans des solvants inertes avec une base forte anhydre et réaction du phosphoranne formé par élimination de $HR_{19}$ avec un aldéhyde de formule

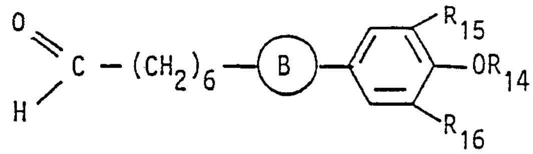

(VIII)

dans laquelle B et $R_{14}$ à $R_{16}$ ont la même définition que ci-dessus, à des températures d'environ — 80 °C à + 30 °C, ou bien

ii) par réaction du phosphoranne formé avec élimination de $HR_{19}$ par l'action d'une base sur le composé de formule

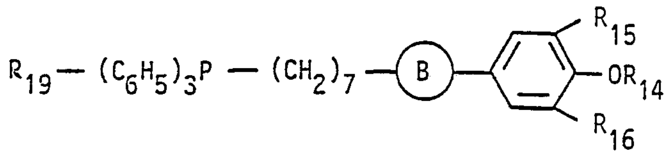

(IX)

dans laquelle B, $R_{14}$ à $R_{16}$ et $R_{19}$ ont la même définition que ci-dessus, avec un aldéhyde de formule

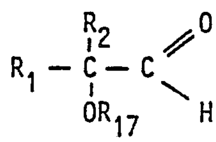

(X)

dans laquelle $R_1$, $R_2$ et $R_{17}$ ont la même définition que ci-dessus, à des températures d'environ — 80 °C à + 30 °C en présence d'un solvant inerte, ou bien

5) (lorsque dans le composé de formule II le groupe B est un groupe —CH=CH—)

i) par réaction d'un phosphoranne forme à partir du composé de formule

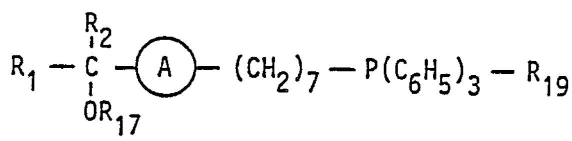

(XI)

dans laquelle A, $R_1$, $R_2$, $R_{17}$ et $R_{19}$ ont la même définition que ci-dessus, par l'action d'une base et avec élimination de $HR_{19}$, avec un aldéhyde de formule

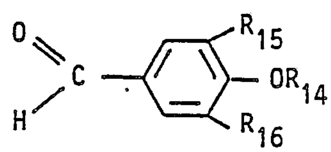

(XII)

dans laquelle $R_{14}$ à $R_{16}$ ont la même définition que ci-dessus, à des températures d'environ — 80 °C à + 30 °C en présence d'un solvant inerte, ou bien

ii) par réaction du phosphoranne obtenu par l'action d'une base sur le composé de formule

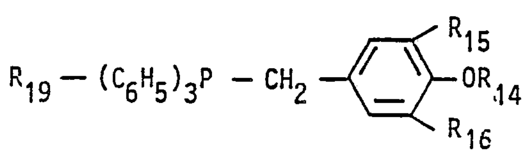

(XIII)

dans laquelle $R_{14}$ à $R_{16}$ et $R_{19}$ ont la même définition que ci-dessus, avec un aldéhyde de formule

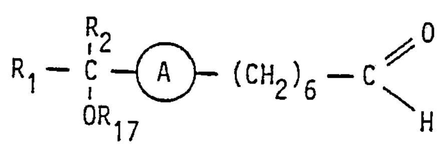

(XIV)

dans laquelle A, $R_1$, $R_2$ et $R_{17}$ ont la même définition que ci-dessus à des températures d'environ — 80 °C à + 30 °C en présence d'un solvant inerte et on élimine du composé ainsi obtenu de formule II, d'une manière connue, les groupes protecteurs contenus dans $R_{14}$ à $R_{17}$ et le cas échéant — lorsqu'au moins l'un des restes $R_3$ et $R_4$ ou $R_5$ et $R_6$ doit représenter ou contenir un reste acétyle ou propionyle — on introduit un ou plusieurs groupes acétyle ou propionyle, ou bien

B) On fait réagir un composé de formule

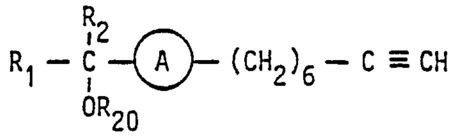
(XV)

dans laquelle A, $R_1$ et $R_2$ ont la même définition que ci-dessus et $R_{20}$ représente un atome d'hydrogène ou a la même définition que $R_{17}$, en présence d'une amine secondaire ou tertiaire liquide à une température d'environ — 10 °C + 80 °C et de quantités catalytiques d'un catalyseur complexe de palladium ainsi que, le cas échéant, de quantités catalytiques d'iodure de cuivre-(I) à une température d'environ 0 °C à 75 °C avec un composé de formule

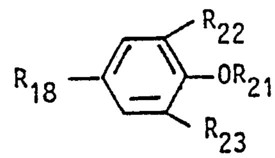
(XVI)

dans laquelle $R_{18}$ a la même définition que ci-dessus, $R_{21}$ représente un atome d'hydrogène, un reste alkyle $R_9$ ou un groupe protecteur éliminable dans des conditions douces, $R_{22}$ représente le groupe $OR_{21}$ ou a la même définition que $R_5$ sous réserve que $R_{10}$ ne puisse pas y être un cation et que les groupes basiques éventuellement présents dans $R_5$ ne puissent pas être sous la forme de sels, ou bien $R_{22}$ forme conjointement avec le groupe $OR_{21}$ un groupe methylènedioxy et $R_{23}$ a la même définition que $R_{16}$ ou représente un groupe hydroxyle, et on élimine du composé obtenu de formule

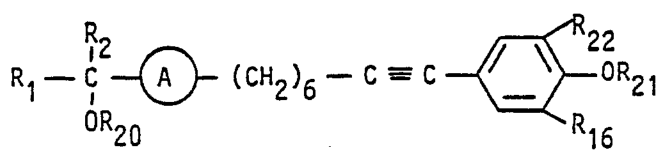
(XVII)

les groupes protecteurs éventuellement contenus dans $R_{20}$ à $R_{23}$ et on introduit éventuellement — lorsqu'au moins l'un des restes $R_3$ et $R_4$ ou $R_5$ et $R_6$ doit représenter ou contenir un reste acétyle ou propionyle — un ou plusieurs groupes acétyle ou propionyle, ou bien

C) On transforme un composé de formule

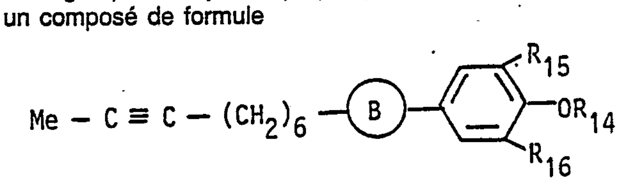
(XVIII)

dans laquelle Me, B et $R_{14}$ à $R_{16}$ ont la même définition que ci-dessus, en présence d'un solvant indifférent, à une température d'environ — 80 °C à + 30 °C, par réaction avec un composé de formule

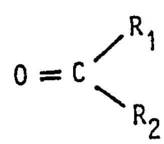
(XIX)

dans laquelle $R_1$ et $R_2$ ont la même définition que ci-dessus, en un composé de formule

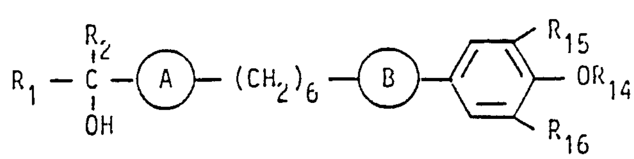
(XX)

dans laquelle A, B, $R_1$, $R_2$ et $R_{14}$ à $R_{16}$ ont la même définition que ci-dessus et on élimine du produit de réaction les groupes protecteurs éventuellement contenus dans $R_{14}$ à $R_{16}$ ainsi que — lorsqu'au moins l'un des restes $R_3$ et $R_4$ ou $R_5$ et $R_6$ doit représenter ou contenir un reste acétyle ou propionyle — on introduit un ou plusieurs groupes acétyle ou propionyle, ou bien

D) On prépare un composé de formule

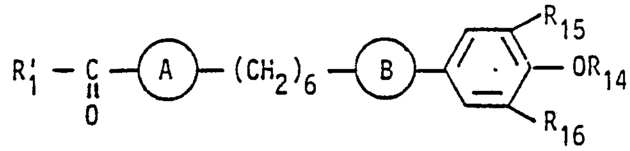

(XXI)

dans laquelle A, B et $R_{14}$ à $R_{16}$ ont la même définition que ci-dessus et $R'_1$, à l'exception de l'hydrogène, à la même définition que $R_1$

1) par traitement d'un composé de formule

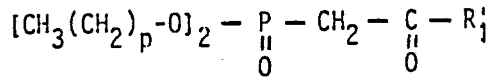

(XXII)

dans laquelle p et $R'_1$ ont la même définition que ci-dessus dans un solvant inerte avec une base anhydre, puis réaction avec un composé de formule VIII à des températures d'environ — 10 °C à + 35 °C, ou bien

2) par réaction d'un composé de formule

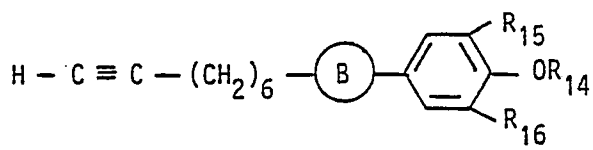

(XXIII)

dans laquelle B et $R_{14}$ à $R_{16}$ ont la même définition que ci-dessus, en présence d'une amine tertiaire bouillant au-dessus d'environ + 80 °C et de quantités catalytiques d'un catalyseur complexe de palladium ainsi que, le cas échéant, de quantités catalytiques d'iodure de cuivre-(I) à une température d'environ 0 °C à 75 °C avec un composé de formule

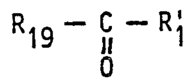

(XXIV)

dans laquelle $R'_1$ et $R_{19}$ ont la même définition que ci-dessus et on transforme ce composé de formule XXI par réduction avec un borohydrure métallique ou par réaction avec un composé de formule $R'_2$-Me, dans laquelle Me à la même définition que ci-dessus et $R'_2$, à l'exception de l'hydrogène, a la même définition que $R_2$, puis élimination de groupes protecteurs présents dans $R_{14}$ à $R_{16}$ et le cas échéant — lorsqu'au moins l'un des restes $R_3$ et $R_4$ ou $R_5$ et $R_6$ doit représenter ou contenir un reste acétyle ou propionyle — introduction d'un ou plusieurs groupes acétyle ou propionyle, en le composé correspondant de formule I (dans laquelle $R_1$ et le cas échéant $R_2$ sont différents de l'hydrogène), et on transforme dans les composés obtenus selon A, B, C et D les restes $R_5$ et $R_{15}$ ou $R_{22}$ en le groupe $R_5$ désiré et/ou on hydrogène par voie catalytique en doubles liaisons des triples liaisons qui y sont éventuellement présentes.

2. Procédé suivant la revendication 1, caractérisé en ce que les groupes protecteurs représentés par $R_{14}$, $R_{17}$, $R_{20}$ ou $R_{21}$ ou contenus dans $R_{15}$ et $R_{22}$ sont des représentants identiques ou différents du groupe comprenant le reste tétrahydropyrannyle-2, le reste tertio-butyldiméthylsilyle et le reste tertio-butyldiphénylsilyle.

3. Procédé suivant les revendications 1 et 2, caractérisé en ce qu'on opère dans les formes de mise en œuvre A1 et A3 en présence d'un solvant aprotique dipolaire et d'un sel de cuivre (I).

4. Procédé suivant les revendications 1 et 2, caractérisé en ce que dans les formes de mise en œuvre A4 et A5, on utilise pour la formation du phosphoranne par élimination de $HR_{19}$, comme base anhydre, le n-butyllithium, le tertio-butylate de potassium ou le bis-(triméthylsilyl)-amidure de sodium.

5. Procédé suivant les revendications 1 et 2, caractérisé en ce que dans les formes de mise en œuvre B et $D_2$, on utilise comme catalyseur complexe de palladium le chlorure ou l'acétate de bis-(triphenylphosphine)-palladium-(II) ou le tétrakis-(triphénylphosphine)-palladium et on opère en présence d'une trialkylamine ayant 2 ou 3 atomes de carbone dans chacun des restes alkyle.

6. Procédé suivant les revendications 1 et 5, caractérisé en ce que dans les formes de mise en œuvre

B et $D_2$, on opère en présence d'un catalyseur complexe de palladium et de quantités catalytiques d'iodure de cuivre-(I).

7. Procédé suivant la revendication 1, caractérisé en ce qu'on utilise pour l'hydrogénation catalytique de triples liaisons présentes dans les groupes A ou B en doubles liaisons, des catalyseurs au palladium empoisonnés au plomb et on opère en présence de quantités au moins catalytiques de pyridine ou de quinoléine.

8. Procédé suivant la revendication 1, caractérisé en ce que, pour la préparation de composés de formule I dans lesquels $R_5$ représente un groupe de formules

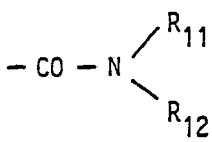 , 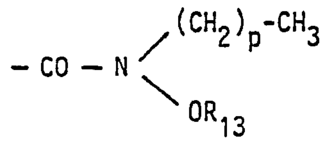 ou 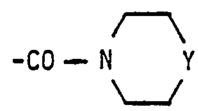

où $R_{11}$ à $R_{13}$, p et Y ont la même définition que ci-dessus, on fait réagir un composé aminé de formules

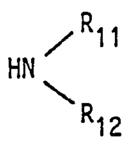 , 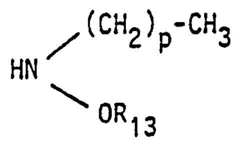 ou 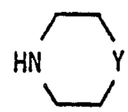

où $R_{11}$ à $R_{13}$, p et Y ont la même définition que ci-dessus,

a) avec un composé de formule I dans laquelle $R_5$ est un groupe carboxyle estérifié, ou bien

b) avec un composé de formule I dans laquelle $R_5$ est un groupe carboxyle libre, en présence d'agents déshydratants ou après formation intermédiaire d'un dérivé fonctionnel réactif du groupe carboxyle.